# EUROPÄISCHE PATENTANMELDUNG

(11) **EP 4 230 621 A1**
(43) Veröffentlichungstag der Anmeldung: **23.08.2023**
(21) Anmeldenummer: 22157930.3
(22) Anmeldetag: 22.02.2022
(51) Int. Cl.: C07D 239/54, A01N 43/50, C07D 401/12, C07D 403/12, C07D 405/12, C07D 409/12, C07D 413/12, C07D 417/12, C07D 487/08, C07D 493/04, C07D 493/08, C07F 5/02, C07F 5/04, C07F 7/08

(54) **SUBSTITUIERTE N-BENZOESÄUREURACILE SOWIE DEREN SALZE UND IHRE VERWENDUNG ALS HERBIZIDE WIRKSTOFFE**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Substituierte N-Benzoesäureuracile sowie deren Salze und ihre Verwendung als herbizide Wirkstoffe.

Die vorliegende Erfindung betrifft substituierte N-Benzoesäureuracile der allgemeinen Formel (I) 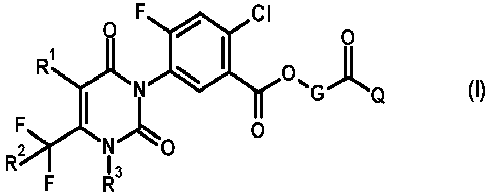 oder deren Salze,
wobei die Reste in der allgemeinen Formel (I) den in der Beschreibung gegebenen Definitionen entsprechen,
sowie deren Verwendung als Herbizide, insbesondere zur Bekämpfung von Unkräutern und/oder Ungräsern in Nutzpflanzenkulturen und/oder als Pflanzenwachstumsregulatoren zur Beeinflussung des Wachstums von Nutzpflanzenkulturen.

## Beschreibung

Die Erfindung betrifft das technische Gebiet der Pflanzenschutzmittel, insbesondere das der Herbizide zur selektiven Bekämpfung von Unkräutern und Ungräsern in Nutzpflanzenkulturen.

Speziell betrifft diese Erfindung substituierte N-Benzoesäureuracile sowie deren Salze, Verfahren zu ihrer Herstellung und ihre Verwendung als Herbizide, insbesondere zur Bekämpfung von Unkräutern und/oder Ungräsern in Nutzpflanzenkulturen und/oder als Pflanzenwachstumsregulatoren zur Beeinflussung des Wachstums von Nutzpflanzenkulturen.

Bisher bekannte Pflanzenschutzmittel zur selektiven Bekämpfung von Schadpflanzen in Nutzpflanzenkulturen oder Wirkstoffe zur Bekämpfung von unerwünschtem Pflanzenwuchs weisen bei ihrer Anwendung teilweise Nachteile auf, sei es, dass sie (a) keine oder aber eine unzureichende herbizide Wirkung gegen bestimmte Schadpflanzen, (b) ein zu geringes Spektrum der Schadpflanzen, das mit einem Wirkstoff bekämpft werden kann, (c) zu geringe Selektivität in Nutzpflanzenkulturen und/oder (d) ein toxikologisch ungünstiges Profil besitzen. Weiterhin führen manche Wirkstoffe, die als Pflanzenwachstumsregulatoren bei einigen Nutzpflanzen eingesetzt werden können, bei anderen Nutzpflanzen zu unerwünscht verminderten Ernteerträgen oder sind mit der Kulturpflanze nicht oder nur in einem engen Aufwandmengenbereich verträglich. Einige der bekannten Wirkstoffe lassen sich wegen schwer zugänglicher Vorprodukte und Reagenzien im industriellen Maßstab nicht wirtschaftlich herstellen oder besitzen nur unzureichende chemische Stabilitäten. Bei anderen Wirkstoffen hängt die Wirkung zu stark von Umweltbedingungen, wie Wetter- und Bodenverhältnissen ab.

Die herbizide Wirkung dieser bekannten Verbindungen, insbesondere bei niedrigen Aufwandmengen, bzw. deren Verträglichkeit gegenüber Kulturpflanzen bleiben verbesserungswürdig.

Es ist aus verschiedenen Schriften bekannt, dass bestimmte substituierte N-verknüpfte Aryluracile als herbizide Wirkstoffe verwendet werden können (vgl. EP408382, EP473551, EP648749, US4943309, US5084084, US5127935, WO91/00278, WO95/29168, WO95/30661, WO96/35679, WO97/01541, WO98/25909, WO2001/39597). Die bekannten Aryluracile weisen jedoch eine Reihe von Wirkungslücken, insbesondere gegenüber monokotylen Unkräutern auf. Eine Reihe von herbiziden Wirkstoffkombinationen auf Basis von N-verknüpften Aryluracilen sind ebenfalls bekannt geworden (vgl. DE4437197, EP714602, WO96/07323, WO96/08151, JP11189506). Die Eigenschaften dieser Wirkstoffkombinationen waren jedoch auch nicht in allen Belangen zufriedenstellend.

Es ist weiterhin bekannt, dass bestimmte N-Aryluracile mit gegebenenfalls weiter substituierten Milchsäuregruppen auch als herbizide Wirkstoffe eingesetzt werden können (vgl. JP2000/302764, JP2001/172265, US6403534, EP408382A1). Es ist darüber hinaus bekannt, dass N-Aryluracile mit speziellen, gegebenenfalls weiter substituierten, Thiomilchsäuregruppen ebenfalls herbizide Wirkungen zeigen (vgl. WO2010/038953, KR2011110420). Ausgewählte substituierte Tetrahydrofurylester von N-Aryluracilen mit gegebenenfalls weiter substituierten Thiomilchsäuregruppen sind in JP09188676 beschrieben.

Ebenfalls bekannt sind substituierte N-Benzoesäureuracile, die Chlorsubstituenten in der Benzoesäureeinheit tragen (vgl. WO91/000278, DE19741411, WO95/32952, US6207830, WO88/10254, EP831091). Weiterhin sind hochsubstituierte 3-amino-1-(3-carboxy-4-cyanophenyl)uracile mit verschiedenen Carboxylatseitenketten beschrieben (vgl. WO98/25909). Hochsubstituierte N-Benzoesäureuracile mit Aminosulfonylaminocarbonylalkoxyseitenkette sind ebenfalls bekannt (vgl. WO2004/009561). Es ist weiterhin bekannt, daß bestimmte substituierte N-Benzoesäurethiobarbiturate als herbizide Wirkstoffe eingesetzt werden können (vgl. WO2021/259224).

N-Benzoesäureuracile mit weiter substituierten Essigäureestern sind dagegen nicht beschrieben.

Überraschenderweise wurde nun gefunden, dass bestimmte N-Benzoesäureuracile mit weiter substituierten Essigäureestern oder deren Salze als Herbizide gut geeignet sind und besonders vorteilhaft als Wirkstoffe zur Bekämpfung von monokotylen und dikotylen Unkräutern in Nutzpflanzenkulturen eingesetzt werden können.

Ein Gegenstand der vorliegenden Erfindung sind damit substituierte N-Benzoesäureuracile der allgemeinen Formel (I) oder deren Salze 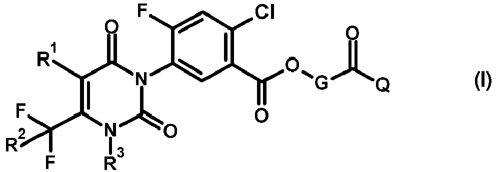 worin
- R¹: für Wasserstoff, Methyl steht,
- R²: für Wasserstoff, Halogen, Trifluormethyl steht,
- R³: für (C₁-C₆)-Alkyl steht,
- G: für einen Rest der nachfolgenden Formel 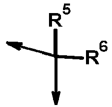 steht,
- R⁵ und R⁶: unabhängig voneinander für Wasserstoff, (C₁-C₈)-Alkyl, (C1-C₈)-Haloalkyl, (C₁-C₈)-Alkoxy-(C₁-C₈)-Alkyl stehen,
- R⁵ und R⁶: mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- Q: für einen Rest der nachfolgenden Formeln 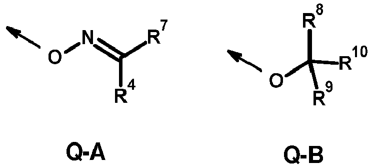 steht,

- R⁴ und R⁷: unabhängig voneinander für (C₁-C₈)-Alkyl, (C₃-C₈)-Cycloalkyl, Aryl, Heteroaryl, Heterocyclyl stehen und
- R⁴ und R⁷: mit dem Kohlenstoffatom, an das sie gebunden sind, einen vollständig gesättigten monocyclischen 3- bis 7-gliedrigen Carbocyclus bilden,
- R⁸: für Wasserstoff, (C₁-C₈)-Alkyl, (C₁-C₈)-Haloalkyl, Aryl, Aryl-(C₁-C₈)-alkyl, Heteroaryl, (C₂-C₈)-Alkinyl, (C₂-C₈)-Alkenyl, C(O)R¹³, C(O)OR¹³, (C₁-C₈)-Alkoxy-(C₁-C₈)-alkyl steht,
- R⁹: für Wasserstoff oder (C₁-C₈)-Alkyl steht,
- R¹⁰: für Halogen, Cyano, NO₂, (C₁-C₈)-Haloalkyl, (C₃-C₈)-Cycloalkyl, (C₃-C₈)-Cycloalkyl-(C₁-C₈)-alkyl, (C₃-C₈)-Halocycloalkyl, (C₃-C₈)-Halocycloalkyl-(C₁-C₈)-alkyl, Aryl, Aryl-(C₁-C₈)-alkyl, Heteroaryl, Heteroaryl-(C₁-C₈)-alkyl, Heterocyclyl, Heterocyclyl-(C₁-C₈)-alkyl, R¹¹R¹²N-(C₁-C₈)-alkyl, R¹³O-(C₁-C₈)-alkyl, Cyano-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, OR¹³, NR¹¹R¹², SR¹⁴, S(O)R¹⁴, SO₂R¹⁴, R¹⁴S-(C₁-C₈)-alkyl, R¹⁴(O)S-(C₁-C₈)-alkyl, R¹⁴O₂S-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, Tris-[(C₁-C₈)-Alkyl]silyl, Bis-hydroxyboryl-(C₁-C₈)-alkyl, Bis-[(C₁-C₈)-alkoxy]boryl-(C₁-C₈)-alkyl, Tetramethyl-1,3,2-Dioxaborolan-2-yl, Tetramethyl-1,3,2-Dioxaborolan-2-yl-(C₁-C₈)-alkyl, Nitro-(C₁-C₈)-alkyl, C(O)OR¹³, C(O)R¹³, C(O)NR¹¹R¹², R¹³O(O)C-(C₁-C₈)-alkyl, R¹¹R¹²N(O)C-(C₁-C₈)-alkyl, Bis-(C₁-C₈)-alkoxy-(C₁-C₈)-alkyl steht,
- R¹¹ und R¹²: gleich oder verschieden sind und unabhängig voneinander für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, C(O)R¹³, SO₂R¹⁴, Heterocyclyl, (C₁-C₈)-Alkoxycarbonyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Aryl-(C₁-C₈)-alkoxycarbonyl, Heteroaryl-(C₁-C₈)-alkoxycarbonyl, (C₂-C₈)-Alkenyloxycarbonyl, (C₂-C₈)-Alkinyloxycarbonyl, Heterocyclyl-(C₁-C₈)-alkyl stehen, oder
- R¹¹ und R¹²: mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- R¹³: für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkoxy-(C₁-C₈)-haloalkyl, (C₁-C₈)-Alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkoxy-(C₁-C₈)-alkyl, Aryl, Aryl-(C₁-C₈)-alkyl, Aryl-(C₁-C₈)-alkoxy-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Bis-[(C₁-C₈)-alkyl]amino-(C₂-C₆)-alkyl, (C₁-C₈)-Alkyl-amino-(C₂-C₆)-alkyl, Aryl-(C₁-C₈)-alkyl-amino-(C₂-C₆)-alkyl, R¹⁴S-(C₁-C₈)-alkyl, R¹⁴(O)S-(C₁-C₈)-alkyl, R¹⁴O₂S-(C₁-C₈)-alkyl, Hydroxycarbonyl-(C₁-C₈)-alkyl, Heterocyclyl, Heterocyclyl-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, Aryloxy-(C₁-C₈)-alkyl, Heteroaryloxy-(C₁-C₈)-alkyl, (C₁-C₈)-Alkoxycarbonyl steht
und
- R¹⁴: für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₁₀)-Cycloalkenyl-(C₁-C₈)-alkyl, Bis-[(C₁-C₈)-alkyl]amino, (C₁-C₈)-Alkyl-amino, Aryl-(C₁-C₈)-amino, Aryl-(C₁-C₆)-alkyl-amino, Aryl-[(C₁-C₈)-alkyl]amino; (C₃-C₈)-Cycloalkyl-amino, (C₃-C₈)-Cycloalkyl-[(C₁-C₈)-alkyl]amino; N-Azetidinyl, N-Pyrrolidinyl, N-Piperidinyl. N-Morpholinyl steht.

Die Verbindungen der allgemeinen Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion. Geeignete Substituenten, die in deprotonierter Form, wie z.B. Sulfonsäuren, bestimmte Sulfonsäureamide oder Carbonsäuren, vorliegen, können innere Salze mit ihrerseits protonierbaren Gruppen, wie Aminogruppen bilden. Salzbildung kann auch durch Einwirkung einer Base auf Verbindungen der allgemeinen Formel (I) erfolgen. Geeignete Basen sind beispielsweise organische Amine, wie Trialkylamine, Morpholin, Piperidin und Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetall-salze oder Erdalkalimetallsalze, insbesondere Natrium- und Kaliumsalze, oder auch Ammoniumsalze, Salze mit organischen Aminen oder quartäre Ammoniumsalze, zum Beispiel mit Kationen der Formel [NR^{a}R^{b}R^{c}R^{d}]⁺, worin R^{a} bis R^{d} jeweils unabhängig voneinander einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Im Folgenden werden die erfindungsgemäß verwendeten Verbindungen der Formel (I) und ihre Salze "Verbindungen der allgemeinen Formel (I)" bezeichnet.

Bevorzugter Erfindungsgegenstand sind Verbindungen der allgemeinen Formel (I), worin
- R¹: für Wasserstoff steht,
- R²: für Wasserstoff, Fluor, Chlor, Brom, Iod steht,
- R³: für (C₁-C₅)-Alkyl steht,
- G: für einen Rest der nachfolgenden Formel 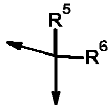 steht,

- R⁵ und R⁶: unabhängig voneinander für Wasserstoff, (C₁-C₇)-Alkyl stehen, oder
- R⁴ und R⁶: mit dem Kohlenstoffatom, an das sie gebunden sind, einen vollständig gesättigten monocyclischen 3- bis 6-gliedrigen Carbocyclus bilden
- Q: für einen Rest der nachfolgenden Formeln 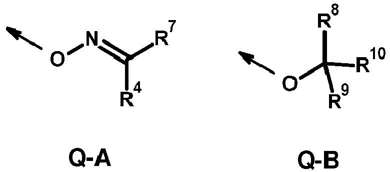 steht,
- R⁴ und R⁷: unabhängig voneinander für (C₁-C₇)-Alkyl, (C₃-C₇)-Cycloalkyl, Aryl, Heteroaryl, Heterocyclyl stehen, oder
- R⁴ und R⁷: mit dem Kohlenstoffatom, an das sie gebunden sind, einen vollständig gesättigten monocyclischen 3- bis 6-gliedrigen Carbocyclus bilden,
- R⁸: für Wasserstoff, (C₁-C₇)-Alkyl, (C₁-C₇)-Haloalkyl, Aryl, Aryl-(C₁-C₇)-alkyl, Heteroaryl, (C₂-C₇)-Alkinyl, (C₂-C₇)-Alkenyl, C(O)R¹³, C(O)OR¹³, (C₁-C₇)-Alkoxy-(C₁-C₇)-alkyl steht,
- R⁹: für Wasserstoff oder (C₁-C₇)-Alkyl steht,
- R¹⁰: für Halogen, Cyano, NO₂, (C₁-C₇)-Haloalkyl, (C₃-C₇)-Cycloalkyl, (C₃-C₇)-Cycloalkyl-(C₁-C₇)-alkyl, (C₃-C₇)-Halocycloalkyl, (C₃-C₇)-Halocycloalkyl-(C₁-C₇)-alkyl, Aryl, Aryl-(C₁-C₇)-alkyl, Heteroaryl, Heteroaryl-(C₁-C₇)-alkyl, Heterocyclyl, Heterocyclyl-(C₁-C₇)-alkyl, R¹¹R¹²N-(C₁-C₇)-alkyl, R¹³O-(C₁-C₇)-alkyl, Cyano-(C₁-C₇)-alkyl, (C₁-C₇)-Alkylcarbonyloxy-(C₁-C₇)-alkyl, (C₃-C₇)-Cycloalkylcarbonyloxy-(C₁-C₇)-alkyl, Arylcarbonyloxy-(C₁-C₇)-alkyl, Heteroarylcarbonyloxy-(C₁-C₈)-alkyl, Heterocyclylcarbonyloxy-(C₁-C₇)-alkyl, OR¹³, NR¹¹R¹², SR¹⁴, S(O)R¹⁴, SO₂R¹⁴, R¹⁴S-(C₁-C₇)-alkyl, R¹⁴(O)S-(C₁-C₇)-alkyl, R¹⁴O₂S-(C₁-C₇)-alkyl, Tris-[(C₁-C₇)-Alkyl]silyl-(C₁-C₇)-alkyl, Bis-[(C₁-C₇)-Alkyl](aryl)silyl(C1-C₇)-alkyl, [(C₁-C₇)-Alkyl]-bis-(aryl)silyl-(C₁-C₇)-alkyl, Tris-[(C₁-C₇)-Alkyl]silyl, Bis-hydroxyboryl-(C₁-C₇)-alkyl, Bis-[(C₁-C₇)-alkoxy]boryl-(C₁-C₇)-alkyl, Tetramethyl-1,3,2-Dioxaborolan-2-yl, Tetramethyl-1,3,2-Dioxaborolan-2-yl-(C₁-C₈)-alkyl, Nitro-(C₁-C₈)-alkyl, C(O)OR¹³, C(O)R¹³ , C(O)NR¹¹R¹², R¹³O(O)C-(C₁-C₈)-alkyl, R¹¹R¹²N(O)C-(C₁-C₈)-alkyl, Bis-(C₁-C₈)-alkoxy-(C₁-C₈)-alkyl steht,
- R¹¹ und R¹²: gleich oder verschieden sind und unabhängig voneinander für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₃-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Haloalkoxy-(C₁-C₇)-alkyl, (C₁-C₇)-Alkylthio-(C₁-C₇)-alkyl, (C₁-C₇)-Haloalkylthio-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, (C₃-C₇)-Cycloalkyl-(C₁-C₇)-alkyl, (C₄-C₁₀)-Cycloalkenyl-(C₁-C₇)-alkyl, C(O)R¹³, SO₂R¹⁴, Heterocyclyl, (C₁-C₇)-Alkoxycarbonyl, Bis-[(C₁-C₇)-alkyl]aminocarbonyl-(C₁-C₇)-alkyl, (C₁-C₇)-Alkyl-aminocarbonyl-(C₁-C₇)-alkyl, Aryl-(C₁-C₇)-alkyl-aminocarbonyl-(C₁-C₇)-alkyl, Aryl-(C₁-C₇)-alkoxycarbonyl, Heteroaryl-(C₁-C₇)-alkoxycarbonyl, (C₂-C₇)-Alkenyloxycarbonyl, (C₂-C₇)-Alkinyloxycarbonyl, Heterocyclyl-(C₁-C₇)-alkyl stehen, oder
- R¹¹ und R¹²: mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- R¹³: für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₃-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Haloalkoxy-(C₁-C₇)-alkyl, (C₁-C₇)-Alkoxy-(C₁-C₇)-haloalkyl, (C₁-C₇)-Alkoxy-(C₁-C₇)-alkoxy-(C₁-C₇)-alkyl, (C₁-C₇)-Alkoxy-(C₁-C₇)-alkoxy-(C₁-C₇)-alkoxy-(C₁-C₇)-alkyl, (C₁-C₇)-Alkoxy-(C₁-C₇)-alkoxy-(C₁-C₇)-alkoxy-(C₁-C₇)-alkoxy-(C₁-C₇)-alkyl, Aryl, Aryl-(C₁-C₇)-alkyl, Aryl-(C₁-C₇)-alkoxy-(C₁-C₇)-alkyl, Heteroaryl, Heteroaryl-(C₁-C₇)-alkyl, (C₃-C₇)-Cycloalkyl-(C₁-C₇)-alkyl, (C₄-C₁₀)-Cycloalkenyl-(C₁-C₇)-alkyl, Bis-[(C₁-C₇)-alkyl]aminocarbonyl-(C₁-C₇)-alkyl, (C₁-C₇)-Alkyl-aminocarbonyl-(C₁-C₇)-alkyl, Aryl-(C₁-C₇)-alkyl-aminocarbonyl-(C₁-C₇)-alkyl, Bis-[(C₁-C₇)-alkyl]amino-(C₂-C₆)-alkyl, (C₁-C₇)-Alkyl-amino-(C₂-C₆)-alkyl, Aryl-(C₁-C₇)-alkyl-amino-(C₂-C₆)-alkyl, R¹⁴S-(C₁-C₇)-alkyl, R¹⁴(O)S-(C₁-C₇)-alkyl, R¹⁴O₂S-(C₁-C₇)-alkyl, Hydroxycarbonyl-(C₁-C₇)-alkyl, Heterocyclyl, Heterocyclyl-(C₁-C₇)-alkyl, Tris-[(C₁-C₇)-Alkyl]silyl-(C₁-C₇)-alkyl, Bis-[(C₁-C₇)-Alkyl](aryl)silyl(C₁-C₇)-alkyl, [(C₁-C₇)-Alkyl]-bis-(aryl)silyl-(C₁-C₇)-alkyl, (C₁-C₇)-Alkylcarbonyloxy-(C₁-C₇)-alkyl, (C₃-C₇)-Cycloalkylcarbonyloxy-(C₁-C₇)-alkyl, Arylcarbonyloxy-(C₁-C₇)-alkyl, Heteroarylcarbonyloxy-(C₁-C₇)-alkyl, Heterocyclylcarbonyloxy-(C₁-C₇)-alkyl, Aryloxy-(C₁-C₇)-alkyl, Heteroaryloxy-(C₁-C₇)-alkyl, (C₁-C₇)-Alkoxycarbonyl steht
und
- R¹⁴: für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₃-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, Heterocyclyl-(C₁-C₇)-alkyl, (C₃-C₇)-Cycloalkyl-(C₁-C₇)-alkyl, (C₄-C₁₀)-Cycloalkenyl-(C₁-C₇)-alkyl, Bis-[(C₁-C₇)-alkyl]amino, (C₁-C₇)-Alkyl-amino, Aryl-(C₁-C₇)-amino, Aryl-(C₁-C₆)-alkyl-amino, Aryl-[(C₁-C₇)-alkyl]amino; (C₃-C₇)-Cycloalkyl-amino, (C₃-C₇)-Cycloalkyl-[(C₁-C₇)-alkyl]amino; N-Azetidinyl, N-Pyrrolidinyl, N-Piperidinyl. N-Morpholinyl, steht.

Besonders bevorzugter Erfindungsgegenstand sind Verbindungen der allgemeinen Formel (I), worin
- R¹: für Wasserstoff steht,
- R²: für Wasserstoff, Fluor, Chlor, Brom steht,
- R³: für (C₁-C₄)-Alkyl steht,
- G: für einen Rest der nachfolgenden Formel 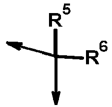 steht,
- R⁵ und R⁶: unabhängig voneinander für Wasserstoff, (C₁-C₆)-Alkyl stehen, oder
- R⁴ und R⁶: mit dem Kohlenstoffatom, an das sie gebunden sind, einen vollständig gesättigten monocyclischen 3- bis 6-gliedrigen Carbocyclus bilden
- Q: für einen Rest der nachfolgenden Formeln 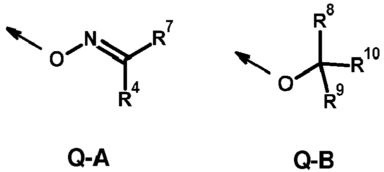 steht,
- R⁴ und R⁷: unabhängig voneinander für (C₁-C₇)-Alkyl, (C₃-C₇)-Cycloalkyl, Aryl stehen, oder
- R⁴ und R⁷: mit dem Kohlenstoffatom, an das sie gebunden sind, einen vollständig gesättigten monocyclischen 3- bis 6-gliedrigen Carbocyclus bilden,
- R⁸: für Wasserstoff, (C₁-C₆)-Alkyl, (C₁-C₆)-Haloalkyl, Aryl, Aryl-(C₁-C₆)-alkyl, Heteroaryl, (C₂-C₆)-Alkinyl, (C₂-C₆)-Alkenyl, C(O)R¹³, C(O)OR¹³, (C₁-C₆)-Alkoxy-(C₁-C₆)-alkyl steht,
- R⁹: für Wasserstoff oder (C₁-C₆)-Alkyl steht,
- R¹⁰: für Halogen, Cyano, NO₂, (C₁-C₆)-Haloalkyl, (C₃-C₆)-Cycloalkyl, (C₃-C₆)-Cycloalkyl-(C₁-C₆)-alkyl, (C₃-C₆)-Halocycloalkyl, (C₃-C₆)-Halocycloalkyl-(C₁-C₆)-alkyl, Aryl, Aryl-(C₁-C₆)-alkyl, Heteroaryl, Heteroaryl-(C₁-C₆)-alkyl, Heterocyclyl, Heterocyclyl-(C₁-C₆)-alkyl, R¹¹R¹²N-(C₁-C₆)-alkyl, R¹³O-(C₁-C₆)-alkyl, Cyano-(C₁-C₆)-alkyl, (C₁-C₆)-Alkylcarbonyloxy-(C₁-C₇)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₈)-alkyl, Heterocyclylcarbonyloxy-(C₁-C₇)-alkyl, OR¹³, NR¹¹R¹², SR¹⁴, S(O)R¹⁴, SO₂R¹⁴, R¹⁴S-(C₁-C₆)-alkyl, R¹⁴(O)S-(C₁-C₆)-alkyl, R¹⁴O₂S-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, Tris-[(C₁-C₆)-Alkyl]silyl, Bis-hydroxyboryl-(C₁-C₆)-alkyl, Bis-[(C₁-C₆)-alkoxy]boryl-(C₁-C₆)-alkyl, Tetramethyl-1,3,2-Dioxaborolan-2-yl, Tetramethyl-1,3,2-Dioxaborolan-2-yl-(C₁-C₆)-alkyl, Nitro-(C₁-C₆)-alkyl, C(O)OR¹³, C(O)R¹³, C(O)NR¹¹R¹², R¹³O(O)C-(C₁-C₆)-alkyl, R¹¹R¹³N(O)C-(C₁-C₈)-alkyl, Bis-(C₁-C₆)-alkoxy-(C₁-C₆)-alkyl steht,
- R¹¹ und R¹²: gleich oder verschieden sind und unabhängig voneinander für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, C(O)R¹³, SO₂R¹⁴, Heterocyclyl, (C₁-C₆)-Alkoxycarbonyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Aryl-(C₁-C₆)-alkoxycarbonyl, Heteroaryl-(C₁-C₆)-alkoxycarbonyl, (C₂-C₆)-Alkenyloxycarbonyl, (C₂-C₆)-Alkinyloxycarbonyl, Heterocyclyl-(C₁-C₆)-alkyl stehen, oder
- R¹¹ und R¹²: mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
- R¹³: für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkoxy-(C₁-C₆)-haloalkyl, (C₁-C₆)-Alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkoxy-(C₁-C₆)-alkyl, Aryl, Aryl-(C₁-C₆)-alkyl, Aryl-(C₁-C₆)-alkoxy-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Bis-[(C₁-C₆)-alkyl]amino-(C₂-C₆)-alkyl, (C₁-C₆)-Alkyl-amino-(C₂-C₆)-alkyl, Aryl-(C₁-C₆)-alkyl-amino-(C₂-C₆)-alkyl, R¹⁴S-(C₁-C₆)-alkyl, R¹⁴(O)S-(C₁-C₆)-alkyl, R¹⁴O₂S-(C₁-C₆)-alkyl, Hydroxycarbonyl-(C₁-C₆)-alkyl, Heterocyclyl, Heterocyclyl-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, (C₁-C₆)-Alkylcarbonyloxy-(C₁-C₆)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, Aryloxy-(C₁-C₆)-alkyl, Heteroaryloxy-(C₁-C₆)-alkyl, (C₁-C₆)-Alkoxycarbonyl steht
und
- R¹⁴: für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-Cycloalkyl-(C₁-C₆)-alkyl, (C₄-C₁₀)-Cycloalkenyl-(C₁-C₆)-alkyl, Bis-[(C₁-C₆)-alkyl]amino, (C₁-C₆)-Alkyl-amino, Aryl-(C₁-C₆)-amino, Aryl-(C₁-C₆)-alkyl-amino, Aryl-[(C₁-C₆)-alkyl]amino; (C₃-C₆)-Cycloalkyl-amino, (C₃-C₆)-Cycloalkyl-[(C₁-C₆)-alkyl]amino; N-Azetidinyl, N-Pyrrolidinyl, N-Piperidinyl. N-Morpholinyl steht.

Ganz besonders bevorzugter Erfindungsgegenstand sind Verbindungen der allgemeinen Formel (I), worin
- R¹: für Wasserstoff steht,
- R²: für Wasserstoff, Fluor, Chlor, steht,
- R³: für Methyl, Ethyl, Prop-1-yl, 1-Methylethyl, But-₁-yl, 1-Methylpropyl, 2-Methylpropyl steht,
- G: für einen Rest der nachfolgenden Formel 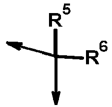 steht,
- R⁵ und R⁶: unabhängig voneinander für Wasserstoff, Methyl, Ethyl, Prop-1-yl, 1-Methylethyl, But-1-yl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-₁-methylpropyl, 1-Ethyl-2-methylpropyl stehen, oder

- R⁴ und R⁶: mit dem Kohlenstoffatom, an das sie gebunden sind, einen vollständig gesättigten monocyclischen 3- bis 6-gliedrigen Carbocyclus bilden,
und
- Q: für eine der nachfolgend spezifisch genannten Gruppierungen Q-1 bis Q-485 steht:

| | | | | |
|---|---|---|---|---|
| 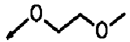 | 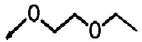 | 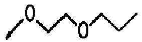 | 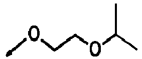 | 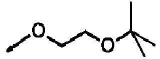 |
| Q-1 | Q-2 | Q-3 | Q-4 | Q-5 |
| 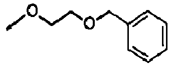 | 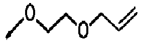 | 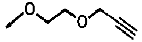 | 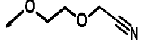 | 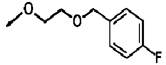 |
| Q-6 | Q-7 | Q-8 | Q-9 | Q-10 |
| 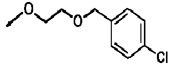 | 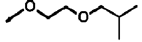 | 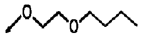 | 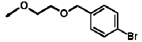 | 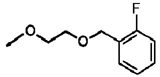 |
| Q-11 | Q-12 | Q-13 | Q-14 | Q-15 |
| 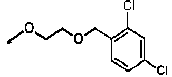 | 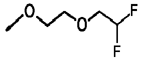 | 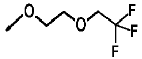 | 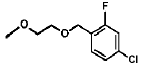 | 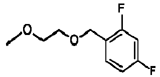 |
| Q-16 | Q-17 | Q-18 | Q-19 | Q-20 |
| 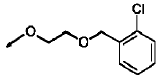 | 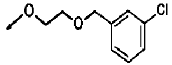 | 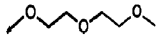 | 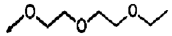 | 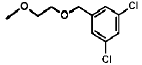 |
| Q-21 | Q-22 | Q-23 | Q-24 | Q-25 |
| 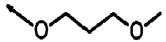 | 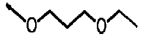 | 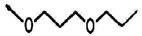 | 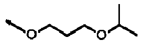 | 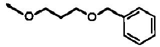 |
| Q-26 | Q-27 | Q-28 | Q-29 | Q-30 |
| 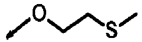 | 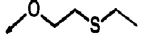 | 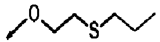 | 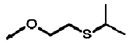 | 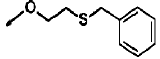 |
| Q-31 | Q-32 | Q-33 | Q-34 | Q-35 |
| 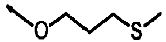 | 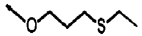 | 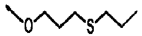 | 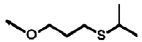 | 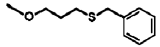 |
| Q-36 | Q-37 | Q-38 | Q-39 | Q-40 |
| 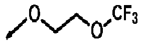 | 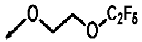 | 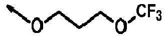 | 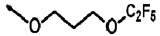 | 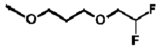 |
| Q-41 | Q-42 | Q-43 | Q-44 | Q-45 |
| 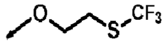 | 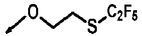 | 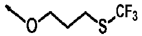 | 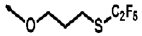 | 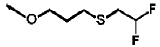 |
| Q-46 | Q-47 | Q-48 | Q-49 | Q-50 |
| 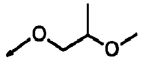 | 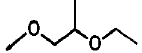 | 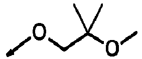 | 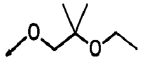 | 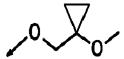 |
| Q-51 | Q-52 | Q-53 | Q-54 | Q-55 |
| 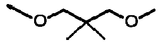 | 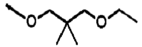 | 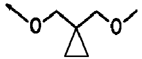 | 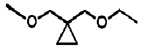 | 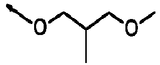 |
| Q-56 | Q-57 | Q-58 | Q-59 | Q-60 |
| 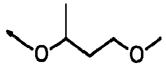 | 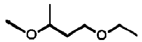 | 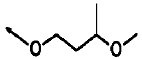 | 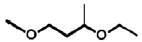 | 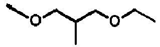 |
| Q-61 | Q-62 | Q-63 | Q-64 | Q-65 |
| 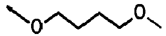 | 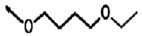 | 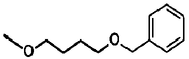 | 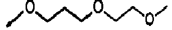 | 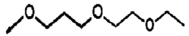 |
| Q-66 | Q-67 | Q-68 | Q-69 | Q-70 |
| 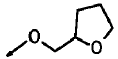 | 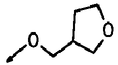 | 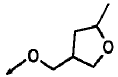 | 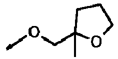 | 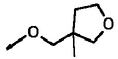 |
| Q-71 | Q-72 | Q-73 | Q-74 | Q-75 |
| 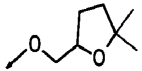 | 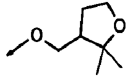 | 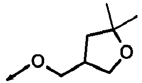 | 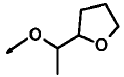 | 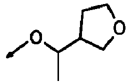 |
| Q-76 | Q-77 | Q-78 | Q-79 | Q-80 |
| 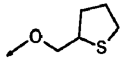 | 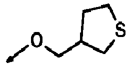 | 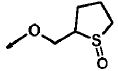 | 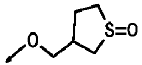 | 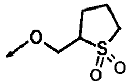 |
| Q-81 | Q-82 | Q-83 | Q-84 | Q-85 |
| 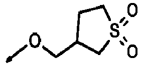 | 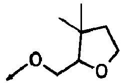 | 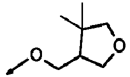 | 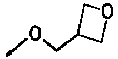 | 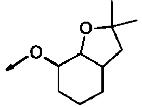 |
| Q-86 | Q-87 | Q-88 | Q-89 | Q-90 |
| 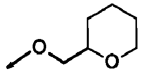 | 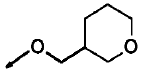 | 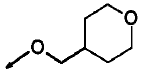 | 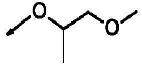 | 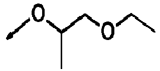 |
| Q-91 | Q-92 | Q-93 | Q-94 | Q-95 |
| 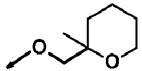 | 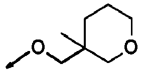 | 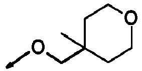 | 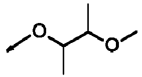 | 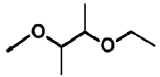 |
| Q-96 | Q-97 | Q-98 | Q-99 | Q-100 |
| 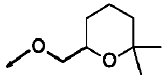 | 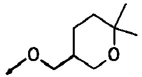 | 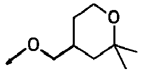 | 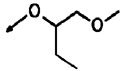 | 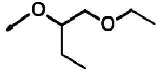 |
| Q-101 | Q-102 | Q-103 | Q-104 | Q-105 |
| 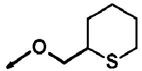 | 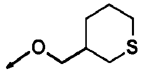 | 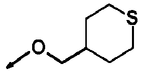 | 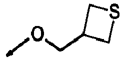 | 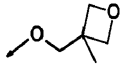 |
| Q-106 | Q-107 | Q-108 | Q-109 | Q-110 |
| 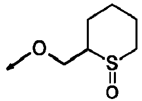 | 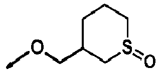 | 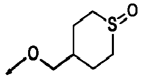 | 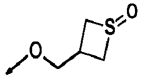 | 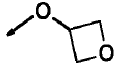 |
| Q-111 | Q-112 | Q-113 | Q-114 | Q-115 |
| 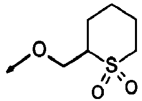 | 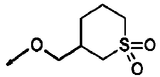 | 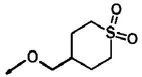 | 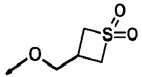 | 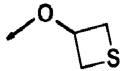 |
| Q-116 | Q-117 | Q-118 | Q-119 | Q-120 |
| 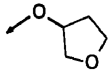 | 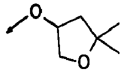 | 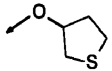 | 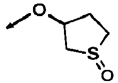 | 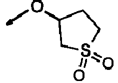 |
| Q-121 | Q-122 | Q-123 | Q-124 | Q-125 |
| 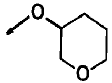 | 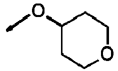 | 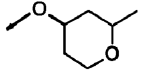 | 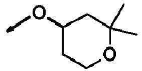 | 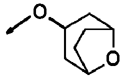 |
| Q-126 | Q-127 | Q-128 | Q-129 | Q-130 |
| 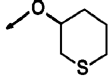 | 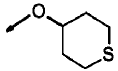 | 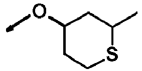 | 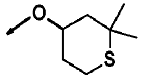 | 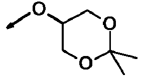 |
| Q-131 | Q-132 | Q-133 | Q-134 | Q-135 |
| 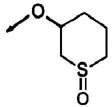 | 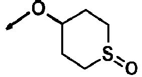 | 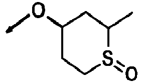 | 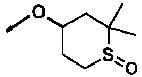 | 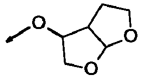 |
| Q-136 | Q-137 | Q-138 | Q-139 | Q-140 |
| 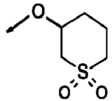 | 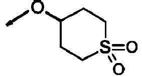 | 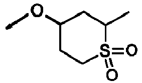 | 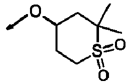 | 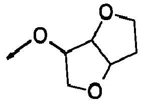 |
| Q-141 | Q-142 | Q-143 | Q-144 | Q-145 |
| 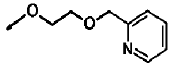 | 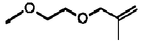 | 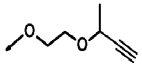 | 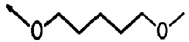 | 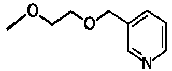 |
| Q-146 | Q-147 | Q-148 | Q-149 | Q-150 |
| 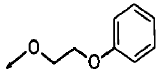 | 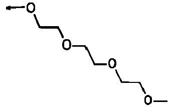 | 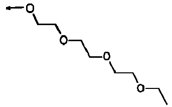 | 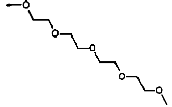 | 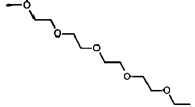 |
| Q-151 | Q-152 | Q-153 | Q-154 | Q-155 |
| 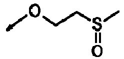 | 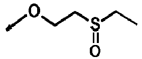 | 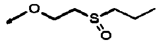 | 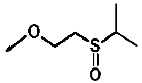 | 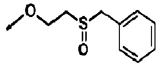 |
| Q-156 | Q-157 | Q-158 | Q-159 | Q-160 |
| 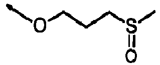 | 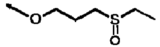 | 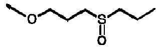 | 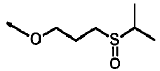 | 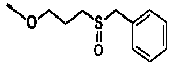 |
| Q-161 | Q-162 | Q-163 | Q-164 | Q-165 |
| 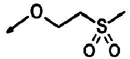 | 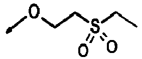 | 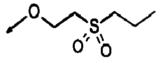 | 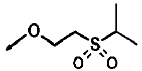 | 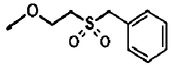 |
| Q-166 | Q-167 | Q-168 | Q-169 | Q-170 |
| 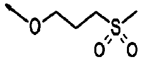 | 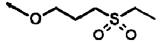 | 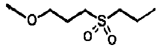 | 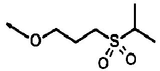 | 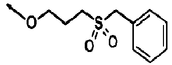 |
| Q-171 | Q-172 | Q-173 | Q-174 | Q-175 |
| 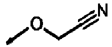 | 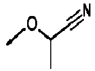 | 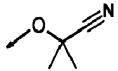 | 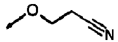 | 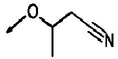 |
| Q-176 | Q-177 | Q-178 | Q-179 | Q-180 |
| 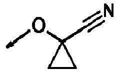 | 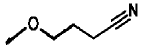 | 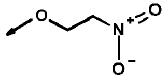 | 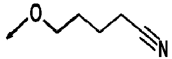 | 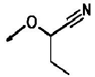 |
| Q-181 | Q-182 | Q-183 | Q-184 | Q-185 |
| 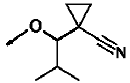 | 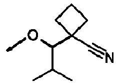 | 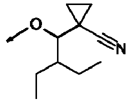 | 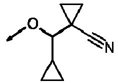 | 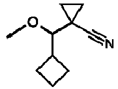 |
| Q-186 | Q-187 | Q-188 | Q-189 | Q-190 |
| 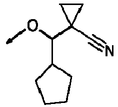 | 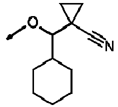 | 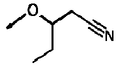 | 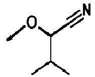 | 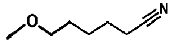 |
| Q-191 | Q-192 | Q-193 | Q-194 | Q-195 |
| 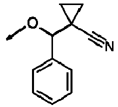 | 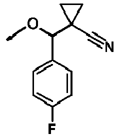 | 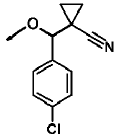 | 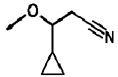 | 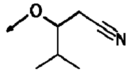 |
| Q-196 | Q-197 | Q-198 | Q-199 | Q-200 |
| 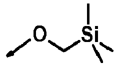 | 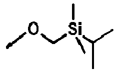 | 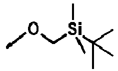 | 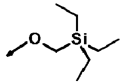 | 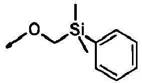 |
| Q-201 | Q-202 | Q-203 | Q-204 | Q-205 |
| 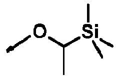 | 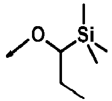 | 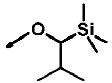 | 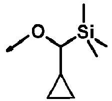 | 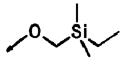 |
| Q-206 | Q-207 | Q-208 | Q-209 | Q-210 |
| 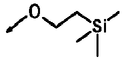 | 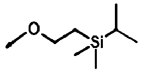 | 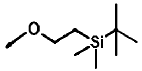 | 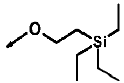 | 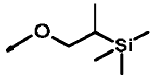 |
| Q-211 | Q-212 | Q-213 | Q-214 | Q-215 |
| 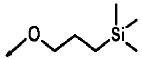 | 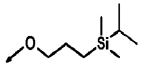 | 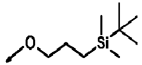 | 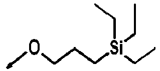 | 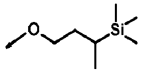 |
| Q-216 | Q-217 | Q-218 | Q-219 | Q-220 |
| 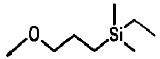 | 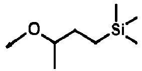 | 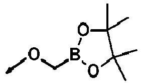 | 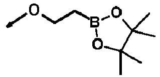 | 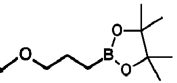 |
| Q-221 | Q-222 | Q-223 | Q-224 | Q-225 |
| 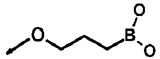 | 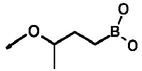 | 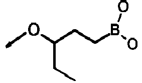 | 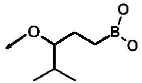 | 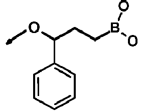 |
| Q-226 | Q-227 | Q-228 | Q-229 | Q-230 |
| 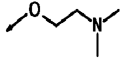 | 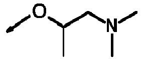 | 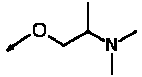 | 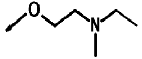 | 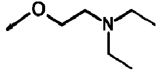 |
| Q-231 | Q-232 | Q-233 | Q-234 | Q-235 |
| 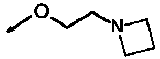 | 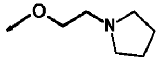 | 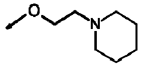 | 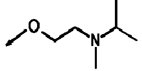 | 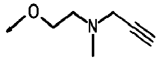 |
| Q-236 | Q-237 | Q-238 | Q-239 | Q-240 |
| 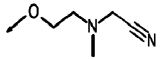 | 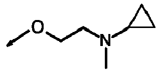 | 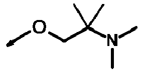 | 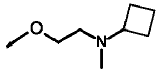 | 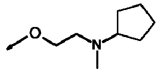 |
| Q-241 | Q-242 | Q-243 | Q-244 | Q-245 |
| 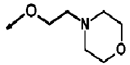 | 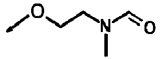 | 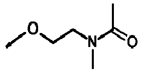 | 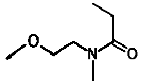 | 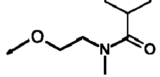 |
| Q-246 | Q-247 | Q-248 | Q-249 | Q-250 |
| 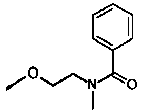 | 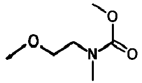 | 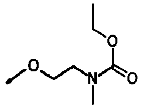 | 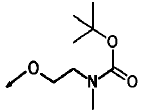 | 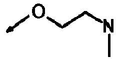 |
| Q-251 | Q-252 | Q-253 | Q-254 | Q-255 |
| 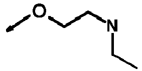 | 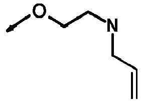 | 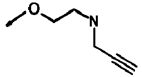 | 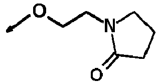 | 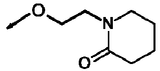 |
| Q-256 | Q-257 | Q-258 | Q-259 | Q-260 |
| 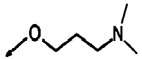 | 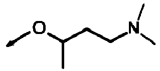 | 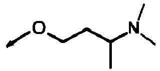 | 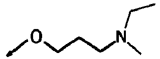 | 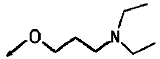 |
| Q-261 | Q-262 | Q-263 | Q-264 | Q-265 |
| 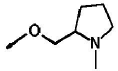 | 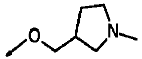 | 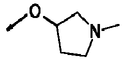 | 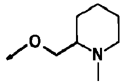 | 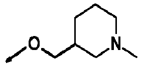 |
| Q-266 | Q-267 | Q-268 | Q-269 | Q-270 |
| 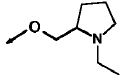 | 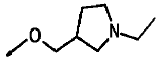 | 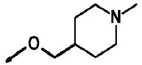 | 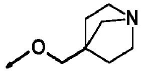 | 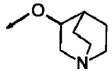 |
| Q-271 | Q-272 | Q-273 | Q-274 | Q-275 |
| 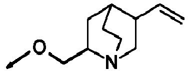 | 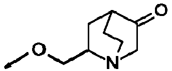 | 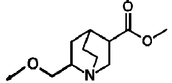 | 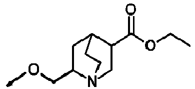 | 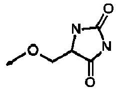 |
| Q-276 | Q-277 | Q-278 | Q-279 | Q-280 |
| 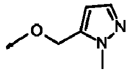 | 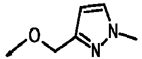 | 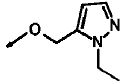 | 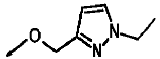 | 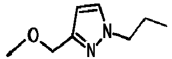 |
| Q-281 | Q-282 | Q-283 | Q-284 | Q-285 |
| 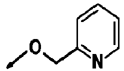 | 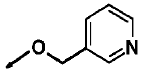 | 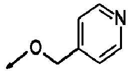 | 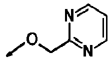 | 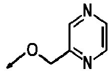 |
| Q-286 | Q-287 | Q-288 | Q-289 | Q-290 |
| 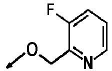 | 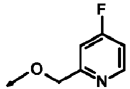 | 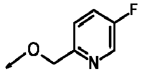 | 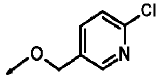 | 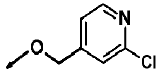 |
| Q-291 | Q-292 | Q-293 | Q-294 | Q-295 |
| 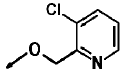 | 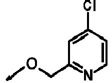 | 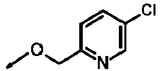 | 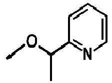 | 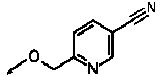 |
| Q-296 | Q-297 | Q-298 | Q-299 | Q-300 |
| 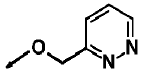 | 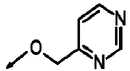 | 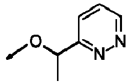 | 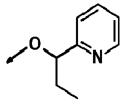 | 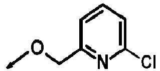 |
| Q-301 | Q-302 | Q-303 | Q-304 | Q-305 |
| 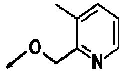 | 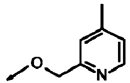 | 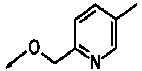 | 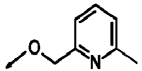 | 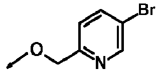 |
| Q-306 | Q-307 | Q-308 | Q-309 | Q-310 |
| 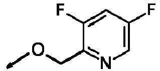 | 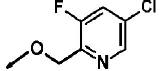 | 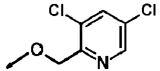 | 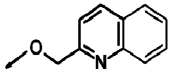 | 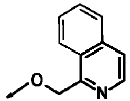 |
| Q-311 | Q-312 | Q-313 | Q-314 | Q-315 |
| 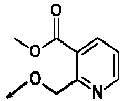 | 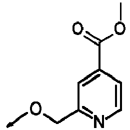 | 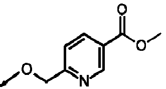 | 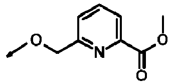 | 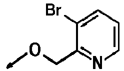 |
| Q-316 | Q-317 | Q-318 | Q-319 | Q-320 |
| 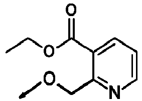 | 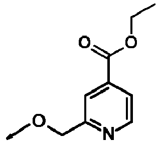 | 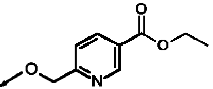 | 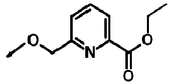 | 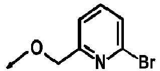 |
| Q-321 | Q-322 | Q-323 | Q-324 | Q-325 |
| 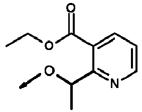 | 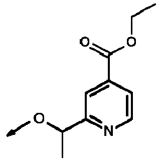 | 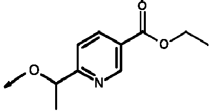 | 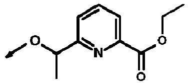 | 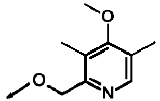 |
| Q-326 | Q-327 | Q-328 | Q-329 | Q-330 |
| 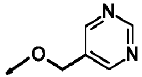 | 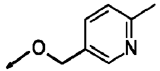 | 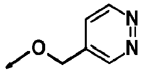 | 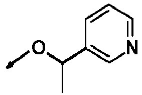 | 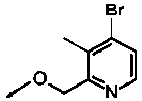 |
| Q-331 | Q-332 | Q-333 | Q-334 | Q-335 |
| 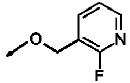 | 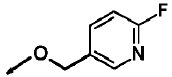 | 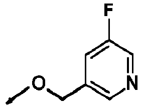 | 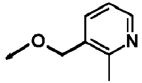 | 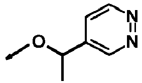 |
| Q-336 | Q-337 | Q-338 | Q-339 | Q-340 |
| 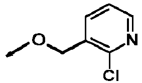 | 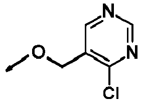 | 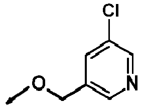 | 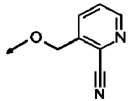 | 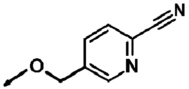 |
| Q-341 | Q-342 | Q-343 | Q-344 | Q-345 |
| 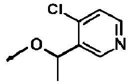 | 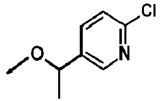 | 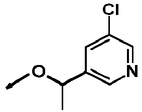 | 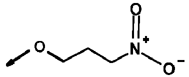 | 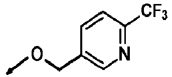 |
| Q-346 | Q-347 | Q-348 | Q-349 | Q-350 |
| 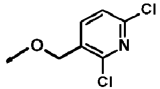 | 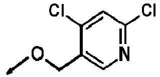 | 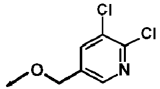 | 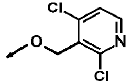 | 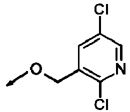 |
| Q-351 | Q-352 | Q-353 | Q-354 | Q-355 |
| 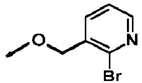 | 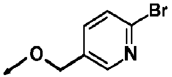 | 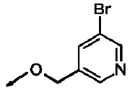 | 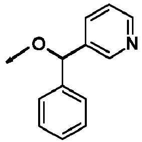 | 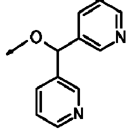 |
| Q-356 | Q-357 | Q-358 | Q-359 | Q-360 |
| 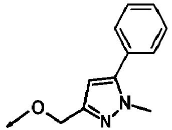 | 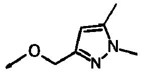 | 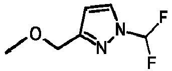 | 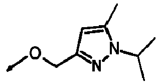 | 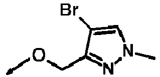 |
| Q-361 | Q-362 | Q-363 | Q-364 | Q-365 |
| 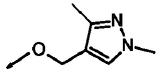 | 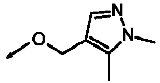 | 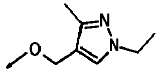 | 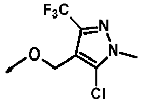 | 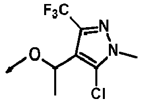 |
| Q-366 | Q-367 | Q-368 | Q-369 | Q-370 |
| 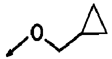 | 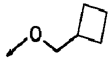 | 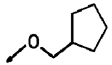 | 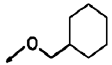 | 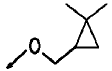 |
| Q-371 | Q-372 | Q-373 | Q-374 | Q-375 |
| 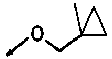 | 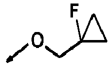 | 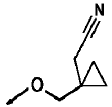 | 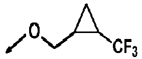 | 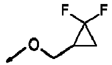 |
| Q-376 | Q-377 | Q-378 | Q-379 | Q-380 |
| 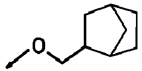 | 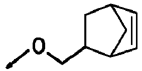 | 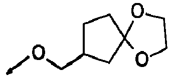 | 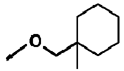 | 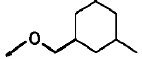 |
| Q-381 | Q-382 | Q-383 | Q-384 | Q-385 |
| 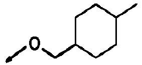 | 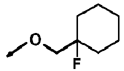 | 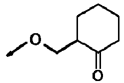 | 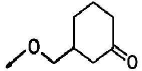 | 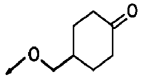 |
| Q-386 | Q-387 | Q-388 | Q-389 | Q-390 |
| 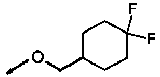 | 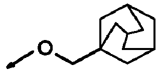 | 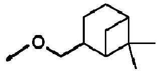 | 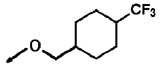 | 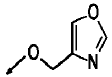 |
| Q-391 | Q-392 | Q-393 | Q-394 | Q-395 |
| 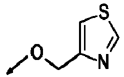 | 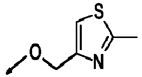 | 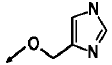 | 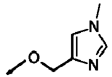 | 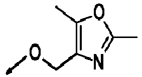 |
| Q-396 | Q-397 | Q-398 | Q-399 | Q-400 |
| 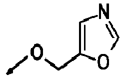 | 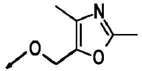 | 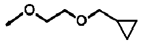 | 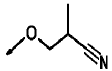 | 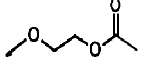 |
| Q-401 | Q-402 | Q-403 | Q-404 | Q-405 |
| 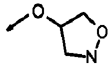 | 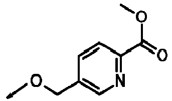 | 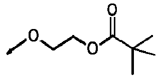 | 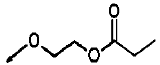 | 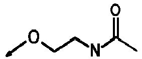 |
| Q-406 | Q-407 | Q-408 | Q-409 | Q-410 |
| 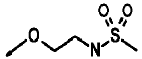 | 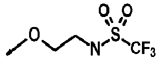 | 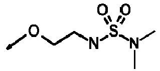 | 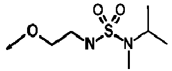 | 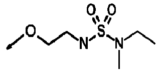 |
| Q-411 | Q-412 | Q-413 | Q-414 | Q-415 |
| 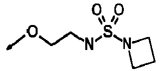 | 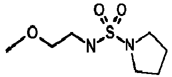 | 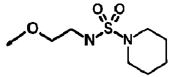 | 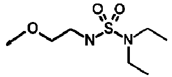 | 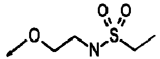 |
| Q-416 | Q-417 | Q-418 | Q-419 | Q-420 |
| 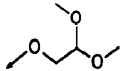 | 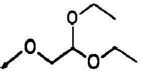 | 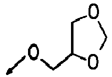 | 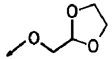 | 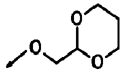 |
| Q-421 | Q-422 | Q-423 | Q-424 | Q-425 |
| 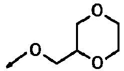 | 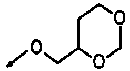 | 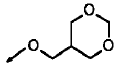 | 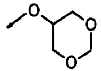 | 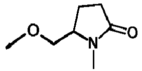 |
| Q-426 | Q-427 | Q-428 | Q-429 | Q-430 |
| 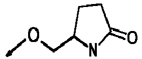 | 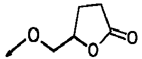 | 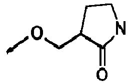 | 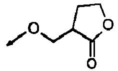 | 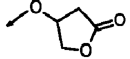 |
| Q-431 | Q-432 | Q-433 | Q-434 | Q-435 |
| 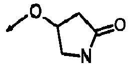 | 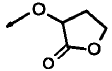 | 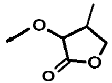 | 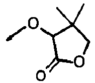 | 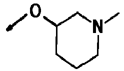 |
| Q-436 | Q-437 | Q-438 | Q-439 | Q-440 |
| 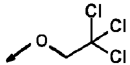 | 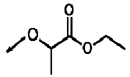 | 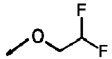 | 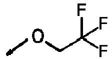 | 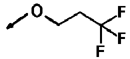 |
| Q-441 | Q-442 | Q-443 | Q-444 | Q-445 |
| 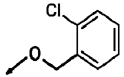 | 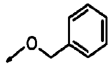 | 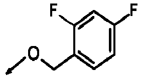 | 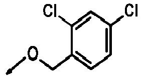 | 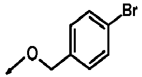 |
| Q-446 | Q-447 | Q-448 | Q-449 | Q-450 |
| 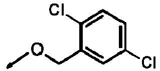 | 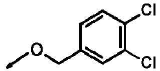 | 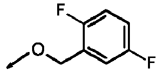 | 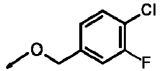 | 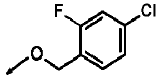 |
| Q-451 | Q-452 | Q-453 | Q-454 | Q-455 |
| 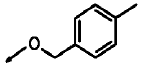 | 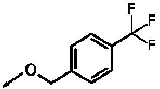 | 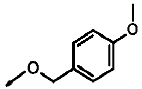 | 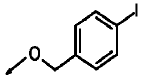 | 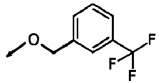 |
| Q-456 | Q-457 | Q-458 | Q-459 | Q-460 |
| 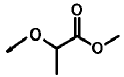 | 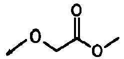 | 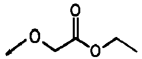 | 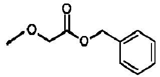 | 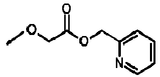 |
| Q-461 | Q-462 | Q-463 | Q-464 | Q-465 |
| 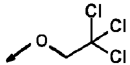 | 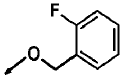 | 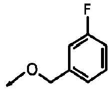 | 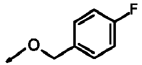 | 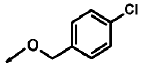 |
| Q-466 | Q-467 | Q-468 | Q-469 | Q-470 |
| 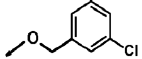 | 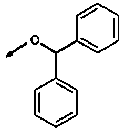 | 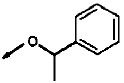 | 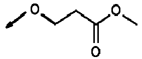 | 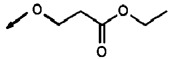 |
| Q-471 | Q-472 | Q-473 | Q-473 | Q-475 |
| 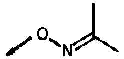 | 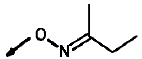 | 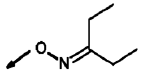 | 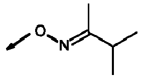 | 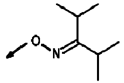 |
| Q-476 | Q-477 | Q-478 | Q-479 | Q-480 |
| 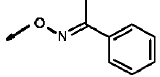 | 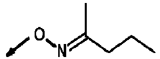 | 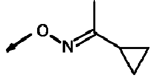 | 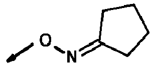 | 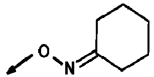 |
| Q-481 | Q-482 | Q-483 | Q-484 | Q-485 |

Im Speziellen bevorzugter Erfindungsgegenstand sind Verbindungen der allgemeinen Formel (I), worin
- R¹: für Wasserstoff steht,
- R²: für Wasserstoff, Fluor, Chlor, steht,
- R³: für Methyl steht,
- G: für einen Rest der nachfolgenden Formel 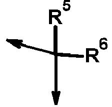 steht,
- R⁵ und R⁶: unabhängig voneinander für Wasserstoff, Methyl, Ethyl, bevorzugt Methyl stehen, oder
- R⁴ und R⁶: mit dem Kohlenstoffatom, an das sie gebunden sind, einen vollständig gesättigten monocyclischen 3- bis 6-gliedrigen Carbocyclus bilden,
- und Q: für eine der zuvor spezifisch genannten Gruppierungen Q-1 bis Q-485 steht:

Im ganz Speziellen bevorzugter Erfindungsgegenstand sind Verbindungen der allgemeinen Formel (I), worin
- R¹: für Wasserstoff steht,
- R²: für Wasserstoff, Fluor, Chlor, steht,
- R³: für Methyl steht,
- G: für einen Rest der nachfolgenden Formel 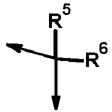 steht,
- R⁵ und R⁶: für Methyl stehen
und
- Q: für eine der zuvor genannten Gruppierungen Q-1, Q-2, Q-41, Q-176, Q-286, Q-301, Q-444, Q-476 steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen, beliebig kombiniert werden.

Im Hinblick auf die erfindungsgemäßen Verbindungen werden die vorstehend und weiter unten verwendeten Bezeichnungen erläutert. Diese sind dem Fachmann geläufig und haben insbesondere die im Folgenden erläuterten Bedeutungen:
Sofern nicht anders definiert, gilt generell für die Bezeichnung von chemischen Gruppen, dass die Anbindung an das Gerüst bzw. den Rest des Moleküls über das zuletzt genannte Strukturelement der betreffenden chemischen Gruppe erfolgt, d.h. beispielsweise im Falle von (C₂-C₈)-Alkenyloxy über das Sauerstoffatom, und im Falle von Heterocyclyl-(C₁-C₈)-alkyl oder R¹³O(O)C-(C₁-C₈)-Alkyl jeweils über das C-Atom der Alkylgruppe. In einer zusammengesetzten chemischen Gruppe wie z. B. Heterocyclyl-(C₁-C₈)-alkyl oder R¹³O(O)C-(C₁-C₈)-Alkyl steht die Bezeichnung "Alkyl" daher auch für eine Alkylengruppe. Bei den funktionellen Gruppen C(=O)R¹³, C(=O)OR¹³, C(=O)NR¹¹R¹² , NR¹¹R¹² , OR¹³, S(O)ₘR¹⁴ erfolgt die Anbindung an das Gerüst bzw. den Rest des Moleküls über das zuerst genannte Strukturelement der betreffenden chemischen Gruppe.

Erfindungsgemäß steht "Alkylsulfonyl" - in Alleinstellung oder als Bestandteil einer chemischen Gruppe - für geradkettiges oder verzweigtes Alkylsulfonyl, vorzugsweise mit 1 bis 8, oder mit 1 bis 6 Kohlenstoffatomen, z.B. (aber nicht beschränkt auf) (C₁-C₆)-Alkylsulfonyl wie Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1 -Ethyl-1-methylpropylsulfonyl und 1-Ethyl-2-methylpropylsulfonyl.

Erfindungsgemäß steht "Heteroarylsulfonyl" für gegebenenfalls substituiertes Pyridylsulfonyl, Pyrimidinylsulfonyl, Pyrazinylsulfonyl oder gegebenenfalls substituiertes polycyclisches Heteroarylsulfonyl, hier insbesondere gegebenenfalls substituiertes Chinolinylsulfonyl, beispielsweise substituiert durch Fluor, Chlor, Brom, Iod, Cyano, Nitro, Alkyl-, Haloalkyl-, Haloalkoxy-, Amino-, Alkylamino-, Alkylcarbonylamino-, Dialkylamino- oder Alkoxygruppen.

Erfindungsgemäß steht "Alkylthio" - in Alleinstellung oder als Bestandteil einer chemischen Gruppe - für geradkettiges oder verzweigtes S-Alkyl, vorzugsweise mit 1 bis 8, oder mit 1 bis 6 Kohlenstoffatomen, wie (C₁-C₁₀)-, (C₁-C₆)- oder (C₁-C₄)-Alkylthio, z.B. (aber nicht beschränkt auf) (C₁-C₆)-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 1,1-Dimethylpropylthio, 1,2-Dimethylpropylthio, 2,2-Dimethylpropylthio, 1-Ethylpropylthio, Hexylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio.

"Alkenylthio" bedeutet erfindungsgemäß ein über ein Schwefelatom gebundenen Alkenylrest, Alkinylthio bedeutet ein über ein Schwefelatom gebundenen Alkinylrest, Cycloalkylthio bedeutet ein über ein Schwefelatom gebundenen Cycloalkylrest und Cycloalkenylthio bedeutet ein über ein Schwefelatom gebundenen Cycloalkenylrest.

"Alkylsulfinyl (Alkyl-S(=O)-)", soweit nicht an anderer Stelle anders definiert steht erfindungsgemäß für Alkylreste, die über -S(=O)- an das Gerüst gebunden sind, wie (C₁-C₁₀)-, (C₁-C₆)- oder (C₁-C₄)-Alkylsulfinyl, z. B. (aber nicht beschränkt auf) (C₁-C₆)-Alkylsulfinyl wie Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 1,1-Dimethylpropylsulfinyl, 1,2-Dimethylpropylsulfinyl, 2,2-Dimethylpropylsulfinyl, 1-Ethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl.

Analog sind "Alkenylsulfinyl" und "Alkinylsulfinyl", erfindungsgemäß definiert als Alkenyl- bzw. Alkinylreste, die über -S(=O)- an das Gerüst gebunden sind, wie (C₂-C₁₀)-, (C₂-C₆)- oder (C₂-C₄)-Alkenylsulfinyl bzw. (C₃-C₁₀)-, (C₃-C₆)- oder (C₃-C₄)-Alkinylsulfinyl.

Analog sind "Alkenylsulfonyl" und "Alkinylsulfonyl" erfindungsgemäß definiert als Alkenyl- bzw. Alkinylreste, die über -S(=O)₂- an das Gerüst gebunden sind, wie (C₂-C₁₀)-, (C₂-C₆)- oder (C₂-C₄)-Alkenylsulfonyl bzw. (C₃-C₁₀)-, (C₃-C₆)- oder (C₃-C₄)-Alkinylsulfonyl.

"Alkoxy" bedeutet ein über ein Sauerstoffatom gebundenen Alkylrest, z. B. (aber nicht beschränkt auf) (C₁-C₆)-Alkoxy wie Methoxy, Ethoxy, Propoxy, 1-Methylethoxy, Butoxy, 1-Methylpropoxy, 2-Methylpropoxy, 1,1-Dimethylethoxy, Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy. Alkenyloxy bedeutet ein über ein Sauerstoffatom gebundenen Alkenylrest, Alkinyloxy bedeutet ein über ein Sauerstoffatom gebundenen Alkinylrest wie (C₂-C₁₀)-, (C₂-C₆)- oder (C₂-C₄)-Alkenoxy bzw. (C₃-C₁₀)-, (C₃-C₆)- oder (C₃-C₄)-Alkinoxy.

"Cycloalkyloxy" bedeutet ein über ein Sauerstoffatom gebundenen Cycloalkylrest und Cycloalkenyloxy bedeutet ein über ein Sauerstoffatom gebundenen Cycloalkenylrest.

"Alkylcarbonyl" (Alkyl-C(=O)-), soweit nicht an anderer Stelle anders definiert, steht erfindungsgemäß für Alkylreste, die über -C(=O)- an das Gerüst gebunden sind, wie (C₁-C₁₀)-, (C₁-C₆)- oder (C₁-C₄)-Alkylcarbonyl. Die Anzahl der C-Atome bezieht sich dabei auf den Alkylrest in der Alkylcarbonylgruppe.

Analog stehen "Alkenylcarbonyl" und "Alkinylcarbonyl", soweit nicht an anderer Stelle anders definiert, erfindungsgemäß für Alkenyl- bzw. Alkinylreste, die über -C(=O)- an das Gerüst gebunden sind, wie (C₂-C₁₀)-, (C₂-C₆)- oder (C₂-C₄)-Alkenylcarbonyl bzw. (C₂-C₁₀)-, (C₂-C₆)- oder (C₂-C₄)-Alkinylcarbonyl. Die Anzahl der C-Atome bezieht sich dabei auf den Alkenyl- bzw. Alkinylrest in der Alkenyl- bzw. Alkinylcarbonylgruppe.

"Alkoxycarbonyl (Alkyl-O-C(=O)-)", soweit nicht an anderer Stelle anders definiert: Alkylreste, die über -O-C(=O)- an das Gerüst gebunden sind, wie (C₁-C₁₀)-, (C₁-C₆)- oder (C₁-C₄)-Alkoxycarbonyl. Die Anzahl der C-Atome bezieht sich dabei auf den Alkylrest in der Alkoxycarbonylgruppe. Analog stehen "Alkenyloxycarbonyl" und "Alkinyloxycarbonyl", soweit nicht an anderer Stelle anders definiert, erfindungsgemäß für Alkenyl- bzw. Alkinylreste, die über -O-C(=O)- an das Gerüst gebunden sind, wie (C₂-C₁₀)-, (C₂-C₆)- oder (C₂-C₄)-Alkenyloxycarbonyl bzw. (C₃-C₁₀)-, (C₃-C₆)- oder (C₃-C₄)-Alkinyloxycarbonyl. Die Anzahl der C-Atome bezieht sich dabei auf den Alkenyl- bzw. Alkinylrest in der Alken- bzw. Alkinyloxycarbonylgruppe.

Der Begriff "Alkylcarbonyloxy" (Alkyl-C(=O)-O-) steht erfindungsgemäß, soweit nicht an anderer Stelle anders definiert, für Alkylreste, die über eine Carbonyloxygruppe (-C(=O)-O-) mit dem Sauerstoff an das Gerüst gebunden sind, wie (C₁-C₁₀)-, (C₁-C₆)- oder (C₁-C₄)-Alkylcarbonyloxy. Die Anzahl der C-Atome bezieht sich dabei auf den Alkylrest in der Alkylcarbonyloxygruppe.

Analog sind "Alkenylcarbonyloxy" und "Alkinylcarbonyloxy" erfindungsgemäß definiert als Alkenyl- bzw. Alkinylreste, die über (-C(=O)-O-) mit dem Sauerstoff an das Gerüst gebunden sind, wie (C₂-C₁₀)-, (C₂-C₆)- oder (C₂-C₄)-Alkenylcarbonyloxy bzw. (C₂-C₁₀)-, (C₂-C₆)- oder (C₂-C₄)-Alkinylcarbonyloxy. Die Anzahl der C-Atome bezieht sich dabei auf den Alkenyl- bzw. Alkinylrest in der Alkenyl- bzw. Alkinylcarbonyloxygruppe.

In Kurzformen wie z.B. C(O)R¹³ , C(O)OR¹³, OC(O)NR¹¹R¹² , oder C(O)NR¹¹R¹² steht die in Klammern aufgeführte Kurzform O für ein über eine Doppelbindung an das benachbarte Kohlenstoffatom gebundenes Sauerstoffatom.

In Kurzformen wie z.B. OC(S)OR¹³, OC(S)SR¹⁴, OC(S)NR¹¹R¹², steht die in Klammern aufgeführte Kurzform S für ein über eine Doppelbindung an das benachbarte Kohlenstoffatom gebundenes Schwefelatom.

Der Begriff "Aryl" bedeutet ein gegebenenfalls substituiertes mono-, bi- oder polycyclisches aromatisches System mit vorzugsweise 6 bis 14, insbesondere 6 bis 10 Ring-C-Atomen, beispielsweise Phenyl, Naphthyl, Anthryl, Phenanthrenyl, und ähnliches, vorzugsweise Phenyl.

Vom Begriff "gegebenenfalls substituiertes Aryl" sind auch mehrcyclische Systeme, wie Tetrahydronaphtyl, Indenyl, Indanyl, Fluorenyl, Biphenylyl, umfasst, wobei die Bindungsstelle am aromatischen System ist. Von der Systematik her ist "Aryl" in der Regel auch von dem Begriff "gegebenenfalls substituiertes Phenyl" umfasst. Bevorzugte Aryl-Substituenten sind hier zum Beispiel Wasserstoff, Halogen, Alkyl, Cycloalkyl, Cycloalkylalkyl, Cycloalkenyl, Halocycloalkyl, Alkenyl, Alkinyl, Aryl, Arylalkyl, Arylalkenyl, Heteroaryl, Heteroarylalkyl, Heterocyclyl, Heterocyclylalkyl, Alkoxyalkyl, Alkylthio, Haloalkylthio, Haloalkyl, Alkoxy, Haloalkoxy, Cycloalkoxy, Cycloalkylalkoxy, Aryloxy, Heteroraryloxy, Alkoxyalkoxy, Alkinylalkoxy, Alkenyloxy, Bis-alkylaminoalkoxy, Tris-[alkyl]silyl, Bis-[alkyl]arylsilyl, Bis-[alkyl]alkylsilyl, Tris-[alkyl]silylalkinyl, Arylalkinyl, Heteroarylalkinyl, Alkylalkinyl, Cycloalkylalkinyl, Haloalkylalkinyl, Heterocyclyl-N-alkoxy, Nitro, Cyano, Amino, Alkylamino, Bis-alkylamino, Alkylcarbonylamino, Cycloalkylcarbonylamino, Arylcarbonylamino, Alkoxycarbonylamino, Alkoxycarbonylalkylamino, Arylalkoxycarbonylalkylamino, Hydroxycarbonyl, Alkoxycarbonyl, Aminocarbonyl, Alkylaminocarbonyl, Cycloalkylaminocarbonyl, Bis-Alkylaminocarbonyl, Heteroarylalkoxy, Arylalkoxy.

Ein heterocyclischer Rest (Heterocyclyl) enthält mindestens einen heterocyclischen Ring (=carbocyclischer Ring, in dem mindestens ein C-Atom durch ein Heteroatom ersetzt ist, vorzugsweise durch ein Heteroatom aus der Gruppe N, O, S, P) der gesättigt, ungesättigt, teilgesättigt oder heteroaromatisch ist und dabei unsubstituiert oder substituiert sein kann, wobei die Bindungsstelle an einem Ringatom lokalisiert ist. Ist der Heterocyclylrest oder der heterocyclische Ring gegebenenfalls substituiert, kann er mit anderen carbocyclischen oder heterocyclischen Ringen annelliert sein. Im Falle von gegebenenfalls substituiertem Heterocyclyl werden auch mehrcyclische Systeme umfasst, wie beispielsweise 8-Aza-bicyclo[3.2.1]octanyl, 8-Aza-bicyclo[2.2.2]octanyl oder 1-Aza-bicyclo[2.2.1]heptyl. Im Falle von gegebenenfalls substituiertem Heterocyclyl werden auch spirocyclische Systeme umfasst, wie beispielsweise 1-Oxa-5-aza-spiro[2.3]hexyl. Wenn nicht anders definiert, enthält der heterocyclische Ring vorzugsweise 3 bis 9 Ringatome, insbesondere 3 bis 6 Ringatome, und ein oder mehrere, vorzugsweise 1 bis 4, insbesondere 1, 2 oder 3 Heteroatome im heterocyclischen Ring, vorzugsweise aus der Gruppe N, O, und S, wobei jedoch nicht zwei Sauerstoffatome direkt benachbart sein sollen, wie beispielsweise mit einem Heteroatom aus der Gruppe N, O und S 1- oder 2- oder 3-Pyrrolidinyl, 3,4-Dihydro-2H-pyrrol-2- oder 3-yl, 2,3-Dihydro-1H-pyrrol-1- oder 2- oder 3- oder 4- oder 5-yl; 2,5-Dihydro-1H-pyrrol-1- oder 2- oder 3-yl, 1- oder 2- oder 3- oder 4-Piperidinyl; 2,3,4,5-Tetrahydropyridin-2- oder 3- oder 4- oder 5-yl oder 6-yl; 1,2,3,6-Tetrahydropyridin-1- oder 2- oder 3- oder 4- oder 5- oder 6-yl; 1,2,3,4-Tetrahydropyridin-1- oder 2-oder 3- oder 4- oder 5- oder 6-yl; 1,4-Dihydropyridin-1- oder 2- oder 3- oder 4-yl; 2,3-Dihydropyridin-2- oder 3- oder 4- oder 5- oder 6-yl; 2,5-Dihydropyridin-2- oder 3- oder 4- oder 5- oder 6-yl, 1- oder 2-oder 3- oder 4-Azepanyl; 2,3,4,5-Tetrahydro-1H-azepin-1- oder 2- oder 3- oder 4- oder 5- oder 6- oder 7-yl; 2,3,4,7-Tetrahydro-1H-azepin-1- oder 2- oder 3- oder 4- oder 5- oder 6- oder 7-yl; 2,3,6,7-Tetrahydro-1H-azepin-1- oder 2- oder 3- oder 4-yl; 3,4,5,6-Tetrahydro-2H-azepin-2- oder 3- oder 4-oder 5- oder 6- oder 7-yl; 4,5-Dihydro-1H-azepin-1- oder 2- oder 3- oder 4-yl; 2,5-Dihydro-1H-azepin-1- oder -2- oder 3- oder 4- oder 5- oder 6- oder 7-yl; 2,7-Dihydro-1H-azepin-1- oder -2- oder 3- oder 4-yl; 2,3-Dihydro-1H-azepin-1- oder -2- oder 3- oder 4- oder 5- oder 6- oder 7-yl; 3,4-Dihydro-2H-azepin-2- oder 3- oder 4- oder 5- oder 6- oder 7-yl; 3,6-Dihydro-2H-azepin-2- oder 3- oder 4- oder 5- oder 6-oder 7-yl; 5,6-Dihydro-2H-azepin-2- oder 3- oder 4- oder 5- oder 6- oder 7-yl; 4,5-Dihydro-3H-azepin-2- oder 3- oder 4- oder 5- oder 6- oder 7-yl; 1H-Azepin-1- oder -2- oder 3- oder 4- oder 5- oder 6- oder 7-yl; 2H-Azepin-2- oder 3- oder 4- oder 5- oder 6- oder 7-yl; 3H-Azepin-2- oder 3- oder 4- oder 5- oder 6- oder 7-yl; 4H-Azepin-2- oder 3- oder 4- oder 5- oder 6- oder 7-yl, 2- oder 3-Oxolanyl (= 2- oder 3-Tetrahydrofuranyl); 2,3-Dihydrofuran-2- oder 3- oder 4- oder 5-yl; 2,5-Dihydrofuran-2- oder 3-yl, 2-oder 3- oder 4-Oxanyl (= 2- oder 3- oder 4-Tetrahydropyranyl); 3,4-Dihydro-2H-pyran-2- oder 3- oder 4- oder 5- oder 6-yl; 3,6-Dihydro-2H-pyran-2- oder 3-oder 4- oder 5- oder 6-yl; 2H-Pyran-2- oder 3-oder 4- oder 5- oder 6-yl; 4H-Pyran-2- oder 3- oder 4-yl, 2- oder 3- oder 4-Oxepanyl; 2,3,4,5-Tetrahydrooxepin-2- oder 3- oder 4- oder 5- oder 6- oder 7-yl; 2,3,4,7-Tetrahydrooxepin-2- oder 3- oder 4- oder 5- oder 6- oder 7-yl; 2,3,6,7-Tetrahydrooxepin-2- oder 3- oder 4-yl; 2,3-Dihydrooxepin-2- oder 3- oder 4- oder 5- oder 6- oder 7-yl; 4,5-Dihydrooxepin-2- oder 3- oder 4-yl; 2,5-Dihydrooxepin-2- oder 3- oder 4- oder 5- oder 6- oder 7-yl; Oxepin-2- oder 3- oder 4- oder 5- oder 6- oder 7-yl; 2- oder 3-Tetrahydrothiophenyl; 2,3-Dihydrothiophen-2- oder 3- oder 4- oder 5-yl; 2,5-Dihydrothiophen-2- oder 3-yl; Tetrahydro-2H-thiopyran-2- oder 3- oder 4-yl; 3,4-Dihydro-2H-thiopyran-2- oder 3- oder 4- oder 5- oder 6-yl; 3,6-Dihydro-2H-thiopyran-2- oder 3- oder 4- oder 5- oder 6-yl; 2H-Thiopyran-2- oder 3-oder 4- oder 5- oder 6-yl; 4H-Thiopyran-2- oder 3- oder 4-yl. Bevorzugte 3-Ring und 4-Ring-Heterocyclen sind beispielsweise 1- oder 2-Aziridinyl, Oxiranyl, Thiiranyl, 1- oder 2- oder 3-Azetidinyl, 2- oder 3-Oxetanyl, 2- oder 3-Thietanyl, 1,3-Dioxetan-2-yl. Weitere Beispiele für "Heterocyclyl" sind ein partiell oder vollständig hydrierter heterocyclischer Rest mit zwei Heteroatomen aus der Gruppe N, O und S, wie beispielsweise 1- oder 2- oder 3- oder 4-Pyrazolidinyl; 4,5-Dihydro-3H-pyrazol- 3- oder 4-oder 5-yl; 4,5-Dihydro-1H-pyrazol-1- oder 3- oder 4- oder 5-yl; 2,3-Dihydro-1H-pyrazol-1- oder 2- oder 3- oder 4- oder 5-yl; 1- oder 2- oder 3- oder 4- Imidazolidinyl; 2,3-Dihydro-1H-imidazol-1- oder 2- oder 3- oder 4-yl; 2,5-Dihydro-1H-imidazol-1- oder 2- oder 4- oder 5-yl; 4,5-Dihydro-1H-imidazol-1- oder 2-oder 4- oder 5-yl; Hexahydropyridazin-1- oder 2- oder 3- oder 4-yl; 1,2,3,4-Tetrahydropyridazin-1- oder 2- oder 3- oder 4- oder 5- oder 6-yl; 1,2,3,6-Tetrahydropyridazin-1- oder 2- oder 3- oder 4- oder 5- oder 6-yl; 1,4,5,6-Tetrahydropyridazin-1- oder 3- oder 4- oder 5- oder 6-yl; 3,4,5,6-Tetrahydropyridazin-3-oder 4- oder 5-yl; 4,5-Dihydropyridazin-3- oder 4-yl; 3,4-Dihydropyridazin-3- oder 4- oder 5- oder 6-yl; 3,6-Dihydropyridazin-3- oder 4-yl; 1,6-Dihydropyriazin-1- oder 3- oder 4- oder 5- oder 6-yl; Hexahydropyrimidin-1- oder 2- oder 3- oder 4-yl; 1,4,5,6-Tetrahydropyrimidin-1- oder 2- oder 4- oder 5- oder 6-yl; 1,2,5,6-Tetrahydropyrimidin-1- oder 2- oder 4- oder 5- oder 6-yl; 1,2,3,4-Tetrahydropyrimidin-1- oder 2- oder 3- oder 4- oder 5- oder 6-yl; 1,6-Dihydropyrimidin-1- oder 2- oder 4- oder 5- oder 6-yl; 1,2-Dihydropyrimidin-1- oder 2- oder 4- oder 5- oder 6-yl; 2,5-Dihydropyrimidin-2- oder 4- oder 5-yl; 4,5-Dihydropyrimidin- 4- oder 5- oder 6-yl; 1,4-Dihydropyrimidin-1- oder 2- oder 4- oder 5- oder 6-yl; 1- oder 2- oder 3-Piperazinyl; 1,2,3,6-Tetrahydropyrazin-1- oder 2- oder 3- oder 5-oder 6-yl; 1,2,3,4-Tetrahydropyrazin-1- oder 2- oder 3- oder 4- oder 5- oder 6-yl; 1,2-Dihydropyrazin-1-oder 2- oder 3- oder 5- oder 6-yl; 1,4-Dihydropyrazin-1- oder 2- oder 3-yl; 2,3-Dihydropyrazin-2- oder 3- oder 5- oder 6-yl; 2,5-Dihydropyrazin-2- oder 3-yl; 1,3-Dioxolan-2- oder 4- oder 5-yl; 1,3-Dioxol-2-oder 4-yl; 1,3-Dioxan-2- oder 4- oder 5-yl; 4H-1,3-Dioxin-2- oder 4- oder 5- oder 6-yl; 1,4-Dioxan-2-oder 3- oder 5- oder 6-yl; 2,3-Dihydro-1,4-dioxin-2- oder 3- oder 5- oder 6-yl; 1,4-Dioxin-2- oder 3-yl; 1,2-Dithiolan-3- oder 4-yl; 3H-1,2-Dithiol-3- oder 4- oder 5-yl; 1,3-Dithiolan-2- oder 4-yl; 1,3-Dithiol-2- oder 4-yl; 1,2-Dithian-3- oder 4-yl; 3,4-Dihydro-1,2-dithiin-3- oder 4- oder 5- oder 6-yl; 3,6-Dihydro-1,2-dithiin-3- oder 4-yl; 1,2-Dithiin-3- oder 4-yl; 1,3-Dithian-2- oder 4- oder 5-yl; 4H-1,3-Dithiin-2-oder 4- oder 5- oder 6-yl; Isoxazolidin-2- oder 3- oder 4- oder 5-yl; 2,3-Dihydroisoxazol-2- oder 3- oder 4- oder 5-yl; 2,5-Dihydroisoxazol-2- oder 3- oder 4- oder 5-yl; 4,5-Dihydroisoxazol-3- oder 4- oder 5-yl; 1,3-Oxazolidin-2- oder 3- oder 4- oder 5-yl; 2,3-Dihydro-1,3-oxazol-2- oder 3- oder 4- oder 5-yl; 2,5-Dihydro-1,3-oxazol-2- oder 4- oder 5-yl; 4,5-Dihydro-1,3-oxazol-2- oder 4- oder 5-yl; 1,2-Oxazinan-2-oder 3- oder 4- oder 5- oder 6-yl; 3,4-Dihydro-2H-1,2-oxazin-2- oder 3- oder 4- oder 5- oder 6-yl; 3,6-Dihydro-2H-1,2-oxazin-2- oder 3- oder 4- oder 5- oder 6-yl; 5,6-Dihydro-2H-1,2-oxazin-2- oder 3- oder 4- oder 5- oder 6-yl; 5,6-Dihydro-4H-1,2-oxazin-3- oder 4- oder 5- oder 6-yl; 2H-1,2-Oxazin-2- oder 3-oder 4- oder 5- oder 6-yl; 6H-1,2-Oxazin-3- oder 4- oder 5- oder 6-yl; 4H-1,2-Oxazin-3- oder 4- oder 5-oder 6-yl; 1,3-Oxazinan-2- oder 3- oder 4- oder 5- oder 6-yl; 3,4-Dihydro-2H-1,3-oxazin-2- oder 3- oder 4- oder 5- oder 6-yl; 3,6-Dihydro-2H-1,3-oxazin-2- oder 3- oder 4- oder 5- oder 6-yl; 5,6-Dihydro-2H-1,3-oxazin-2- oder 4- oder 5- oder 6-yl; 5,6-Dihydro-4H-1,3-oxazin-2- oder 4- oder 5- oder 6-yl; 2H-1,3-Oxazin-2- oder 4- oder 5- oder 6-yl; 6H-1,3-Oxazin-2- oder 4- oder 5- oder 6-yl; 4H-1,3-Oxazin-2-oder 4- oder 5- oder 6-yl; Morpholin-2- oder 3- oder 4-yl; 3,4-Dihydro-2H-1,4-oxazin-2- oder 3- oder 4-oder 5- oder 6-yl; 3,6-Dihydro-2H-1,4-oxazin-2- oder 3- oder 5- oder 6-yl; 2H-1,4-oxazin-2- oder 3-oder 5- oder 6-yl; 4H-1,4-oxazin-2- oder 3-yl; 1,2-Oxazepan-2- oder 3- oder 4- oder 5- oder 6- oder 7-yl; 2,3,4,5-Tetrahydro-1,2-oxazepin-2- oder 3- oder 4- oder 5- oder 6- oder 7-yl; 2,3,4,7-Tetrahydro-1,2-oxazepin-2- oder 3- oder 4- oder 5- oder 6- oder 7-yl; 2,3,6,7-Tetrahydro-1,2-oxazepin-2- oder 3- oder 4- oder 5- oder 6- oder 7-yl; 2,5,6,7-Tetrahydro-1,2-oxazepin-2- oder 3- oder 4- oder 5- oder 6- oder 7-yl; 4,5,6,7-Tetrahydro-1,2-oxazepin-3- oder 4- oder 5- oder 6- oder 7-yl; 2,3-Dihydro-1,2-oxazepin-2-oder 3- oder 4- oder 5- oder 6- oder 7-yl; 2,5-Dihydro-1,2-oxazepin-2- oder 3- oder 4- oder 5- oder 6-oder 7-yl; 2,7-Dihydro-1,2-oxazepin-2- oder 3- oder 4- oder 5- oder 6- oder 7-yl; 4,5-Dihydro-1,2-oxazepin-3- oder 4- oder 5- oder 6- oder 7-yl; 4,7-Dihydro-1,2-oxazepin-3- oder 4- oder 5- oder 6- oder 7-yl; 6,7-Dihydro-1,2-oxazepin-3- oder 4- oder 5- oder 6- oder 7-yl; 1,2-Oxazepin-3- oder 4- oder 5-oder 6- oder 7-yl; 1,3-Oxazepan-2- oder 3- oder 4- oder 5- oder 6- oder 7-yl; 2,3,4,5-Tetrahydro-1,3-oxazepin-2- oder 3- oder 4- oder 5- oder 6- oder 7-yl; 2,3,4,7-Tetrahydro-1,3-oxazepin-2- oder 3- oder 4- oder 5- oder 6- oder 7-yl; 2,3,6,7-Tetrahydro-1,3-oxazepin-2- oder 3- oder 4- oder 5- oder 6- oder 7-yl; 2,5,6,7-Tetrahydro-1,3-oxazepin-2- oder 4- oder 5- oder 6- oder 7-yl; 4,5,6,7-Tetrahydro-1,3-oxazepin-2- oder 4- oder 5- oder 6- oder 7-yl; 2,3-Dihydro-1,3-oxazepin-2- oder 3- oder 4- oder 5- oder 6- oder 7-yl; 2,5-Dihydro-1,3-oxazepin-2- oder 4- oder 5- oder 6- oder 7-yl; 2,7-Dihydro-1,3-oxazepin-2- oder 4- oder 5- oder 6- oder 7-yl; 4,5-Dihydro-1,3-oxazepin-2- oder 4- oder 5- oder 6- oder 7-yl; 4,7-Dihydro-1,3-oxazepin-2- oder 4- oder 5- oder 6- oder 7-yl; 6,7-Dihydro-1,3-oxazepin-2- oder 4- oder 5-oder 6- oder 7-yl; 1,3-Oxazepin-2- oder 4- oder 5- oder 6- oder 7-yl; 1,4-Oxazepan-2- oder 3- oder 5-oder 6- oder 7-yl; 2,3,4,5-Tetrahydro-1,4-oxazepin-2- oder 3- oder 4- oder 5- oder 6- oder 7-yl; 2,3,4,7-Tetrahydro-1,4-oxazepin-2- oder 3- oder 4- oder 5- oder 6- oder 7-yl; 2,3,6,7-Tetrahydro-1,4-oxazepin-2- oder 3- oder 5- oder 6- oder 7-yl; 2,5,6,7-Tetrahydro-1,4-oxazepin-2- oder 3- oder 5- oder 6- oder 7-yl; 4,5,6,7-Tetrahydro-1,4-oxazepin-2- oder 3- oder 4- oder 5- oder 6- oder 7-yl; 2,3-Dihydro-1,4-oxazepin-2- oder 3- oder 5- oder 6- oder 7-yl; 2,5-Dihydro-1,4-oxazepin-2- oder 3- oder 5- oder 6- oder 7-yl; 2,7-Dihydro-1,4-oxazepin-2- oder 3- oder 5- oder 6- oder 7-yl; 4,5-Dihydro-1,4-oxazepin-2- oder 3- oder 4- oder 5- oder 6- oder 7-yl; 4,7-Dihydro-1,4-oxazepin-2- oder 3- oder 4- oder 5- oder 6- oder 7-yl; 6,7-Dihydro-1,4-oxazepin-2- oder 3- oder 5- oder 6- oder 7-yl; 1,4-Oxazepin-2- oder 3- oder 5- oder 6- oder 7-yl; Isothiazolidin-2- oder 3- oder 4- oder 5-yl; 2,3-Dihydroisothiazol-2- oder 3- oder 4- oder 5-yl; 2,5-Dihydroisothiazol-2- oder 3- oder 4- oder 5-yl; 4,5-Dihydroisothiazol-3- oder 4- oder 5-yl; 1,3-Thiazolidin-2- oder 3- oder 4- oder 5-yl; 2,3-Dihydro-1,3-thiazol-2- oder 3- oder 4- oder 5-yl; 2,5-Dihydro-1,3-thiazol-2- oder 4- oder 5-yl; 4,5-Dihydro-1,3-thiazol-2- oder 4- oder 5-yl; 1,3-Thiazinan-2-oder 3- oder 4- oder 5- oder 6-yl; 3,4-Dihydro-2H-1,3-thiazin-2- oder 3- oder 4- oder 5- oder 6-yl; 3,6-Dihydro-2H-1,3-thiazin-2- oder 3- oder 4- oder 5- oder 6-yl; 5,6-Dihydro-2H-1,3-thiazin-2- oder 4- oder 5- oder 6-yl; 5,6-Dihydro-4H-1,3-thiazin-2- oder 4- oder 5- oder 6-yl; 2H-1,3-Thiazin-2- oder 4- oder 5-oder 6-yl; 6H-1,3-Thiazin-2- oder 4- oder 5- oder 6-yl; 4H-1,3-Thiazin-2- oder 4- oder 5- oder 6-yl. Weitere Beispiele für "Heterocyclyl" sind ein partiell oder vollständig hydrierter heterocyclischer Rest mit 3 Heteroatomen aus der Gruppe N, O und S, wie beispielsweise 1,4,2-Dioxazolidin-2- oder 3- oder 5-yl; 1,4,2-Dioxazol-3- oder 5-yl; 1,4,2-Dioxazinan-2- oder -3- oder 5- oder 6-yl; 5,6-Dihydro-1,4,2-dioxazin-3- oder 5- oder 6-yl; 1,4,2-Dioxazin-3- oder 5- oder 6-yl; 1,4,2-Dioxazepan-2- oder 3- oder 5-oder 6- oder 7-yl; 6,7-Dihydro-5H-1,4,2-Dioxazepin-3- oder 5- oder 6- oder 7-yl; 2,3-Dihydro-7H-1,4,2-Dioxazepin-2- oder 3- oder 5- oder 6- oder 7-yl; 2,3-Dihydro-5H-1,4,2-Dioxazepin-2- oder 3- oder 5-oder 6- oder 7-yl; 5H-1,4,2-Dioxazepin-3- oder 5- oder 6- oder 7-yl; 7H-1,4,2-Dioxazepin-3- oder 5-oder 6- oder 7-yl. Strukturbeispiele für gegebenenfalls weiter substituierte Heterocyclen sind auch im Folgenden aufgeführt:

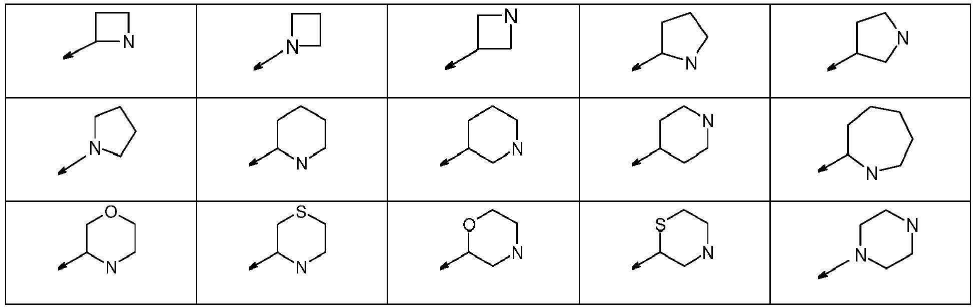

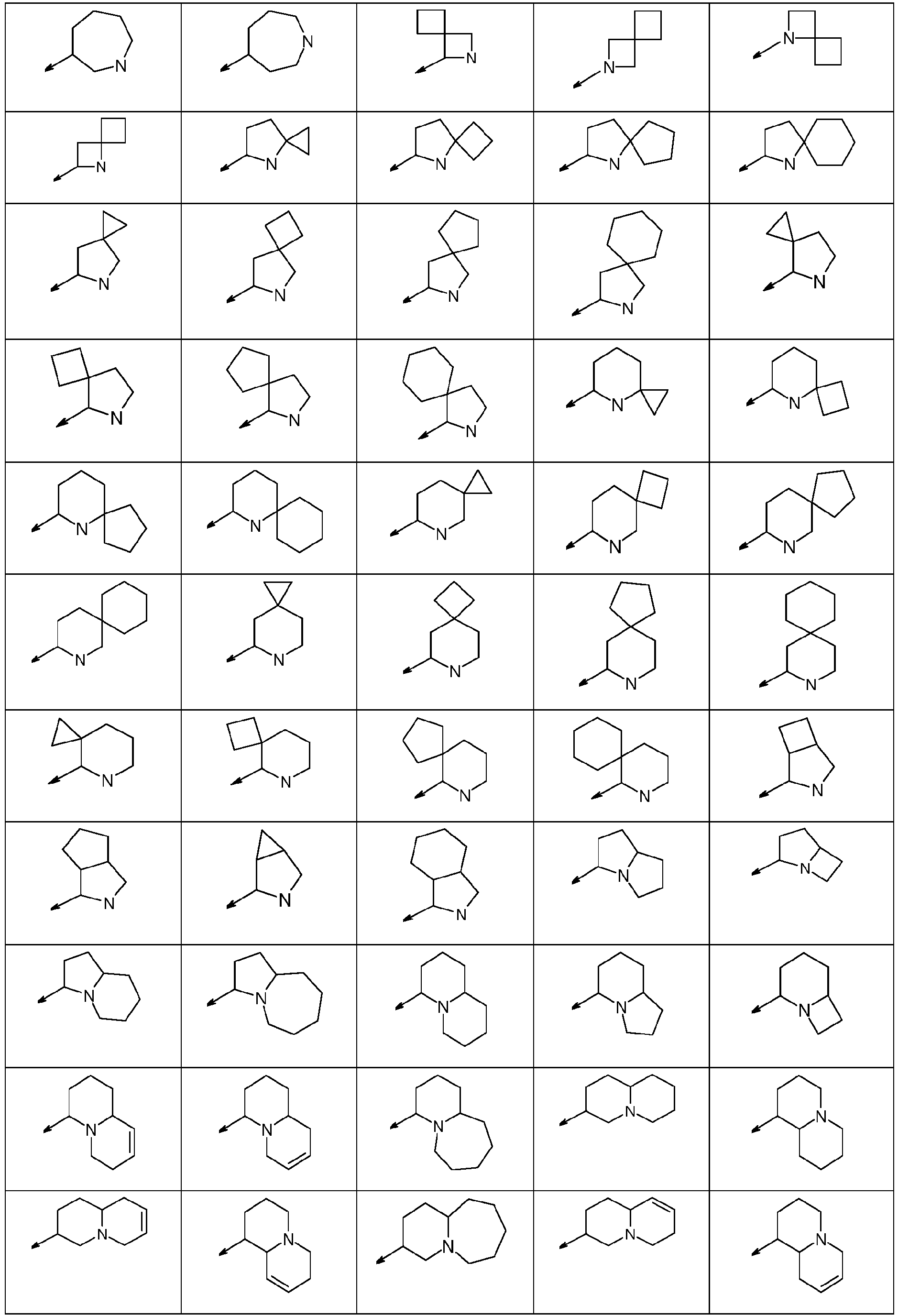

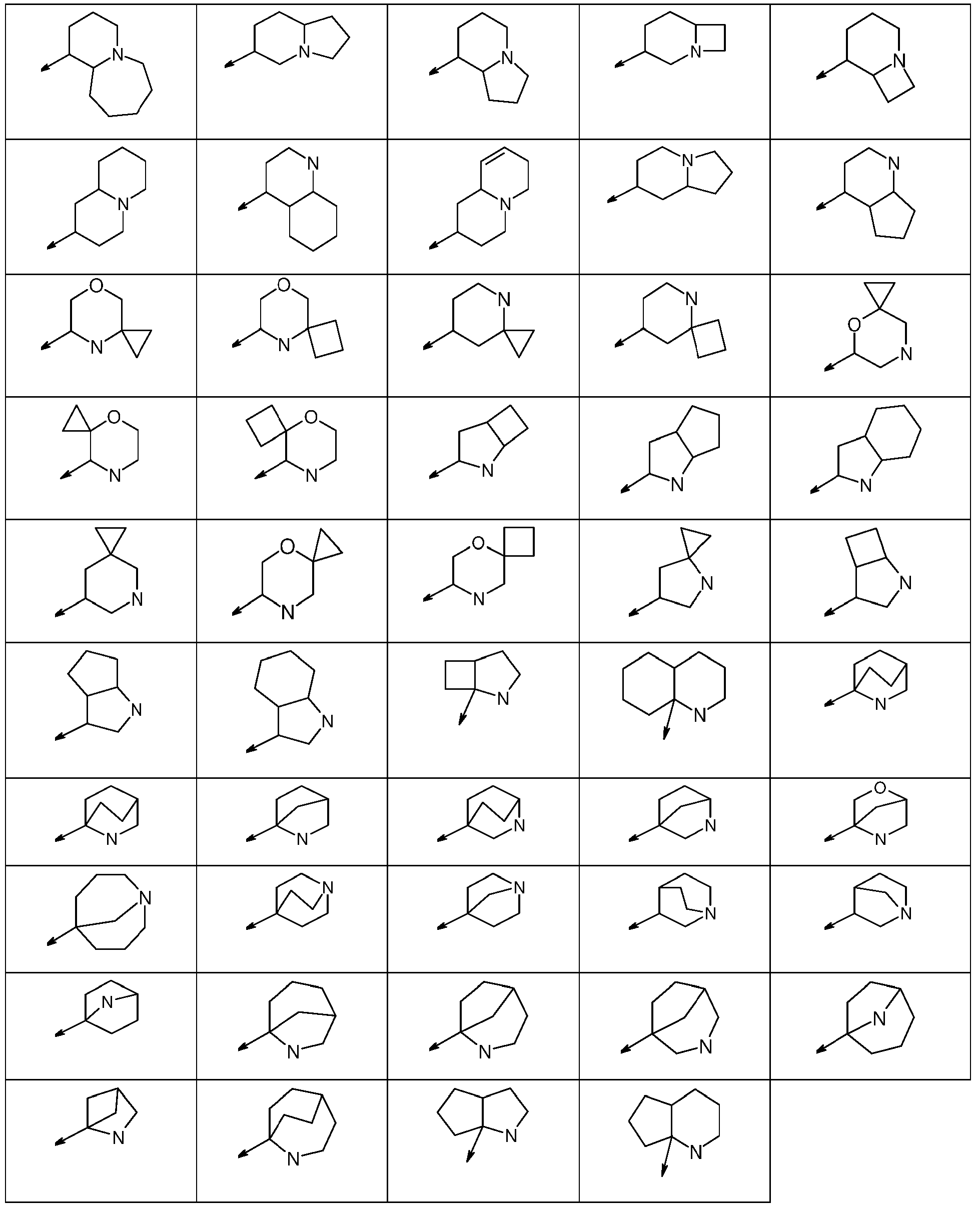

Die oben aufgeführten Heterocyclen sind bevorzugt beispielsweise durch Wasserstoff, Halogen, Alkyl, Haloalkyl, Hydroxy, Alkoxy, Cycloalkoxy, Aryloxy, Alkoxyalkyl, Alkoxyalkoxy, Cycloalkyl, Halocycloalkyl, Aryl, Arylalkyl, Heteroaryl, Heterocyclyl, Alkenyl, Alkylcarbonyl, Cycloalkylcarbonyl, Arylcarbonyl, Heteroarylcarbonyl, Alkoxycarbonyl, Hydroxycarbonyl, Cycloalkoxycarbonyl, Cycloalkylalkoxycarbonyl, Alkoxycarbonylalkyl, Arylalkoxycarbonyl, Arylalkoxycarbonylalkyl, Alkinyl, Alkinylalkyl, Alkylalkinyl, Tris-alkylsilylalkinyl, Nitro, Amino, Cyano, Haloalkoxy, Haloalkylthio, Alkylthio, Hydrothio, Hydroxyalkyl, Oxo, Heteroarylalkoxy, Arylalkoxy, Heterocyclylalkoxy, Heterocyclylalkylthio, Heterocyclyloxy, Heterocyclylthio, Heteroaryloxy, Bis-alkylamino, Alkylamino, Cycloalkylamino, Hydroxycarbonylalkylamino, Alkoxycarbonylalkylamino, Arylalkoxycarbonylalkylamino, Alkoxycarbonylalkyl(alkyl)amino, Aminocarbonyl, Alkylaminocarbonyl, Bis-alkylaminocarbonyl, Cycloalkylaminocarbonyl, Hydroxycarbonylalkylaminocarbonyl, Alkoxycarbonylalkylaminocarbonyl, Arylalkoxycarbonylalkylaminocarbonyl substituiert.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Handelt es sich es sich um einen teilweise oder vollständig gesättigten Stickstoff-Heterocyclus, so kann dieser sowohl über Kohlenstoff als auch über den Stickstoff mit dem Rest des Moleküls verknüpft sein.

Als Substituenten für einen substituierten heterocyclischen Rest kommen die weiter unten genannten Substituenten in Frage, zusätzlich auch Oxo und Thioxo. Die Oxogruppe als Substituent an einem Ring-C-Atom bedeutet dann beispielsweise eine Carbonylgruppe im heterocyclischen Ring. Dadurch sind vorzugsweise auch Lactone und Lactame umfasst. Die Oxogruppe kann auch an den Heteroringatomen, die in verschiedenen Oxidationsstufen existieren können, z.B. bei N und S, auftreten und bilden dann beispielsweise die divalenten Gruppen N(O), S(O) (auch kurz SO) und S(O)₂ (auch kurz SO₂) im heterocyclischen Ring. Im Fall von -N(O)- und -S(O)-Gruppen sind jeweils beide Enantiomere umfasst.

Erfindungsgemäß steht der Ausdruck "Heteroaryl" für heteroaromatische Verbindungen, d. h. vollständig ungesättigte aromatische heterocyclische Verbindungen, vorzugsweise für 5- bis 7-gliedrige Ringe mit 1 bis 4, vorzugsweise 1 oder 2 gleichen oder verschiedenen Heteroatomen, vorzugsweise O, S oder N. Erfindungsgemäße Heteroaryle sind beispielsweise 1H-Pyrrol-1-yl; 1H-Pyrrol-2-yl; 1H-Pyrrol-3-yl; Furan-2-yl; Furan-3-yl; Thien-2-yl; Thien-3-yl, 1H-Imidazol-1-yl; 1H-Imidazol-2-yl; 1H-Imidazol-4-yl; 1H-Imidazol-5-yl; 1H-Pyrazol-1-yl; 1H-Pyrazol-3-yl; 1H-Pyrazol-4-yl; 1H-Pyrazol-5-yl, 1H-1,2,3-Triazol-1-yl, 1H-1,2,3-Triazol-4-yl, 1H-1,2,3-Triazol-5-yl, 2H-1,2,3-Triazol-2-yl, 2H-1,2,3-Triazol-4-yl, 1H-1,2,4-Triazol-1-yl, 1H-1,2,4-Triazol-3-yl, 4H-1,2,4-Triazol-4-yl, 1,2,4-Oxadiazol-3-yl, 1,2,4-Oxadiazol-5-yl, 1,3,4-Oxadiazol-2-yl, 1,2,3-Oxadiazol-4-yl, 1,2,3-Oxadiazol-5-yl, 1,2,5-Oxadiazol-3-yl, Azepinyl, Pyridin-2-yl, Pyridin-3-yl, Pyridin-4-yl, Pyrazin-2-yl, Pyrazin-3-yl, Pyrimidin-2-yl, Pyrimidin-4-yl, Pyrimidin-5-yl, Pyridazin-3-yl, Pyridazin-4-yl, 1,3,5-Triazin-2-yl, 1,2,4-Triazin-3-yl, 1,2,4-Triazin-5-yl, 1,2,4-Triazin-6-yl, 1,2,3-Triazin-4-yl, 1,2,3-Triazin-5-yl, 1,2,4-, 1,3,2-, 1,3,6- und 1,2,6-Oxazinyl, Isoxazol-3-yl, Isoxazol-4-yl, Isoxazol-5-yl, 1,3-Oxazol-2-yl, 1,3-Oxazol-4-yl, 1,3-Oxazol-5-yl, Isothiazol-3-yl, Isothiazol-4-yl, Isothiazol-5-yl, 1,3-Thiazol-2-yl, 1,3-Thiazol-4-yl, 1,3-Thiazol-5-yl, Oxepinyl, Thiepinyl, 1,2,4-Triazolonyl und 1,2,4-Diazepinyl, 2H-1,2,3,4-Tetrazol-5-yl, 1H-1,2,3,4-Tetrazol-5-yl, 1,2,3,4-Oxatriazol-5-yl, 1,2,3,4-Thiatriazol-5-yl, 1,2,3,5-Oxatriazol-4-yl, 1,2,3,5-Thiatriazol-4-yl. Die erfindungsgemäßen Heteroarylgruppen können ferner mit einem oder mehreren, gleichen oder verschiedenen Resten substituiert sein. Sind zwei benachbarte Kohlenstoffatome Bestandteil eines weiteren aromatischen Rings, so handelt es sich um annellierte heteroaromatische Systeme, wie benzokondensierte oder mehrfach annellierte Heteroaromaten. Bevorzugt sind beispielsweise Chinoline (z. B. Chinolin-2-yl, Chinolin-3-yl, Chinolin-4-yl, Chinolin-5-yl, Chinolin-6-yl, Chinolin-7-yl, Chinolin-8-yl); Isochinoline (z. B. Isochinolin-1-yl, Isochinolin-3-yl, Isochinolin-4-yl, Isochinolin-5-yl, Isochinolin-6-yl, Isochinolin-7-yl, Isochinolin-8-yl); Chinoxalin; Chinazolin; Cinnolin; 1,5-Naphthyridin; 1,6-Naphthyridin; 1,7-Naphthyridin; 1,8-Naphthyridin; 2,6-Naphthyridin; 2,7-Naphthyridin; Phthalazin; Pyridopyrazine; Pyridopyrimidine; Pyridopyridazine; Pteridine; Pyrimidopyrimidine. Beispiele für Heteroaryl sind auch 5- oder 6-gliedrige benzokondensierte Ringe aus der Gruppe 1H-Indol-1-yl, 1H-Indol-2-yl, 1H-Indol-3-yl, 1H-Indol-4-yl, 1H-Indol-5-yl, 1H-Indol-6-yl, 1H-Indol-7-yl, 1-Benzofuran-2-yl, 1-Benzofuran-3-yl, 1-Benzofuran-4-yl, 1-Benzofuran-5-yl, 1-Benzofuran-6-yl, 1-Benzofuran-7-yl, 1-Benzothiophen-2-yl, 1-Benzothiophen-3-yl, 1-Benzothiophen-4-yl, 1-Benzothiophen-5-yl, 1-Benzothiophen-6-yl, 1-Benzothiophen-7-yl, 1H-Indazol-1-yl, 1H-Indazol-3-yl, 1H-Indazol-4-yl, 1H-Indazol-5-yl, 1H-Indazol-6-yl, 1H-Indazol-7-yl, 2H-Indazol-2-yl, 2H-Indazol-3-yl, 2H-Indazol-4-yl, 2H-Indazol-5-yl, 2H-Indazol-6-yl, 2H-Indazol-7-yl, 2H-Isoindol-2-yl, 2H-Isoindol-1-yl, 2H-Isoindol-3-yl, 2H-Isoindol-4-yl, 2H-Isoindol-5-yl, 2H-Isoindol-6-yl; 2H-Isoindol-7-yl, 1H-Benzimidazol-1-yl, 1H-Benzimidazol-2-yl, 1H-Benzimidazol-4-yl, 1H-Benzimidazol-5-yl, 1H-Benzimidazol-6-yl, 1H-Benzimidazol-7-yl, 1,3-Benzoxazol-2-yl, 1,3-Benzoxazol-4-yl, 1,3-Benzoxazol-5-yl, 1,3-Benzoxazol-6-yl, 1,3-Benzoxazol-7-yl, 1,3-Benzthiazol-2-yl, 1,3-Benzthiazol-4-yl, 1,3-Benzthiazol-5-yl, 1,3-Benzthiazol-6-yl, 1,3-Benzthiazol-7-yl, 1,2-Benzisoxazol-3-yl, 1,2-Benzisoxazol-4-yl, 1,2-Benzisoxazol-5-yl, 1,2-Benzisoxazol-6-yl, 1,2-Benzisoxazol-7-yl, 1,2-Benzisothiazol-3-yl, 1,2-Benzisothiazol-4-yl, 1,2-Benzisothiazol-5-yl, 1,2-Benzisothiazol-6-yl, 1,2-Benzisothiazol-7-yl.

Die Bezeichnung "Halogen" bedeutet beispielsweise Fluor, Chlor, Brom oder Iod. Wird die Bezeichnung für einen Rest verwendet, dann bedeutet "Halogen" beispielsweise ein Fluor-, Chlor-, Brom- oder Iodatom.

Erfindungsgemäß bedeutet "Alkyl" einen geradkettigen oder verzweigten offenkettigen, gesättigten Kohlenwasserstoffrest, der gegebenenfalls ein- oder mehrfach substituiert ist und im letzteren Falle als "substituiertes Alkyl" bezeichnet wird. Bevorzugte Substituenten sind Halogenatome, Alkoxy-, Haloalkoxy-, Cyano-, Alkylthio, Haloalkylthio-, Amino- oder Nitrogruppen, besonders bevorzugt sind Methoxy, Methyl, Fluoralkyl, Cyano, Nitro, Fluor, Chlor, Brom oder Iod. Die Vorsilbe "Bis" schließt auch die Kombination unterschiedlicher Alkylreste ein, z. B. Methyl(Ethyl) oder Ethyl(Methyl).

"Haloalkyl", "-alkenyl" und "-alkinyl" bedeuten durch gleiche oder verschiedene Halogenatome, teilweise oder vollständig substituiertes Alkyl, Alkenyl bzw. Alkinyl, z.B. Monohaloalkyl (= Monohalogenalkyl) wie z. B. CH₂CH₂Cl, CH₂CH₂Br, CHClCH₃, CH₂Cl, CH₂F; Perhaloalkyl wie z. B. CCl₃, CClF₂, CFCl₂, CF₂CClF₂, CF₂CClFCF₃; Polyhaloalkyl wie z. B. CH₂CHFCl, CF₂CClFH, CF₂CBrFH, CH₂CF₃; Der Begriff Perhaloalkyl umfasst dabei auch den Begriff Perfluoralkyl.

"Teilfluoriertes Alkyl" bedeutet einen geradkettigen oder verzweigten, gesättigten Kohlenwasserstoff, der einfach oder mehrfach durch Fluor substituiert ist, wobei sich die entsprechenden Fluoratome als Substituenten an einem oder mehreren verschiedenen Kohlenstoffatomen der geradkettigen oder verzweigten Kohlenwasserstoffkette befinden können, wie z. B. CHFCH₃, CH₂CH₂F, CH₂CH₂CF₃, CHF₂, CH₂F, CHFCF₂CF₃

"Teilfluoriertes Haloalkyl" bedeutet einen geradkettigen oder verzweigten, gesättigten Kohlenwasserstoff, der durch verschiedene Halogenatome mit mindestens einem Fluoratom substituiert ist, wobei alle anderen gegebenenfalls vorhandenen Halogenatome ausgewählt sind aus der Gruppe Fluor, Chlor oder Brom, Iod. Die entsprechenden Halogenatome können sich dabei als Substituenten an einem oder mehreren verschiedenen Kohlenstoffatomen der geradkettigen oder verzweigten Kohlenwasserstoffkette befinden. Teilfluoriertes Haloalkyl schließt auch die vollständige Substitution der geradkettigen oder verzweigten Kette durch Halogen unter Beteiligung von mindestens einem Fluoratom ein.

"Haloalkoxy" ist z.B. OCF₃, OCHF₂, OCH₂F, OCF₂CF₃, OCH₂CF₃ und OCH₂CH₂Cl; Entsprechendes gilt für Haloalkenyl und andere durch Halogen substituierten Reste.

Der hier beispielhaft genannte Ausdruck "(C₁-C₄)-Alkyl" bedeutet eine Kurzschreibweise für geradkettiges oder verzweigtes Alkyl mit einem bis 4 Kohlenstoffatomen entsprechend der Bereichsangabe für C-Atome, d. h. umfasst die Reste Methyl, Ethyl, 1-Propyl, 2-Propyl, 1-Butyl, 2-Butyl, 2-Methylpropyl oder tert-Butyl. Allgemeine Alkylreste mit einem größeren angegebenen Bereich von C-Atomen, z. B. "(C₁-C₆)-Alkyl", umfassen entsprechend auch geradkettige oder verzweigte Alkylreste mit einer größeren Zahl von C-Atomen, d. h. gemäß Beispiel auch die Alkylreste mit 5 und 6 C-Atomen.

Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen bzw. bei ungesättigten Gruppen mit 2 bis 6 C-Atomen, bevorzugt. Alkylreste, auch in den zusammengesetzten Resten wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste, wobei mindestens eine Doppelbindung bzw. Dreifachbindung enthalten ist. Bevorzugt sind Reste mit einer Doppelbindung bzw. Dreifachbindung.

Der Begriff "Alkenyl" schließt insbesondere auch geradkettige oder verzweigte offenkettige Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl. Alkenyl bedeutet z.B. Vinyl, welches ggf. durch weitere Alkylreste substituiert sein kann, z B. (aber nicht beschränkt auf) (C₂-C₆)-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl.

Der Begriff "Alkinyl" schließt insbesondere auch geradkettige oder verzweigte offenkettige Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl. (C₂-C₆)-Alkinyl bedeutet z.B. Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Di-methyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl.

Der Begriff "Cycloalkyl" bedeutet ein carbocyclisches, gesättigtes Ringsystem mit vorzugsweise 3-8 Ring-C-Atomen, z.B. Cyclopropyl, Cyclobutyl, Cyclopentyl oder Cyclohexyl, das gegebenenfalls weiter substituiert ist, bevorzugt durch Wasserstoff, Alkyl, Alkoxy, Cyano, Nitro, Alkylthio, Haloalkylthio, Halogen, Alkenyl, Alkinyl, Haloalkyl, AMino, Alkylamino, Bisalkylamino, Alkocycarbonyl, Hydroxycarbonyl, Arylalkoxycarbonyl, Aminocarbonyl, Alkylaminocarbonyl, Cycloalkylaminocarbonyl. Im Falle von gegebenenfalls substituiertem Cycloalkyl werden cyclische Systeme mit Substituenten umfasst, wobei auch Substituenten mit einer Doppelbindung am Cycloalkylrest, z. B. eine Alkylidengruppe wie Methyliden, umfasst sind. Im Falle von gegebenenfalls substituiertem Cycloalkyl werden auch mehrcyclische aliphatische Systeme umfasst, wie beispielsweise Bicyclo[1.1.0]butan-1-yl, Bicyclo[1.1.0]butan-2-yl, Bicyclo[2.1.0]pentan-1-yl, Bicyclo[1.1.1]pentan-1-yl, Bicyclo[2.1.0]pentan-2-yl, Bicyclo[2.1.0]pentan-5-yl, Bicyclo[2.1.1]hexyl, Bicyclo[2.2.1]hept-2-yl, Bicyclo[2.2.2]octan-2-yl, Bicyclo[3.2.1]octan-2-yl, Bicyclo[3.2.2]nonan-2-yl, Adamantan-1-yl und Adamantan-2-yl, aber auch Systeme wie z. B. 1,1'-Bi(cyclopropyl)-1-yl, 1,1'-Bi(cyclopropyl)-2-yl. Der Ausdruck "(C₃-C₇)-Cycloalkyl" bedeutet eine Kurzschreibweise für Cycloalkyl mit drei bis 7 Kohlenstoffatomen entsprechend der Bereichsangabe für C-Atome.

Im Falle von substituiertem Cycloalkyl werden auch spirocyclische aliphatische Systeme umfasst, wie beispielsweise Spiro[2.2]pent-1-yl, Spiro[2.3]hex-1-yl, Spiro[2.3]hex-4-yl, 3-Spiro[2.3]hex-5-yl, Spiro[3.3]hept-1-yl, Spiro[3.3]hept-2-yl.

"Cycloalkenyl" bedeutet ein carbocyclisches, nicht aromatisches, partiell ungesättigtes Ringsystem mit vorzugsweise 4-8 C-Atomen, z.B. 1-Cyclobutenyl, 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl, wobei auch Substituenten mit einer Doppelbindung am Cycloalkenylrest, z. B. eine Alkylidengruppe wie Methyliden, umfasst sind. Im Falle von gegebenenfalls substituiertem Cycloalkenyl gelten die Erläuterungen für substituiertes Cycloalkyl entsprechend.

Der Begriff "Alkyliden", z. B. auch in der Form (C₁-C₁₀)-Alkyliden, bedeutet den Rest eines geradkettigen oder verzweigten offenkettigen Kohlenwasserstoffrests, der über eine Zweifachbindung gebunden ist. Als Bindungsstelle für Alkyliden kommen naturgemäß nur Positionen am Grundkörper in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅ Cycloalkyliden bedeutet ein carbocyclischer Rest, der über eine Zweifachbindung gebunden ist.

"Cycloalkylalkyloxy" bedeutet ein über ein Sauerstoffatom gebundenen Cycloalkylalkylrest und "Arylalkyloxy" bedeutet ein über ein Sauerstoffatom gebundenen Arylalkylrest.

"Alkoxyalkyl" steht für einen über eine Alkylgruppe gebundenen Alkoxyrest und "Alkoxyalkoxy" bedeutet einen über ein Sauerstoffatom gebundenen Alkoxyalkylrest, z.B. (aber nicht beschränkt auf) Methoxymethoxy, Methoxyethoxy, Ethoxyethoxy, Methoxy-n-propyloxy.

"Alkylthioalkyl" steht für einen über eine Alkylgruppe gebundenen Alkylthiorest und "Alkylthioalkylthio" bedeutet einen über ein Sauerstoffatom gebundenen Alkylthioalkylrest.

"Arylalkoxyalkyl" steht für einen über eine Alkylgruppe gebundenen Aryloxyrest und "Heteroaryloxyalkyl" bedeutet einen über eine Alkylgruppe gebundenen Heteroaryloxyrest.

"Haloalkoxyalkyl" steht für einen gebundenen Haloalkoxyrest und "Haloalkylthioalkyl" bedeutet einen über eine Alkylgruppe gebundenen Haloalkylthiorest.

"Arylalkyl" steht für einen über eine Alkylgruppe gebundenen Arylrest, "Heteroarylalkyl" bedeutet einen über eine Alkylgruppe gebundenen Heteroarylrest, und "Heterocyclylalkyl" bedeutet einen über eine Alkylgruppe gebundenen Heterocyclylrest.

"Cycloalkylalkyl" steht für einen über eine Alkylgruppe gebundenen Cycloalkylrest, z. B. (aber nicht beschränkt auf) Cyclopropylmethyl, Cyclobutylmethyl, Cyclopentylmethyl, Cyclohexylmethyl, 1-Cyclopropyleth-1-yl, 2-Cyclopropyleth-1-yl, 1-Cyclopropylprop-1-yl, 3-Cyclopropylprop-1-yl.

"Arylalkenyl" steht für einen über eine Alkenylgruppe gebundenen Arylrest, "Heteroarylalkenyl" bedeutet einen über eine Alkenylgruppe gebundenen Heteroarylrest, und "Heterocyclylalkenyl" bedeutet einen über eine Alkenylgruppe gebundenen Heterocyclylrest.

"Arylalkinyl" steht für einen über eine Alkinylgruppe gebundenen Arylrest, "Heteroarylalkinyl" bedeutet einen über eine Alkinylgruppe gebundenen Heteroarylrest, und "Heterocyclylalkinyl" bedeutet einen über eine Alkinylgruppe gebundenen Heterocyclylrest.

Erfindungsgemäß steht "Haloalkylthio" - in Alleinstellung oder als Bestandteil einer chemischen Gruppe - für geradkettiges oder verzweigtes S-Halogenalkyl, vorzugsweise mit 1 bis 8, oder mit 1 bis 6 Kohlenstoffatomen, wie (C₁-C₈)-, (C₁-C₆)- oder (C₁-C₄)-Haloalkylthio, z.B. (aber nicht beschränkt auf) Trifluormethylthio, Pentafluorethylthio, Difluormethyl, 2,2-Difluoreth-1-ylthio, 2,2,2-Difluoreth-1-ylthio, 3,3,3-prop-1-ylthio.

"Halocycloalkyl" und "Halocycloalkenyl" bedeuten durch gleiche oder verschiedene Halogenatome, wie z. B. F, Cl und Br, oder durch Haloalkyl, wie z. B. Trifluormethyl oder Difluormethyl teilweise oder vollständig substituiertes Cycloalkyl oder Cycloalkenyl , z.B. "1-Fluorcycloprop-1-yl, 2-Fluorcycloprop-1-yl, 2,2-Difluorcycloprop-1-yl, 1-Fluorcyclobut-1-yl, 1-Trifluormethylcycloprop-1-yl, 2-Trifluormethylcycloprop-1-yl, 1-Chlor-cycloprop-1-yl, 2-Chlorcycloprop-1-yl, 2,2-Dichlorcycloprop-1-yl, 3,3-Difluorcyclobutyl,

Erfindungsgemäß steht "Trialkylsilyl" - in Alleinstellung oder als Bestandteil einer chemischen Gruppe - für geradkettiges oder verzweigtes Si-Alkyl, vorzugsweise mit 1 bis 8, oder mit 1 bis 6 Kohlenstoffatomen, wie Tri-[(C₁-C₈)-, (C₁-C₆)- oder (C₁-C₄)-alkyl]silyl, z.B. (aber nicht beschränkt auf) Trimethylsilyl, Triethylsilyl, Tri-(n-propyl)silyl, Tri-(iso-propyl)silyl, Tri-(n-butyl)silyl, Tri-(1-methylprop-1-yl)silyl, Tri-(2-methylprop-1-yl)silyl, Tri(1,1-Dimethyleth-1-yl)silyl, Tri(2,2-Dimethyleth-1-yl)silyl.

"Trialkylsilylalkinyl" steht für einen über eine Alkinylgruppe gebundenen Trialkylsilylrest.

Wenn die Verbindungen durch Wasserstoffverschiebung Tautomere bilden können, welche strukturell formal nicht durch die Formel (I) erfasst würden, so sind diese Tautomere gleichwohl von der Definition der erfindungsgemäßen Verbindungen der Formel (I) umfasst, sofern nicht ein bestimmtes Tautomer Gegenstand der Betrachtung ist. So können beispielsweise viele Carbonylverbindungen sowohl in der Ketoform wie auch in der Enolform vorliegen, wobei beide Formen durch die Definition der Verbindung der Formel (I) umfasst werden.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der Formel (I) umfasst. Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Die chromatographische Trennung kann sowohl im analytischen Maßstab zur Feststellung des Enantiomerenüberschusses bzw. des Diastereomerenüberschusses, wie auch im präparativen Maßstab zur Herstellung von Prüfmustern für die biologische Ausprüfung erfolgen. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (I) umfasst, jedoch nicht mit ihrer spezifischen Stereoform angegeben sind, sowie deren Gemische.

Sofern die Verbindungen als Feststoffe erhalten werden, kann die Reinigung auch durch Umkristallisieren oder Digerieren erfolgen. Sofern einzelne Verbindungen (I) nicht auf den nachstehend beschriebenen Wegen zufriedenstellend zugänglich sind, können sie durch Derivatisierung anderer Verbindungen (I) hergestellt werden.

Als Isolierungs-, Reinigungs- und Stereoisomerenauftrennungsverfahren von Verbindungen der Formel (I) kommen Methoden in Frage, die dem Fachmann aus analogen Fällen allgemein bekannt sind, z.B. durch physikalische Verfahren wie Kristallisation, Chromatographieverfahren, vor allem Säulenchromatographie und HPLC (Hochdruckflüssigchromatographie), Destillation, gegebenenfalls unter reduziertem Druck, Extraktion und andere Verfahren, können gegebenfalls verbleibende Gemische in der Regel durch chromatographische Trennung, z.B. an chiralen Festphasen, getrennt werden. Für präparative Mengen oder im industriellen Maßstab kommen Verfahren in Frage wie Kristallisation, z.B. diastereomerer Salze, die aus den Diastereomerengemischen mit optisch aktiven Säuren und gegebenenfalls bei vorhandenen sauren Gruppen mit optisch aktiven Basen erhalten werden können.

### Synthese von substituierten N-Benzoesäureuracilen der allgemeinen Formel (I):

Die erfindungsgemäßen substituierten N-Benzoesäureuracile der allgemeinen Formel (I) können ausgehend von bekannten Verfahren hergestellt werden. Die eingesetzten und untersuchten Syntheserouten gehen dabei von kommerziell erhältlichen oder leicht herstellbaren Synthesebausteinen aus. Die Gruppierungen G, Q, R¹, R², R³, R⁴, R⁵, R⁶, R⁷, R¹², R¹³ und R¹⁴ der allgemeinen Formel (I) haben in den nachfolgenden Schemata die zuvor definierten Bedeutungen, sofern nicht beispielhafte, aber nicht einschränkende, Definitionen erfolgen.

Die Synthese der Verbindungen der allgemeinen Formel (I) verläuft, wie nachfolgend beispielhaft in Schema 1 beschrieben. Ausgehend von kommerziell verfügbarer 2-Chlor-4-fluor-5-nitrobenzoesäure (II) erhält man durch Veresterung mit einem geeigneten alpha-Hydroxy-carbonsäure-Allylester (III), hier beispielhaft aber nicht einschränkend als kommerziell erhältliches Allyl 2-hydroxy-2-methylpropanoat dargestellt, den adäquat substituierten Nitrobenzoesäureester (IV).

Die Veresterung kann, wie beispielhaft aber nicht einschränkend in Schema 1 dargestellt via Transformation ins Säurechlorid mittels Thionylchlorid erfolgen, wobei ein geeignetes polar-aprotischen Lösemittel [z. B. Dichlormethan (DCM), Chloroform, N,N-Dimethylacetamid (DMA) oder N,N-Dimethylformamid (DMF)] Verwendung findet. Alternativ können die Nitrobenzoesäureester (IV) unter Vermittlung geeigneter Kupplungsreagenzien (z. B. HOBt = 1-Hydroxybenzotriazol, EDC = 1-Ethyl-3-(3-dimethylaminopropyl)carbodiimid, HATU = O-(7-Azabenzotriazol-1-yl)-N,N,N',N'-tetramethyluronium-hexafluorphosphat, T3P = 2,4,6-Tripropyl-1,3,5,2,4,6-trioxatriphosphorinane-2,4,6-trioxid) und geeigneter Basen (z. B. Diisopropylethylamin, Triethylamin) in einem geeigneten polar-aprotischen Lösemittel (z. B. Dichlormethan, Chloroform) erhalten werden. Die nachfolgende Reduktion der Nitrogruppe liefert den entsprechenden 3-Aminobenzoesäureester (V). Die Reduktion erfolgt dabei mit einem geeigneten Reduktionsmittel (z. B. Wasserstoff, Palladium auf Kohle in einem geeigneten polar-protischen Lösemittel) oder, wie in Schema 1 beispielhaft dargestellt, unter Verwendung von Eisenpulver in Essigsäure.

Die Synthese der 2-(Dimethylamino)-4-(haloalkyl)-6H-1,3-oxazin-6-one vom Typ (VII) erfolgt in einer zweistufigen Synthesesequenz (wie in WO2000049002 A1 beschrieben) ausgehend von den entsprechenden Aminoacrylsäureestern, , beispielhaft aber nicht einschränkend z.B. ausgehend von Ethyl-(2Z)-3-amino-4,4,4-trifluorbut-2-enoat (vgl. Journal of Fluorine Chemistry (2016), 181, 1-6), mittels Umsetzung mit Dimethylcarbamoylchlorid in N,N-Dimethylformamid (DMF) unter Verwendung einer geeigneten Base (z. B. Natriumhydrid oder Kalium-tert-butylat) und nachfolgender Cyclisierung zum Oxazin-6-on (VII) unter Verwendung von Phosphorpentachlorid sowie Phosphoroxychlorid.

Die nachfolgende Kondensationsreaktion des 3-Aminobenzoesäureesters (V) mit dem so erhaltenen Oxazin-6-on (VII) unter Verwendung von Essigsäure als Lösungsmittel bei geeigneter Temperatur liefert das Uracil (VIII).

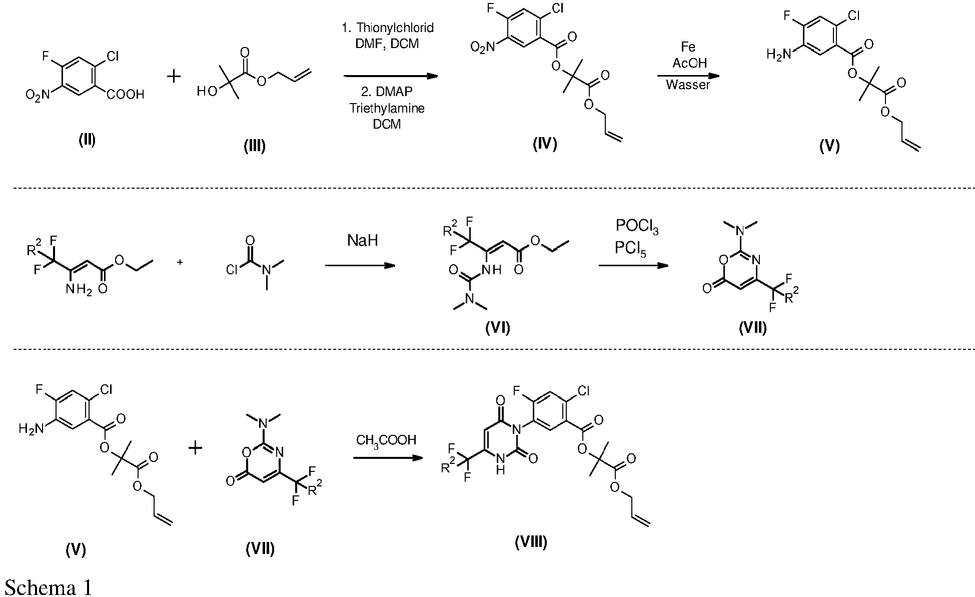

Das so zugängliche Uracil (VIII) wird dann durch nachfolgende N-Alkylierung in das N-Alkyl-N'-Benzoesäureuracil (Ia) überführt, hier beispielhaft aber nicht einschränkend als N-Methylierung dargestellt. Die Alkylierung erfolgt dabei unter Verwendung einer geeigneten Base (z. B. Natriumhydrid, Kalium-tert-butylat oder Kaliumcarbonat) in einem geeigneten polar-aprotischen Lösemittel (z. B. Dichlormethan, Chloroform, N,N-Dimethylacetamid oder N,N-Dimethylformamid). Die selektive Esterspaltung der endständigen Allylestergruppe des N-Alkyl-N'-Benzoesäureuracils (Ia) gelingt unter Verwendung von Phenylsilan in Gegenwart eines geeigneten Pd-Katalysators, z.B. Tetrakis(triphenylphosphin)palladium(0) in Dichlormethan und ergibt die N-Alkyl-N'-Benzoesäureuracile (Ib) in Form der Carbonsäure. Diese kann dann durch Veresterung mit einem geeigneten Alkohol R-OH zu verschiedensten Estervarianten der N-Benzoesäureuracile (Ic) überführt werden.

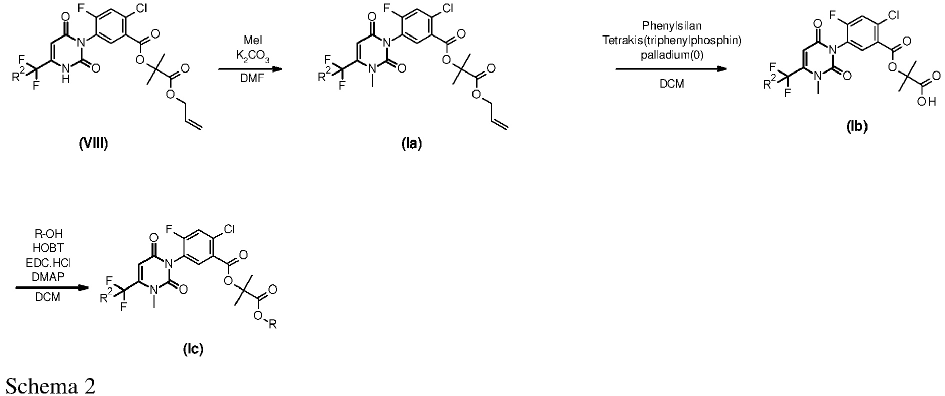

Die Veresterung kann, wie beispielhaft aber nicht einschränkend in Schema 1 dargestellt, unter Vermittlung geeigneter Kupplungsreagenzien (z. B. HOBt = 1-Hydroxybenzotriazol, EDC = 1-Ethyl-3-(3-dimethylaminopropyl)carbodiimid, HATU = O-(7-Azabenzotriazol-1-yl)-N,N,N',N'-tetramethyluronium-hexafluorphosphat, T3P = 2,4,6-Tripropyl-1,3,5,2,4,6-trioxatriphosphorinane-2,4,6-trioxid) und geeigneter Basen (z. B. Diisopropylethylamin, Triethylamin) in einem geeigneten polar-aprotischen Lösemittel (z. B. Dichlormethan, Chloroform) durchgeführt werden. Alternativ kann die Veresterung via Transformation ins Säurechlorid mittels Thionylchlorid und nachfolgender Umsetzung mit dem Alkohol R-OH erfolgen, wobei ein geeignetes polar-aprotischen Lösemittel (z. B. Dichlormethan (DCM), Chloroform, N,N-Dimethylacetamid (DMA) oder N,N-Dimethylformamid (DMF)) Verwendung findet.

Ausgewählte detaillierte Synthesebeispiele für die erfindungsgemäßen Verbindungen der allgemeinen Formeln (I) sind im Folgenden aufgeführt. Die angegebenen Beispielnummern entsprechen den in den nachstehenden Tabellen I.1 bis I.48 genannten Nummerierungen. Die ¹H-NMR-, ¹³C-NMR- und ¹⁹F-NMR-spektroskopischen Daten, die für die in den nachfolgenden Abschnitten beschriebenen chemischen Beispiele angegeben sind, (400 MHz bei ¹H-NMR und 150 MHz bei ¹³C-NMR und 375 MHz bei ¹⁹F-NMR, Lösungsmittel CDCl₃, CD₃OD oder d₆-DMSO, interner Standard: Tetramethylsilan δ = 0.00 ppm), wurden mit einem Gerät der Firma Bruker erhalten, und die bezeichneten Signale haben die nachfolgend aufgeführten Bedeutungen: br = breit(es); s = Singulett, d = Dublett, t = Triplett, dd = Doppeldublett, ddd = Dublett eines Doppeldubletts, m = Multiplett, q = Quartett, quint = Quintett, sext = Sextett, sept = Septett, dq = Doppelquartett, dt = Doppeltriplett. Bei Diastereomerengemischen werden entweder die jeweils signifikanten Signale beider Diastereomere oder das charakteristische Signal des Hauptdiastereomers angegeben. Die verwendeten Abkürzungen für chemische Gruppen haben beispielsweise die nachfolgenden Bedeutungen: Me = CH₃, Et = CH₂CH₃, t-Hex = C(CH₃)₂CH(CH₃)₂, t-Bu = C(CH₃)₃, n-Bu = unverzweigtes Butyl, n-Pr = unverzweigtes Propyl, i-Pr = verzweigtes Propyl, c-Pr = Cyclopropyl, c-Hex = Cyclohexyl.

### Synthesebeispiele:

Nr. I.1-176: 1-(Cyanmethoxy)-2-methyl-1-oxopropan-2-yl-2-chlor-4-fluor-5-[3-methyl-2,6-dioxo-4-(trifluormethyl)-3,6-dihydropyrimidin-1(2H)-yl]benzoat

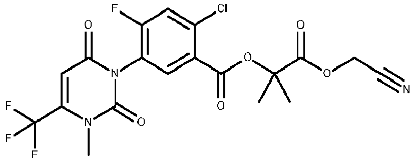

2-Chlor-4-fluoro-5-nitrobenzoesäure (1000 mg, 4.55 mmol;) wird in 40 mL Dichlormethan vorgelegt und mit Oxalylchlorid (752 mg, 5.92 mmol) und katalytischen Mengen von N,N-Dimethylformamid (1 Tropfen) versetzt. Nach 3stündigem Rühren bei 40 Grad Celsius erhielt man eine klare Reaktionslösung, die nachfolgend unter vermindertem Druck eingeengt wurde. Das so erhaltene Säurechlorid wurde in 5 mL Dichlormethan aufgenommen und tropfenweise zu einer Lösung von Allyl 2-hydroxy-2-methylpropanoat (1313 mg, 9.10 mmol), Triethylamin (599 mg, 5.92 mmol) und 4-Dimethylaminoyridin (18.51 mg, 0.15 mmol) in 40 mL Dichlormethan zugetropft. Die Reaktionsmischung wurde 6 Stunden gerührt, nachfolgend wurde die Reaktionsmischung auf Wasser/Dichlormethan gegeben. Nach Trennung der Phasen wurde die organische Phase getrocknet und unter vermindertem Druck eingeengt. Durch säulenchromatographische Reinigung des erhaltenen Rohproduktes wurde 1-(Allyloxy)-2-methyl-1-oxopropan-2-yl-2-chlor-4-fluor-5-nitrobenzoat (620 mg, Reinheit: 95%, 39% der Theorie) erhalten.

Das so zugängliche 1-(Allyloxy)-2-methyl-1-oxopropan-2-yl-2-chlor-4-fluor-5-nitrobenzoat (1640 mg, 4.74 mmol) wurde in 150 mL Eisessig vorgelegt und mit Eisenpulver (2538 mg, 47.4 mmol) versetzt.

Die Reaktionsmischung wurde bei Raumtemperatur 2 Stunden gerührt. Nachfolgend wurde die Reaktionsmischung mit einer Mischung von Wasser und Dichlormethan versetzt und überschüssiges Eisen mittels eines Magneten entfernt. Nach Trennung der Phasen wurde die organische Phase mit Natriumhydrogencarbonat-Lösung gewaschen, und nach erneuter Phasentrennung mittels Filtration durch eine Separatorkartusche getrocknet und unter vermindertem Druck eingeengt. Durch säulenchromatographische Reinigung des erhaltenen Rohproduktes wurde 1-(Allyloxy)-2-methyl-1-oxopropan-2-yl-5-amino-2-chlor-4-fluorbenzoat (1360 mg, Reinheit: 99%, 89% der Theorie) erhalten.

Das so zugängliche 1-(Allyloxy)-2-methyl-1-oxopropan-2-yl-5-amino-2-chlor-4-fluorbenzoat (1040 mg, 3.29 mmol) wurde zusammen mit 2-(Dimethylamino)-4-(trifluormethyl)-6H-1,3-oxazin-6-on (1032 mg, 4.61 mmol; Herstellung gemäß WO2000049002 A1) in 20 mL Essigsäure aufgenommen und 4h bei 110 Grad Celsius gerührt. Nach Stehen über Nacht wurde das Reaktionsgemisch mit Wasser versetzt und anschließend gründlich mit Dichlormethan extrahiert. Die vereinigten organischen Phasen wurden mit Natriumhydrogencarbonat-Lösung gewaschen, nach Phasentrennung über Natriumsulfat getrocknet, abfiltriert und unter vermindertem Druck eingeengt. Durch säulenchromatographische Reinigung des erhaltenen Rohproduktes wurde 1-(Allyloxy)-2-methyl-1-oxopropan-2-yl-2-chlor-5-[4-(trifluormethyl)-2,6-dioxo-3,6-dihydropyrimidin-1(2H)-yl]-4-fluorbenzoat (1430 mg, Reinheit: 95%, 86% der Theorie) in Form eines farblosen Feststoffes erhalten.

Das so erhaltene 1-(Allyloxy)-2-methyl-1-oxopropan-2-yl-2-chlor-5-[4-(trifluormethyl)-2,6-dioxo-3,6-dihydropyrimidin-1(2H)-yl]-4-fluorbenzoat (1430 mg, 2.98 mmol) wurde in abs. N,N-Dimethylformamid (25 mL) gelöst und mit Kaliumcarbonat (2064 mg, 14.9 mol) versetzt. Danach wurde eine Lösung von Methyliodid (2120 mg, 14.9 mol) in abs. N,N-Dimethylformamid (ca. 20 mL) zugegeben und das resultierende Reaktionsgemisch wurde 3 h lang bei Raumtemperatur nachgerührt. Nach vollständigem Umsatz wurde das Reaktionsgemisch auf eine Mischung von Wasser/Dichlormethan gegossen und die organische Phase abgetrennt. Die wäßrige Phase wurde anschließend dreimal mit Dichlormethan extrahiert. Die vereinigten organischen Phasen wurden über Natriumsulfat getrocknet, abfiltriert und unter vermindertem Druck eingeengt. Durch säulenchromatographische Reinigung des erhaltenen Rohproduktes wurde 1-(Allyloxy)-2-methyl-1-oxopropan-2-yl-2-chlor-4-fluor-5-[3-methyl-2,6-dioxo-4-(trifluormethyl)-3,6-dihydropyrimidin-1(2H)-yl]benzoat (1000 mg, Reinheit: 97%, 67% der Theorie) in Form eines farblosen Öles erhalten, welches nach längerem Stehen teilweise kristallisierte.

Das so erhaltene 1-(Allyloxy)-2-methyl-1-oxopropan-2-yl-2-chlor-4-fluor-5-[3-methyl-2,6-dioxo-4-(trifluormethyl)-3,6-dihydropyrimidin-1(2H)-yl]benzoat (1000 mg, 2.02 mmol) wurde unter Argonatmosphäre in 20 mL Dichlormethan gelöst. Bei Raumtemperatur wurden Phenylsilan (439 mg, 4.05 mmol) und nachfolgend Tetrakis(triphenylphosphin)palladium(0) (117 mg, 0.10 mmol) zugesetzt. Die Reaktionsmischung wurde ca. 2h bei Raumtemperatur gerührt. Mittels Dünnschichtchromatographie wurde vollständige Umsetzung detektiert, so dass die Reaktion nachfolgend durch Zugabe von 5 mL Wasser gequencht wurde. Nach Phasentrennung wurde die wässrige Phase mehrfach mit Dichlormethan extrahiert. Die vereinigten organischen Phasen wurden mit gesättigter Kochsalz-Lösung gewaschen und nachfolgend über Natriumsulfat getrocknet. Nach Filtration und Auffangen der organischen Phase wurde das Lösungsmittel im Vakuum entfernt. Die so erhaltene 2-({2-Chlor-4-fluor-5-[3-methyl-2,6-dioxo-4-(trifluormethyl)-3,6-dihydropyrimidin-1(2H)-yl]benzoyl}oxy)-2-methylpropansäure (970 mg, Reinheit: 97%, 90% der Theorie) wurde ohne weitere Aufreinigung in der Folgestufe umgesetzt.

Zu einer Lösung aus Bromacetonitril (41.4 mg, 0.33 mmol) in 2 ml Aceton wurde die so erhaltene 2-({2-Chlor-4-fluor-5-[3-methyl-2,6-dioxo-4-(trifluormethyl)-3,6-dihydropyrimidin-1(2H)-yl]benzoyl}oxy)-2-methylpropansäure (100 mg, 0.22 mmol) zugegeben, anschließend wurde mit Triethylamin (34 mg, 0.33 mmol) versetzt. Der Ansatz wurde 3h bei RT gerührt und über Nacht bei RT stehen gelassen. Nachfolgend wurden 5 ml Dichlormethan und 5 ml Wasser hinzugesetzt. Die Reaktionsmischung wurde mittels einer Separatorkartusche von der wässrigen Phase getrennt und nach Auffangen der organischen Phase wurde das Lösungsmittel im Vakuum entfernt. Durch säulenchromatographische Reinigung des erhaltenen Rohproduktes wurde 1-(Cyanmethoxy)-2-methyl-1-oxopropan-2-yl-2-chlor-4-fluor-5-[3-methyl-2,6-dioxo-4-(trifluormethyl)-3,6-dihydropyrimidin-1(2H)-yl]benzoat (90 mg, Reinheit: 98%, 73% der Theorie) erhalten.

¹H NMR (CDCl₃, ppm): 7.87 (d, 1H), 7.40 (d, 1H), 6.38 (s, 1H), 4.79 (s, 2H), 3.58 (s, 3H), 1.72 (s, 6H).

Nr. I.2-1: 1-(2-Methoxyethoxy)-2-methyl-1-oxopropan-2-yl-2-chlor-5-[4-(difluormethyl)-3-methyl-2,6-dioxo-3,6-dihydropyrimidin-1(2H)-yl]-4-fluorbenzoat

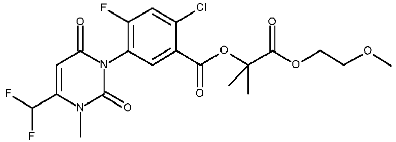

Zu einer Lösung aus 3-Methoxy-1-propanol (34 mg, 0.44 mmol) in 4 ml Dichlormethan wurde 2-({2-Chlor-4-fluor-5-[3-methyl-2,6-dioxo-4-(trifluormethyl)-3,6-dihydropyrimidin-1(2H)-yl]benzoyl}oxy)-2-methylpropansäure (150 mg, 0.34 mmol) zugegeben und anschließend 1-Hydroxy-1H-benzotriazol Hydrat (69 mg, 0.44 mmol), 1-(3-Dimethylaminopropyl)-3-ethylcarbodiimidhydrochlorid (86 mg, 0.44 mmol) und 4-Dimethylaminopyridin (10 mol%) zugegeben. Der Ansatz wurde 5 h bei RT gerührt, über Nacht bei RT stehen gelassen. Dünnschichtchromatographische Reaktionskontrolle zeigte vollständige Umsetzung, so dass die Reaktionsmischung mit ca. 5mL Wasser verrührt wurde. Nach Phasentrennung mittels Separatorkartusche wurde das Lösungsmittel im Vakuum entfernt. Der Rückstand wurde säulenchromatographisch gereinigt (Gradient Essigsäureethylester/n-Heptan) und 1-(2-Methoxyethoxy)-2-methyl-1-oxopropan-2-yl-2-chlor-5-[4-(difluormethyl)-3-methyl-2,6-dioxo-3,6-dihydropyrimidin-1(2H)-yl]-4-fluorbenzoat als farbloses Öl erhalten (132 mg, Reinheit: 97 %, 76% der Theorie) ¹H NMR (CDCl₃, ppm): 7.86 (d, 1H), 7.37 (d, 1H), 6.47 (t, 1H, CHF2), 6.13 (s, 1H), 4.30 (t, 2H), 3.57 (t, 2H), 3.56 (s, 3H), 3.31 (s, 3H), 1.69 (s, 6H).

Nr. I.3-476: 1-[(Isopropylidenamino)oxy]-2-methyl-1-oxopropan-2-yl-2-chlor-5-{4-[chlor(difluor)methyl]-3-methyl-2,6-dioxo-3,6-dihydropyrimidin-1(2H)-yl}-4-fluorbenzoat

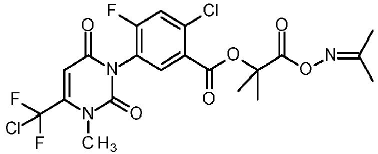

Zu einer Lösung aus Acetonoxim (30 mg, 0.41 mmol) in 4 mL Dichlormethan wurde 2-[(2-Chlor-5-{4-[chlor(difluor)methyl]-3-methyl-2,6-dioxo-3,6-dihydropyrimidin-1(2H)-yl}-4-fluorbenzoyl)oxy]-2-methylpropansäure (150 mg, 0.32 mmol) zugegeben und anschließend 1-Hydroxy-1H-benzotriazol Hydrat (64 mg, 0.41 mmol), 1-(3-Dimethylaminopropyl)-3-ethylcarbodiimidhydrochlorid (80 mg, 0.41 mmol) und 4-Dimethylaminopyridin (10 mol%) zugesetzt. Der Ansatz wurde 5h bei RT gerührt, über Nacht bei RT stehen gelassen. Nachfolgend wurde durch Zugabe von 1 mL Wasser gequencht und die Reaktionsmischung verrührt. Nach Phasentrennung wurde die organische Phase getrocknet und nachfolgend wurde das Lösungsmittel im Vakuum entfernt. Der Rückstand wurde säulenchromatographisch gereinigt (Gradient Essigsäureethylester/n-Heptan) und 1-[(Isopropylidenamino)oxy]-2-methyl-1-oxopropan-2-yl-2-chlor-5-{4-[chlor(difluor)methyl]-3-methyl-2,6-dioxo-3,6-dihydropyrimidin-1(2H)-yl}-4-fluorbenzoat als farbloses Öl erhalten (124 mg, Reinheit: 95 %, Ausbeute 70%)

¹H NMR (CDCl₃, ppm): 7.90 (d, 1H), 7.38 (d, 1H), 6.32 (s, 1H), 3.64 (s, 3H), 2.05 (s, 3H), 1.94 (s, 3H), 1.77 (s, 6H).

In Analogie zu den oben angeführten und an entsprechender Stelle rezitierten Herstellungsbeispielen und unter Berücksichtigung der allgemeinen Angaben zur Herstellung von substituierten N-Benzoesäureuracilen erhält man die nachfolgend genannten Verbindungen. Wenn in Tabelle 1 ein Strukturelement durch eine Strukturformel definiert ist, welches eine gestrichelte Linie enthält, so bedeutet diese gestrichelte Linie, dass an dieser Position die betreffende Gruppe mit dem Rest des Moleküls verbunden ist. Wenn in Tabelle 1 ein Strukturelement durch eine Strukturformel definiert ist, welches einen Pfeil enthält, so steht der Pfeil für eine Bindung der jeweiligen Gruppe Q zur Carbonylgruppe in der allgemeinen Formel (I).

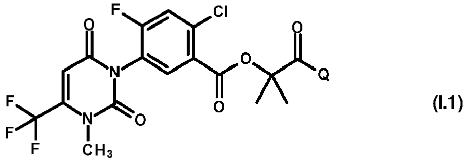

Tabelle I.1: Bevorzugte Verbindungen der Formel (I.1) sind die Verbindungen I.1-1 bis I.1-485, worin der Rest Q die in der jeweiligen Zeile angegebenen Bedeutungen der Tabelle 1 hat. Die Verbindungen I.1-1 bis I.1-485 der Tabelle I.1 sind somit durch die Bedeutung der jeweiligen Einträge Nr.1 bis 485 für Q der Tabelle 1 definiert.

**Tabelle 1:**

| Nr. | Q |
|---|---|
| 1 | Q-1 |
| 2 | Q-2 |
| 3 | Q-3 |
| 4 | Q-4 |
| 5 | Q-5 |
| 6 | Q-6 |
| 7 | Q-7 |
| 8 | Q-8 |
| 9 | Q-9 |
| 10 | Q-10 |
| 11 | Q-11 |
| 12 | Q-12 |
| 13 | Q-13 |
| 14 | Q-14 |
| 15 | Q-15 |
| 16 | Q-16 |
| 17 | Q-17 |
| 18 | Q-18 |
| 19 | Q-19 |
| 20 | Q-20 |
| 21 | Q-21 |
| 22 | Q-22 |
| 23 | Q-23 |
| 24 | Q-24 |
| 25 | Q-25 |
| 26 | Q-26 |
| 27 | Q-27 |
| 28 | Q-28 |
| 29 | Q-29 |
| 30 | Q-30 |
| 31 | Q-31 |
| 32 | Q-32 |
| 33 | Q-33 |
| 34 | Q-34 |
| 35 | Q-35 |
| 36 | Q-36 |
| 37 | Q-37 |
| 38 | Q-38 |
| 39 | Q-39 |
| 40 | Q-40 |
| 41 | Q-41 |
| 42 | Q-42 |
| 43 | Q-43 |
| 44 | Q-44 |
| 45 | Q-45 |
| 46 | Q-46 |
| 47 | Q-47 |
| 48 | Q-48 |
| 49 | Q-49 |
| 50 | Q-50 |
| 51 | Q-51 |
| 52 | Q-52 |
| 53 | Q-53 |
| 54 | Q-54 |
| 55 | Q-55 |
| 56 | Q-56 |
| 57 | Q-57 |
| 58 | Q-58 |
| 59 | Q-59 |
| 60 | Q-60 |
| 61 | Q-61 |
| 62 | Q-62 |
| 63 | Q-63 |
| 64 | Q-64 |
| 65 | Q-65 |
| 66 | Q-66 |
| 67 | Q-67 |
| 68 | Q-68 |
| 69 | Q-69 |
| 70 | Q-70 |
| 71 | Q-71 |
| 72 | Q-72 |
| 73 | Q-73 |
| 74 | Q-74 |
| 75 | Q-75 |
| 76 | Q-76 |
| 77 | Q-77 |
| 78 | Q-78 |
| 79 | Q-79 |
| 80 | Q-80 |
| 81 | Q-81 |
| 82 | Q-82 |
| 83 | Q-83 |
| 84 | Q-84 |
| 85 | Q-85 |
| 86 | Q-86 |
| 87 | Q-87 |
| 88 | Q-88 |
| 89 | Q-89 |
| 90 | Q-90 |
| 91 | Q-91 |
| 92 | Q-92 |
| 93 | Q-93 |
| 94 | Q-94 |
| 95 | Q-95 |
| 96 | Q-96 |
| 97 | Q-97 |
| 98 | Q-98 |
| 99 | Q-99 |
| 100 | Q-100 |
| 101 | Q-101 |
| 102 | Q-102 |
| 103 | Q-103 |
| 104 | Q-104 |
| 105 | Q-105 |
| 106 | Q-106 |
| 107 | Q-107 |
| 108 | Q-108 |
| 109 | Q-109 |
| 110 | Q-110 |
| 111 | Q-111 |
| 112 | Q-112 |
| 113 | Q-113 |
| 114 | Q-114 |
| 115 | Q-115 |
| 116 | Q-116 |
| 117 | Q-117 |
| 118 | Q-118 |
| 119 | Q-119 |
| 120 | Q-120 |
| 121 | Q-121 |
| 122 | Q-122 |
| 123 | Q-123 |
| 124 | Q-124 |
| 125 | Q-125 |
| 126 | Q-126 |
| 127 | Q-127 |
| 128 | Q-128 |
| 129 | Q-129 |
| 130 | Q-130 |
| 131 | Q-131 |
| 132 | Q-132 |
| 133 | Q-133 |
| 134 | Q-134 |
| 135 | Q-135 |
| 136 | Q-136 |
| 137 | Q-137 |
| 138 | Q-138 |
| 139 | Q-139 |
| 140 | Q-140 |
| 141 | Q-141 |
| 142 | Q-142 |
| 143 | Q-143 |
| 144 | Q-144 |
| 145 | Q-145 |
| 146 | Q-146 |
| 147 | Q-147 |
| 148 | Q-148 |
| 149 | Q-149 |
| 150 | Q-150 |
| 151 | Q-151 |
| 152 | Q-152 |
| 153 | Q-153 |
| 154 | Q-154 |
| 155 | Q-155 |
| 156 | Q-156 |
| 157 | Q-157 |
| 158 | Q-158 |
| 159 | Q-159 |
| 160 | Q-160 |
| 161 | Q-161 |
| 162 | Q-162 |
| 163 | Q-163 |
| 164 | Q-164 |
| 165 | Q-165 |
| 166 | Q-166 |
| 167 | Q-167 |
| 168 | Q-168 |
| 169 | Q-169 |
| 170 | Q-170 |
| 171 | Q-171 |
| 172 | Q-172 |
| 173 | Q-173 |
| 174 | Q-174 |
| 175 | Q-175 |
| 176 | Q-176 |
| 177 | Q-177 |
| 178 | Q-178 |
| 179 | Q-179 |
| 180 | Q-180 |
| 181 | Q-181 |
| 182 | Q-182 |
| 183 | Q-183 |
| 184 | Q-184 |
| 185 | Q-185 |
| 186 | Q-186 |
| 187 | Q-187 |
| 188 | Q-188 |
| 189 | Q-189 |
| 190 | Q-190 |
| 191 | Q-191 |
| 192 | Q-192 |
| 193 | Q-193 |
| 194 | Q-194 |
| 195 | Q-195 |
| 196 | Q-196 |
| 197 | Q-197 |
| 198 | Q-198 |
| 199 | Q-199 |
| 200 | Q-200 |
| 201 | Q-201 |
| 202 | Q-202 |
| 203 | Q-203 |
| 204 | Q-204 |
| 205 | Q-205 |
| 206 | Q-206 |
| 207 | Q-207 |
| 208 | Q-208 |
| 209 | Q-209 |
| 210 | Q-210 |
| 211 | Q-211 |
| 212 | Q-212 |
| 213 | Q-213 |
| 214 | Q-214 |
| 215 | Q-215 |
| 216 | Q-216 |
| 217 | Q-217 |
| 218 | Q-218 |
| 219 | Q-219 |
| 220 | Q-220 |
| 221 | Q-221 |
| 222 | Q-222 |
| 223 | Q-223 |
| 224 | Q-224 |
| 225 | Q-225 |
| 226 | Q-226 |
| 227 | Q-227 |
| 228 | Q-228 |
| 229 | Q-229 |
| 230 | Q-230 |
| 231 | Q-231 |
| 232 | Q-232 |
| 233 | Q-233 |
| 234 | Q-234 |
| 235 | Q-235 |
| 236 | Q-236 |
| 237 | Q-237 |
| 238 | Q-238 |
| 239 | Q-239 |
| 240 | Q-240 |
| 241 | Q-241 |
| 242 | Q-242 |
| 243 | Q-243 |
| 244 | Q-244 |
| 245 | Q-245 |
| 246 | Q-246 |
| 247 | Q-247 |
| 248 | Q-248 |
| 249 | Q-249 |
| 250 | Q-250 |
| 251 | Q-251 |
| 252 | Q-252 |
| 253 | Q-253 |
| 254 | Q-254 |
| 255 | Q-255 |
| 256 | Q-256 |
| 257 | Q-257 |
| 258 | Q-258 |
| 259 | Q-259 |
| 260 | Q-260 |
| 261 | Q-261 |
| 262 | Q-262 |
| 263 | Q-263 |
| 264 | Q-264 |
| 265 | Q-265 |
| 266 | Q-266 |
| 267 | Q-267 |
| 268 | Q-268 |
| 269 | Q-269 |
| 270 | Q-270 |
| 271 | Q-271 |
| 272 | Q-272 |
| 273 | Q-273 |
| 274 | Q-274 |
| 275 | Q-275 |
| 276 | Q-276 |
| 277 | Q-277 |
| 278 | Q-278 |
| 279 | Q-279 |
| 280 | Q-280 |
| 281 | Q-281 |
| 282 | Q-282 |
| 283 | Q-283 |
| 284 | Q-284 |
| 285 | Q-285 |
| 286 | Q-286 |
| 287 | Q-287 |
| 288 | Q-288 |
| 289 | Q-289 |
| 290 | Q-290 |
| 291 | Q-291 |
| 292 | Q-292 |
| 293 | Q-293 |
| 294 | Q-294 |
| 295 | Q-295 |
| 296 | Q-296 |
| 297 | Q-297 |
| 298 | Q-298 |
| 299 | Q-299 |
| 300 | Q-300 |
| 301 | Q-301 |
| 302 | Q-302 |
| 303 | Q-303 |
| 304 | Q-304 |
| 305 | Q-305 |
| 306 | Q-306 |
| 307 | Q-307 |
| 308 | Q-308 |
| 309 | Q-309 |
| 310 | Q-310 |
| 311 | Q-311 |
| 312 | Q-312 |
| 313 | Q-313 |
| 314 | Q-314 |
| 315 | Q-315 |
| 316 | Q-316 |
| 317 | Q-317 |
| 318 | Q-318 |
| 319 | Q-319 |
| 320 | Q-320 |
| 321 | Q-321 |
| 322 | Q-322 |
| 323 | Q-323 |
| 324 | Q-324 |
| 325 | Q-325 |
| 326 | Q-326 |
| 327 | Q-327 |
| 328 | Q-328 |
| 329 | Q-329 |
| 330 | Q-330 |
| 331 | Q-331 |
| 332 | Q-332 |
| 333 | Q-333 |
| 334 | Q-334 |
| 335 | Q-335 |
| 336 | Q-336 |
| 337 | Q-337 |
| 338 | Q-338 |
| 339 | Q-339 |
| 340 | Q-340 |
| 341 | Q-341 |
| 342 | Q-342 |
| 343 | Q-343 |
| 344 | Q-344 |
| 345 | Q-345 |
| 346 | Q-346 |
| 347 | Q-347 |
| 348 | Q-348 |
| 349 | Q-349 |
| 350 | Q-350 |
| 351 | Q-351 |
| 352 | Q-352 |
| 353 | Q-353 |
| 354 | Q-354 |
| 355 | Q-355 |
| 356 | Q-356 |
| 357 | Q-357 |
| 358 | Q-358 |
| 359 | Q-359 |
| 360 | Q-360 |
| 361 | Q-361 |
| 362 | Q-362 |
| 363 | Q-363 |
| 364 | Q-364 |
| 365 | Q-365 |
| 366 | Q-366 |
| 367 | Q-367 |
| 368 | Q-368 |
| 369 | Q-369 |
| 370 | Q-370 |
| 371 | Q-371 |
| 372 | Q-372 |
| 373 | Q-373 |
| 374 | Q-374 |
| 375 | Q-375 |
| 376 | Q-376 |
| 377 | Q-377 |
| 378 | Q-378 |
| 379 | Q-379 |
| 380 | Q-380 |
| 381 | Q-381 |
| 382 | Q-382 |
| 383 | Q-383 |
| 384 | Q-384 |
| 385 | Q-385 |
| 386 | Q-386 |
| 387 | Q-387 |
| 388 | Q-388 |
| 389 | Q-389 |
| 390 | Q-390 |
| 391 | Q-391 |
| 392 | Q-392 |
| 393 | Q-393 |
| 394 | Q-394 |
| 395 | Q-395 |
| 396 | Q-396 |
| 397 | Q-397 |
| 398 | Q-398 |
| 399 | Q-399 |
| 400 | Q-400 |
| 401 | Q-401 |
| 402 | Q-402 |
| 403 | Q-403 |
| 404 | Q-404 |
| 405 | Q-405 |
| 406 | Q-406 |
| 407 | Q-407 |
| 408 | Q-408 |
| 409 | Q-409 |
| 410 | Q-410 |
| 411 | Q-411 |
| 412 | Q-412 |
| 413 | Q-413 |
| 414 | Q-414 |
| 415 | Q-415 |
| 416 | Q-416 |
| 417 | Q-417 |
| 418 | Q-418 |
| 419 | Q-419 |
| 420 | Q-420 |
| 421 | Q-421 |
| 422 | Q-422 |
| 423 | Q-423 |
| 424 | Q-424 |
| 425 | Q-425 |
| 426 | Q-426 |
| 427 | Q-427 |
| 428 | Q-428 |
| 429 | Q-429 |
| 430 | Q-430 |
| 431 | Q-431 |
| 432 | Q-432 |
| 433 | Q-433 |
| 434 | Q-434 |
| 435 | Q-435 |
| 436 | Q-436 |
| 437 | Q-437 |
| 438 | Q-438 |
| 439 | Q-439 |
| 440 | Q-440 |
| 441 | Q-441 |
| 442 | Q-442 |
| 443 | Q-443 |
| 444 | Q-444 |
| 445 | Q-445 |
| 446 | Q-446 |
| 447 | Q-447 |
| 448 | Q-448 |
| 449 | Q-449 |
| 450 | Q-450 |
| 451 | Q-451 |
| 452 | Q-452 |
| 453 | Q-453 |
| 454 | Q-454 |
| 455 | Q- 455 |
| 456 | Q-456 |
| 457 | Q-457 |
| 458 | Q-458 |
| 459 | Q-459 |
| 460 | Q-460 |
| 461 | Q-461 |
| 462 | Q-462 |
| 463 | Q-463 |
| 464 | Q-464 |
| 465 | Q-465 |
| 466 | Q-466 |
| 467 | Q-467 |
| 468 | Q-468 |
| 469 | Q-469 |
| 470 | Q-470 |
| 471 | Q-471 |
| 472 | Q-472 |
| 473 | Q-473 |
| 474 | Q-474 |
| 475 | Q-475 |
| 476 | Q-476 |
| 477 | Q-477 |
| 478 | Q-478 |
| 479 | Q-479 |
| 480 | Q-480 |
| 481 | Q-481 |
| 482 | Q-482 |
| 483 | Q-483 |
| 484 | Q-484 |
| 485 | Q-485 |

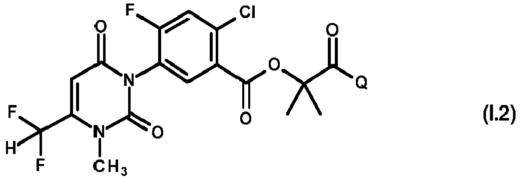

Tabelle I.2: Bevorzugte Verbindungen der Formel (I.2) sind die Verbindungen I.2-1 bis I.2-485, worin der Rest Q die in der jeweiligen Zeile angegebenen Bedeutungen der Tabelle 1 hat. Die Verbindungen I.2-1 bis I.2-485 der Tabelle I.2 sind somit durch die Bedeutung der jeweiligen Einträge Nr. 1 bis 485 für Q der Tabelle 1 definiert.

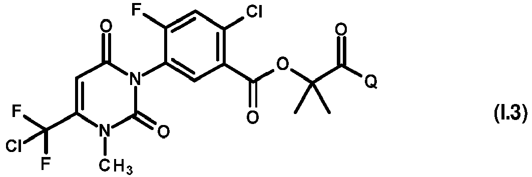

Tabelle I.3: Bevorzugte Verbindungen der Formel (I.3) sind die Verbindungen I.3-1 bis I.3-485, worin der Rest Q die in der jeweiligen Zeile angegebenen Bedeutungen der Tabelle 1 hat. Die Verbindungen I.3-1 bis I.3-485 der Tabelle I.3 sind somit durch die Bedeutung der jeweiligen Einträge Nr. 1 bis 485 für Q der Tabelle 1 definiert.

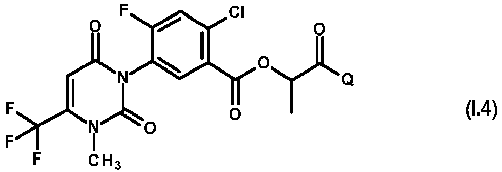

Tabelle I.4: Bevorzugte Verbindungen der Formel (I.4) sind die Verbindungen I.4-1 bis I.4-485, worin der Rest Q die in der jeweiligen Zeile angegebenen Bedeutungen der Tabelle 1 hat. Die Verbindungen I.4-1 bis I.4-485 der Tabelle I.4 sind somit durch die Bedeutung der jeweiligen Einträge Nr. 1 bis 485 für Q der Tabelle 1 definiert.

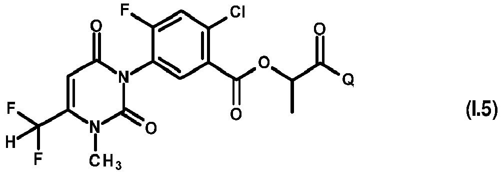

Tabelle I.5: Bevorzugte Verbindungen der Formel (I.5) sind die Verbindungen I.5-1 bis I.5-485, worin der Rest Q die in der jeweiligen Zeile angegebenen Bedeutungen der Tabelle 1 hat. Die Verbindungen I.5-1 bis I.5-485 der Tabelle I.7 sind somit durch die Bedeutung der jeweiligen Einträge Nr. 1 bis 485 für Q der Tabelle 1 definiert.

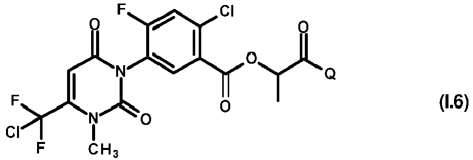

Tabelle I.6: Bevorzugte Verbindungen der Formel (I.6) sind die Verbindungen I.6-1 bis I.6-485, worin der Rest Q die in der jeweiligen Zeile angegebenen Bedeutungen der Tabelle 1 hat. Die Verbindungen

I.6-1 bis I.6-485 der Tabelle I.6 sind somit durch die Bedeutung der jeweiligen Einträge Nr. 1 bis 485 für Q der Tabelle 1 definiert.

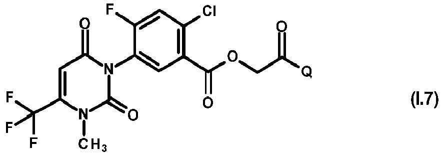

Tabelle I.7: Bevorzugte Verbindungen der Formel (I.7) sind die Verbindungen I.7-1 bis I.7-485, worin der Rest Q die in der jeweiligen Zeile angegebenen Bedeutungen der Tabelle 1 hat. Die Verbindungen I.7-1 bis I.7-485 der Tabelle I.7 sind somit durch die Bedeutung der jeweiligen Einträge Nr. 1 bis 485 für Q der Tabelle 1 definiert.

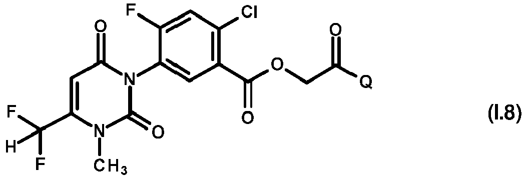

Tabelle I.8: Bevorzugte Verbindungen der Formel (I.8) sind die Verbindungen I.8-1 bis I.8-485, worin der Rest Q die in der jeweiligen Zeile angegebenen Bedeutungen der Tabelle 1 hat. Die Verbindungen I.8-1 bis I.8-485 der Tabelle I.8 sind somit durch die Bedeutung der jeweiligen Einträge Nr. 1 bis 485 für Q der Tabelle 1 definiert.

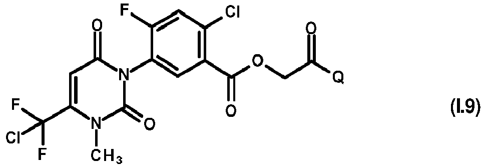

Tabelle I.9: Bevorzugte Verbindungen der Formel (I.9) sind die Verbindungen I.9-1 bis I.9-485, worin der Rest Q die in der jeweiligen Zeile angegebenen Bedeutungen der Tabelle 1 hat. Die Verbindungen I.9-1 bis I.9-485 der Tabelle I.9 sind somit durch die Bedeutung der jeweiligen Einträge Nr. 1 bis 485 für Q der Tabelle 1 definiert.

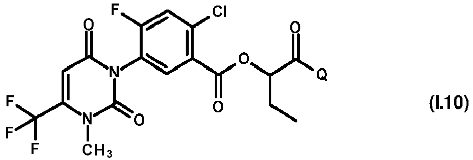

Tabelle I.10: Bevorzugte Verbindungen der Formel (I.10) sind die Verbindungen I.10-1 bis I.10-485, worin der Rest Q die in der jeweiligen Zeile angegebenen Bedeutungen der Tabelle 1 hat. Die Verbindungen I.10-1 bis I.10-485 der Tabelle I.10 sind somit durch die Bedeutung der jeweiligen Einträge Nr. 1 bis 485 für Q der Tabelle 1 definiert.

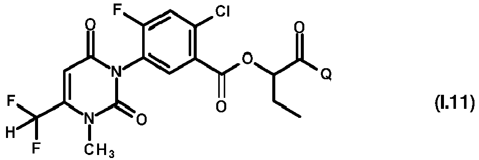

Tabelle I.11: Bevorzugte Verbindungen der Formel (I.11) sind die Verbindungen I.11-1 bis I.11-485, worin der Rest Q die in der jeweiligen Zeile angegebenen Bedeutungen der Tabelle 1 hat. Die Verbindungen I.11-1 bis I.11-485 der Tabelle I.11 sind somit durch die Bedeutung der jeweiligen Einträge Nr. 1 bis 485 für Q der Tabelle 1 definiert.

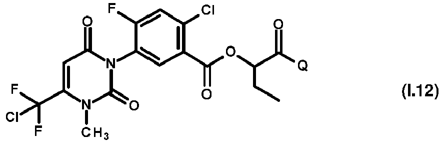

Tabelle I.12: Bevorzugte Verbindungen der Formel (I.12) sind die Verbindungen I.12-1 bis I.12-485, worin der Rest Q die in der jeweiligen Zeile angegebenen Bedeutungen der Tabelle 1 hat. Die Verbindungen 1.12-1 bis I.12-485 der Tabelle I.12 sind somit durch die Bedeutung der jeweiligen Einträge Nr. 1 bis 485 für Q der Tabelle 1 definiert.

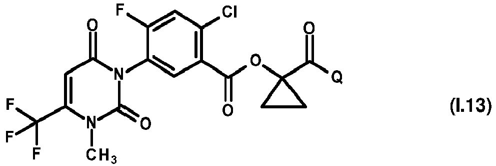

Tabelle I.13: Bevorzugte Verbindungen der Formel (I.13) sind die Verbindungen I.13-1 bis I.13-485, worin der Rest Q die in der jeweiligen Zeile angegebenen Bedeutungen der Tabelle 1 hat. Die Verbindungen I.13-1 bis I.13-485 der Tabelle I.13 sind somit durch die Bedeutung der jeweiligen Einträge Nr. 1 bis 485 für Q der Tabelle 1 definiert.

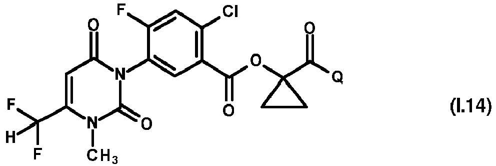

Tabelle I.14: Bevorzugte Verbindungen der Formel (I.14) sind die Verbindungen I.14-1 bis I.14-485, worin der Rest Q die in der jeweiligen Zeile angegebenen Bedeutungen der Tabelle 1 hat. Die Verbindungen 1.14-1 bis I.14-485 der Tabelle I.14 sind somit durch die Bedeutung der jeweiligen Einträge Nr. 1 bis 485 für Q der Tabelle 1 definiert.

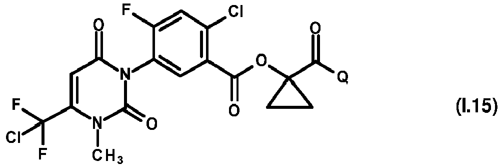

Tabelle I.15: Bevorzugte Verbindungen der Formel (I.15) sind die Verbindungen I.15-1 bis I.15-485, worin der Rest Q die in der jeweiligen Zeile angegebenen Bedeutungen der Tabelle 1 hat. Die Verbindungen I.15.-1 bis I.15-485 der Tabelle I.15 sind somit durch die Bedeutung der jeweiligen Einträge Nr. 1 bis 485 für Q der Tabelle 1 definiert.

### a) klassische NMR-Interpretation

### Beispiel Nr.: I.1-1:

¹H NMR (CDCl₃, ppm): 7.86 (d, 1H), 7.38 (d, 1H), 6.38 (s, 1H), 4.30 (t, 2H), 3.57 (m, 5H), 3.31 (s, 3H), 1.70 (s, 6H).

### Beispiel Nr.: I.1-41:

¹H NMR (CDCl₃, ppm): 7.84 (d, 1H), 7.39 (d, 1H), 6.38 (s, 1H), 4.39 (t, 2H), 4.16 (t, 3H), 3.57 (s, 3H), 1.70 (s, 6H).

### Beispiel Nr.: I.1-2:

¹H NMR (CDCl₃, ppm): 7.86 (d, 1H), 7.38 (d, 1H), 6.37 (s, 1H), 4.30 (m, 2H), 3.61 (t, 2H), 3.57 (s, 3H), 3.48 (q, 2H), 1.69 (s, 6H), 1.13 (t, 3H).

### Beispiel Nr.: I.1-476:

¹H NMR (CDCl₃, ppm): 7.89 (d, 1H), 7.38 (d, 1H), 6.38 (s, 1H), 3.57 (s, 3H), 2.04 (s, 3H), 1.94 (s, 3H), 1.69 (s, 6H).

### Beispiel Nr.: I.1-286:

¹H NMR (CDCl₃, ppm): 8.54 (t, 1H), 7.89 (d, 1H), 7.68 (dt, 1H), 7.36 (m, 2H), 7.20 (dd, 1H), 6.38 (s, 1H), 5.31 (s, 2H), 3.57 (s, 3H), 1.74 (s, 6H).

### Beispiel Nr.: I.1-301:

¹H NMR (CDCl₃, ppm): 9.13 (dd, 1H), 7.84 (d, 1H), 7.57 (dd, 1H), 7.49 (dd, 2H), 7.38 (d, 1H), 6.38 (s, 1H), 5.55 (s, 2H), 3.57 (s, 3H), 1.74 (s, 6H).

### b) NMR-Peak-Listenverfahren

Die ¹H-NMR-Daten ausgewählter Beispiele können auch in Form von ¹H-NMR-Peaklisten notiert werden. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet. Die Peakliste eines Beispieles hat daher die Form:
δ ₁ (Intensität₁⁾; δ₂ (Intensität₂);........; δᵢ (Intensitätᵢ⁾; ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden. Zur Kalibrierung der chemischen Verschiebung von 1H-NMR-Spektren benutzen wir Tetramethylsilan und/oder die chemische Verschiebung des Lösungsmittels, besonders im Falle von Spektren, die in DMSO gemessen werden. Daher kann in NMR-Peaklisten der Tetramethylsilan-Peak vorkommen, muss es aber nicht. Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden. Darüber hinaus können sie wie klassische ¹H-NMR-Ausdrucke Lösungsmittelsignale, Signale von Stereoisomeren der Zielver-bindungen, die ebenfalls Gegenstand der Erfindung sind, und/oder Peaks von Verunreinigungen zeigen. Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen. Die Peaks von Stereoisomeren der Zielverbindungen und/oder Peaks von Verunreinigungen haben gewöhnlich im Durchschnitt eine geringere Intensität als die Peaks der Zielverbindungen (zum Beispiel mit einer Reinheit von >90%). Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen. Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation. Weitere Details zu ¹H-NMR-Peaklisten können der Research Disclosure Database Number 564025 entnommen werden.

### Beispiel Nr.:

### 1.2-1: ¹H-NMR (400.6 MHz, CDCl3):

δ= 7.8674 (1.8); 7.8481 (1.8); 7.3816 (1.8); 7.3587 (1.8); 7.2622 (9.1); 6.6041 (0.6); 6.4727 (1.4); 6.3413 (0.7); 6.1314 (2.2); 4.3148 (1.9); 4.3053 (1.4); 4.3028 (2.1); 4.2996 (1.2); 4.2905 (2.1); 3.5850 (2.1); 3.5757 (1.3); 3.5728 (2.2); 3.5701 (1.3); 3.5607 (2.1); 3.5499 (6.7); 3.3152 (16.0); 1.6926 (10.6); 1.6901 (10.1); 1.5721 (1.6); -0.0002 (7.2)

### I.2-444: ¹H-NMR (400.6 MHz, CDCl3):

δ= 7.8525 (2.6); 7.8334 (2.7); 7.3948 (2.8); 7.3719 (2.8); 7.2612 (13.3); 6.6037 (1.0); 6.4722 (2.1); 6.3409 (1.1); 6.1380 (3.3); 4.5659 (1.4); 4.5451 (4.6); 4.5243 (4.7); 4.5035 (1.6); 3.5539 (10.3); 1.7167 (16.0); 1.7131 (14.6); 1.5517 (3.8); -0.0002 (10.5)

### I.2-176: ¹H-NMR (400.6 MHz, CDCl3):

δ= 7.8773 (2.7); 7.8582 (2.8); 7.4025 (2.8); 7.3797 (2.8); 7.2616 (14.0); 6.6071 (1.0); 6.4758 (2.2); 6.3445 (1.1); 6.1407 (3.4); 4.7881 (14.8); 3.5552 (10.5); 2.0449 (0.7); 1.7159 (16.0); 1.7131 (15.8); 1.5555 (3.5); -0.0002 (9.4)

### I.2-286: ¹H-NMR (400.6 MHz, CDCl3):

δ= 8.5560 (1.1); 8.5538 (1.2); 8.5518 (1.3); 8.5495 (1.1); 8.5439 (1.2); 8.5416 (1.3); 8.5396 (1.3); 8.5374 (1.1); 7.8635 (3.1); 7.8442 (3.1); 7.7062 (0.8); 7.7017 (0.8); 7.6869 (1.7); 7.6825 (1.7); 7.6676 (1.0); 7.6632 (1.0); 7.3762 (3.2); 7.3534 (3.9); 7.3376 (1.6); 7.2624 (16.9); 7.2184 (0.9); 7.2158 (0.9); 7.2062 (1.0); 7.2049 (0.9); 7.2035 (1.0); 7.1995 (1.0); 7.1968 (0.9); 7.1874 (0.9); 7.1847 (0.8); 6.6036 (1.1); 6.4721 (2.4); 6.3408 (1.2); 6.1313 (3.7); 5.3117 (9.4); 3.5483 (11.5); 1.7409 (15.7); 1.7367 (16.0); -0.0002 (13.2); -0.0084 (0.6)

### I.2-476: ¹H-NMR (400.6 MHz, CDCl3):

δ= 7.8959 (2.0); 7.8766 (2.0); 7.3855 (2.0); 7.3627 (2.1); 7.2630 (10.5); 6.6145 (0.7); 6.4831 (1.5); 6.3518 (0.8); 6.1327 (2.5); 3.5487 (7.5); 2.0507 (14.5); 1.9348 (16.0); 1.7611 (15.5); 1.5751 (0.5); - 0.0002 (6.8)

### 1.3-1: ¹H-NMR (400.6 MHz, CDCl3):

δ= 7.8747 (1.9); 7.8554 (1.9); 7.3906 (1.9); 7.3676 (2.0); 7.2615 (9.4); 6.3167 (5.0); 4.3167 (2.0); 4.3073 (1.3); 4.3047 (2.2); 4.3015 (1.3); 4.2925 (2.2); 3.6387 (6.6); 3.6356 (3.9); 3.5858 (2.1); 3.5765 (1.3); 3.5737 (2.2); 3.5710 (1.3); 3.5616 (2.0); 3.4008 (0.7); 3.3157 (16.0); 1.6962 (10.3); 1.6929 (10.3); 1.5596 (3.3); 1.2643 (0.5); 1.2596 (0.5); 0.8821 (0.8); -0.0002 (9.4)

### I.3-176: ¹H-NMR (400.6 MHz, CDCl3):

δ= 7.8855 (2.8); 7.8664 (2.8); 7.4114 (2.9); 7.3886 (2.9); 7.2607 (20.0); 6.3259 (7.2); 4.7898 (14.5); 3.6449 (10.1); 3.6418 (5.9); 1.7195 (15.8); 1.7161 (16.0); 1.5444 (7.6); -0.0002 (17.9); -0.0084 (0.9)

### I.3-286: ¹H-NMR (400.6 MHz, CDCl3):

δ= 8.5568 (1.1); 8.5547 (1.2); 8.5528 (1.3); 8.5506 (1.1); 8.5448 (1.2); 8.5426 (1.3); 8.5407 (1.3); 8.5384 (1.1); 7.8695 (3.1); 7.8503 (3.1); 7.7052 (0.9); 7.7007 (0.9); 7.6858 (1.8); 7.6814 (1.7); 7.6666 (1.1); 7.6622 (1.0); 7.3848 (3.2); 7.3618 (3.5); 7.3565 (2.0); 7.3368 (1.7); 7.2630 (19.4); 7.2186 (1.0); 7.2162 (1.0); 7.2065 (1.0); 7.2039 (1.0); 7.1999 (1.0); 7.1973 (0.9); 7.1876 (0.9); 7.1851 (0.9); 6.3160 (8.4); 5.3110 (9.8); 3.6370 (11.0); 3.6339 (6.2); 1.7437 (16.0); 1.7389 (16.0); -0.0002 (9.0)

### I.3-444: ¹H-NMR (400.6 MHz, CDCl3):

δ= 7.8618 (2.7); 7.8427 (2.7); 7.4042 (2.8); 7.3814 (2.8); 7.2618 (9.4); 6.3232 (7.3); 4.5671 (1.4); 4.5463 (4.5); 4.5255 (4.7); 4.5047 (1.6); 3.6446 (6.8); 3.6416 (10.1); 3.6385 (5.2); 1.7200 (16.0); 1.7160 (14.5); 1.5594 (2.2); -0.0002 (5.5)

### I.3-476: ¹H-NMR (400.6 MHz, CDCl3):

δ= 7.9051 (1.8); 7.8859 (1.9); 7.3952 (1.9); 7.3724 (2.0); 7.2616 (18.5); 6.3167 (5.5); 3.6388 (6.7); 3.6357 (3.7); 2.0533 (14.2); 2.0455 (0.6); 1.9374 (16.0); 1.7653 (13.1); -0.0002 (13.3); -0.0085 (0.5)

Gegenstand der vorliegenden Erfindung ist weiterhin die Verwendung einer oder mehrerer erfindungsgemäßer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (I.1-1) bis (I.15-485) und/oder deren Salze, jeweils wie oben definiert, als Herbizid und/oder Pflanzenwachstumsregulator, vorzugsweise in Kulturen von Nutz- und/oder Zierpflanzen.

Gegenstand der vorliegenden Erfindung ist ferner ein Verfahren zur Bekämpfung von Schadpflanzen und/oder zur Wachstumsregulierung von Pflanzen, dadurch gekennzeichnet, dass eine wirksame Menge
- einer oder mehrerer erfindungsgemäßer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (1.1-1) bis (1.15-485) und/oder deren Salze, jeweils wie oben definiert, oder
- eines erfindungsgemäßen Mittels, wie nachstehend definiert,
auf die (Schad)Pflanzen, (Schad)Pflanzensamen, den Boden, in dem oder auf dem die (Schad)Pflanzen wachsen, oder die Anbaufläche appliziert wird.

Gegenstand der vorliegenden Erfindung ist auch ein Verfahren zur Bekämpfung von unerwünschten Pflanzen, vorzugsweise in Nutzpflanzenkulturen, dadurch gekennzeichnet, dass eine wirksame Menge
- einer oder mehrerer Verbindungen der allgemeinen Formel (I) und/oder deren Salze, wie oben definiert, vorzugsweise in einer der als bevorzugt bzw. besonders bevorzugt gekennzeichneten Ausgestaltung, insbesondere einer oder mehrerer Verbindungen der Formeln (1.1-1) bis (I.15-485) und/oder deren Salze, jeweils wie oben definiert, oder
- eines erfindungsgemäßen Mittels, wie nachstehend definiert,
auf unerwünschte Pflanzen (z.B. Schadpflanzen wie mono- oder dikotyle Unkräuter oder unerwünschte Kulturpflanzen), das Saatgut der unerwünschten Pflanzen (d.h. Pflanzensamen, z.B. Körner, Samen oder vegetative Vermehrungsorgane wie Knollen oder Sprossteile mit Knospen), den Boden, in dem oder auf dem die unerwünschte Pflanzen wachsen, (z.B. den Boden von Kulturland oder Nicht-Kulturland) oder die Anbaufläche (d.h. Fläche, auf der die unerwünschte Pflanzen wachsen werden) appliziert wird.

Gegenstand der vorliegenden Erfindung ist ferner auch Verfahren zur Bekämpfung zur Wachstumsregulierung von Pflanzen, vorzugsweise von Nutzpflanzen, dadurch gekennzeichnet, dass eine wirksame Menge
- einer oder mehrerer Verbindungen der allgemeinen Formel (I) und/oder deren Salzen, wie oben definiert, vorzugsweise in einer der als bevorzugt bzw. besonders bevorzugt gekennzeichneten Ausgestaltung, insbesondere einer oder mehrerer Verbindungen der Formeln (I.1-1) bis (1.15-485) und/oder deren Salze, jeweils wie oben definiert, oder
- eines erfindungsgemäßen Mittels, wie nachstehend definiert,
die Pflanze, das Saatgut der Pflanze (d.h. Pflanzensamen, z.B. Körner, Samen oder vegetative Vermehrungsorgane wie Knollen oder Sprossteile mit Knospen), den Boden, in dem oder auf dem die Pflanzen wachsen, (z.B. den Boden von Kulturland oder Nicht-Kulturland) oder die Anbaufläche (d.h. Fläche, auf der die Pflanzen wachsen werden) appliziert wird.

Dabei können die erfindungsgemäßen Verbindungen der allgemeinen Formel (I) bzw. die erfindungsgemäßen Mittel z.B. im Vorsaat- (ggf. auch durch Einarbeitung in den Boden), Vorauflauf- und/oder Nachauflaufverfahren ausgebracht werden. Im Einzelnen seien beispielhaft einige Vertreter der mono- und dikotylen Unkrautflora genannt, die durch die die erfindungsgemäßen Verbindungen kontrolliert werden können, ohne dass durch die Nennung eine Beschränkung auf bestimmte Arten erfolgen soll.

Vorzugsweise werden in einem erfindungsgemäßen Verfahren zur Bekämpfung von Schadpflanzen oder zur Wachstumsregulierung von Pflanzen eine oder mehrere Verbindungen der allgemeinen Formel (I) und/oder deren Salze zur Bekämpfung von Schadpflanzen oder zur Wachstumsregulierung in Kulturen von Nutzpflanzen oder Zierpflanzen eingesetzt, wobei die Nutzpflanzen oder Zierpflanzen in einer bevorzugten Ausgestaltung transgene Pflanzen sind.

Die erfindungsgemäßen Verbindungen allgemeinen Formel (I) und/oder deren Salze eignen sich zur Bekämpfung der folgenden Gattungen von monokotylen und dikotylen Schadpflanzen:
Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Dikotyle Schadpflanzen der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen der allgemeinen Formel (I) vor dem Keimen der Schadpflanzen (Ungräser und/oder Unkräuter) auf die Erdoberfläche appliziert (Vorauflaufverfahren), so wird entweder das Auflaufen der Ungras- bzw. Unkrautkeimlinge vollständig verhindert oder diese wachsen bis zum Keimblattstadium heran, stellen jedoch dann ihr Wachstum ein und sterben schließlich nach Ablauf von drei bis vier Wochen vollkommen ab.

Bei Applikation von Verbindungen der allgemeinen Formel (I) auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen der allgemeinen Formel (I) eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen der allgemeinen Formel (I) (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Dessikkation und Wuchsstauchung eingesetzt werden. Des Weiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die erfindungsgemäßen Verbindungen der allgemeinen Formel (I) auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen der allgemeinen Formel (I) und/oder deren Salze in wirtschaftlich bedeutenden transgenen Kulturen von Nutz und Zierpflanzen, z.B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen der allgemeinen Formel (I) auch als Herbizide in Nutzpflanzenkulturen eingesetzt werden, welche gegenüber den phytotoxischen Wirkungen der Herbizide resistent sind bzw. gentechnisch resistent gemacht worden sind.

Aufgrund ihrer herbiziden und pflanzenwachstumsregulatorischen Eigenschaften können die Verbindungen der allgemeinen Formel (I) auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z.B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt. Weitere besondere Eigenschaften können in einer Toleranz oder Resistenz gegen abiotische Stressoren z.B. Hitze, Kälte, Trockenheit, Salz und ultraviolette Strahlung liegen.

Bevorzugt ist die Anwendung der erfindungsgemäßen Verbindungen der allgemeinen Formel (I) oder deren Salze in wirtschaftlich bedeutenden transgenen Kulturen von Nutz-und Zierpflanzen, z.B. von Getreide wie Weizen, Gerste, Roggen, Hafer, Triticale, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der allgemeinen Formel (I) als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden.

Zahlreiche molekularbiologische Techniken, mit denen neue transgene Pflanzen mit veränderten Eigenschaften hergestellt werden können, sind dem Fachmann bekannt. Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z.B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z.B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen der allgemeinen Formel (I) in transgenen Kulturen eingesetzt werden, welche gegen Wuchsstoffe, wie z.B. Dicamba oder gegen Herbizide, die essentielle Pflanzenenzyme, z.B. Acetolactatsynthasen (ALS), EPSP Synthasen, Glutaminsynthasen (GS) oder Hydoxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Verbindungen der allgemeinen Formel (I) in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der allgemeinen Formel (I) und/oder deren Salze als Herbizide zur Bekämpfung von Schadpflanzen in Kulturen von Nutz- oder Zierpflanzen, gegebenenfalls in transgenen Kulturpflanzen.

Bevorzugt ist die Verwendung in Getreide, dabei vorzugsweise Mais, Weizen, Gerste, Roggen, Hafer, Hirse, oder Reis, im Vor- oder Nachauflauf.

Bevorzugt ist auch die Verwendung in Soja im Vor- oder Nachauflauf.

Die erfindungsgemäße Verwendung von Verbindungen der allgemeinen Formel (I) zur Bekämpfung von Schadpflanzen oder zur Wachstumsregulierung von Pflanzen schließt auch den Fall ein, bei dem die Verbindung der allgemeinen Formel (I) oder dessen Salz erst nach der Ausbringung auf der Pflanze, in der Pflanze oder im Boden aus einer Vorläufersubstanz ("Prodrug") gebildet wird.

Gegenstand der Erfindung ist auch die Verwendung einer oder mehrerer Verbindungen der allgemeinen Formel (I) oder deren Salzen bzw. eines erfindungsgemäßen Mittels (wie nachstehend definiert) (in einem Verfahren) zur Bekämpfung von Schadpflanzen oder zur Wachstumsregulierung von Pflanzen, dadurch gekennzeichnet, dass man eine wirksame Menge einer oder mehreren Verbindungen der allgemeinen Formel (I) oder deren Salzen auf die Pflanzen (Schadpflanzen, ggf. zusammen mit den Nutzpflanzen) Pflanzensamen, den Boden, in dem oder auf dem die Pflanzen wachsen, oder die Anbaufläche appliziert.

Gegenstand der Erfindung ist auch ein herbizides und/oder pflanzenwachstumsregulierendes Mittel, dadurch gekennzeichnet, dass das Mittel
(a) eine oder mehrere Verbindungen der allgemeinen Formel (I) und/oder deren Salze enthält wie oben definiert, vorzugsweise in einer der als bevorzugt bzw. besonders bevorzugt gekennzeichneten Ausgestaltung, insbesondere eine oder mehrere Verbindungen der Formeln (1.1-1) bis (1.15-485) und/oder deren Salze, jeweils wie oben definiert,
   und
(b) ein oder mehrere weitere Stoffe ausgewählt aus den Gruppen (i) und/oder (ii):
   (i) ein oder mehrere weitere agrochemisch wirksame Stoffe, vorzugsweise ausgewählt aus der Gruppe bestehend aus Insektiziden, Akariziden, Nematiziden, weiteren Herbiziden (d.h. solche, die nicht der oben definierten Formel (I) entsprechen), Fungiziden, Safenern, Düngemitteln und/oder weiteren Wachstumsregulatoren,
   (ii) ein oder mehrere im Pflanzenschutz übliche Formulierungshilfsmittel.

Die weiteren agrochemischen wirksamen Stoffe des Bestandteils (i) eines erfindungsgemäßen Mittels sind dabei vorzugsweise ausgewählt aus der Gruppe der Stoffe, die in "The Pesticide Manual", 16th edition, The British Crop Protection Council und the Royal Soc. of Chemistry, 2012 genannt sind.

Ein erfindungsgemäßes herbizides oder pflanzenwachstumsregulierendes Mittel, umfasst vorzugsweise ein, zwei, drei oder mehr im Pflanzenschutz übliche Formulierungshilfsmittel (ii) ausgewählt aus der Gruppe bestehend aus Tensiden, Emulgatoren, Dispergiermitteln, Filmbildnern, Verdickungsmitteln, anorganischen Salzen, Stäubemitteln, bei 25 °C und 1013 mbar festen Trägerstoffen, vorzugsweise adsorptionsfähigen, granulierten Inertmaterialien, Netzmitteln, Antioxidationsmitteln, Stabilisatoren, Puffersubstanzen, Antischaummitteln, Wasser, organischen Lösungsmitteln, vorzugsweise bei 25 °C und 1013 mbar mit Wasser in jedem beliebigen Verhältnis mischbare organische Lösungsmittel.

Die erfindungsgemäßen Verbindungen der allgemeinen Formel (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) und/oder deren Salze enthalten.

Die Verbindungen der allgemeinen Formel (I) und/oder deren Salze können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen und die Formulierungshilfsmittel wie Inertmaterialien, Tenside, Lösungsmittel und weitere Zusatzstoffe sind dem Fachmann bekannt, und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesellschaft, Stuttgart 1976; Winnacker-Küchler, "Chemische Technologie", Band 7, C. Hanser Verlag München, 4. Aufl. 1986.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wässrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulaten siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen, vorzugsweise herbizide oder pflanzenwachstumsregulierende Mittel der vorliegenden Erfindung enthalten vorzugsweise eine Gesamtmenge von 0,1 bis 99 Gew.-%, bevorzugt 0,5 bis 95 Gew.-%, weiter bevorzugt 1 bis 90 Gew.-%, insbesondere bevorzugt 2 bis 80 Gew.-%, an Wirkstoffen der Formel (I) und deren Salzen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstoffformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel. Beispiele für Formulierungshilfsmittel sind unter anderem in "Chemistry and Technology of Agrochemical Formulations", ed. D. A. Knowles, Kluwer Academic Publishers (1998) beschrieben.

Die Verbindungen der Formel (I) oder deren Salze können als solche oder in Form ihrer Zubereitungen (Formulierungen) mit anderen pestizid wirksamen Stoffen, wie z.B. Insektiziden, Akariziden, Nematiziden, Herbiziden, Fungiziden, Safenern, Düngemitteln und/oder Wachstumsregulatoren kombiniert eingesetzt werden, z.B. als Fertigformulierung oder als Tankmischungen. Die Kombinationsformulierungen können dabei auf Basis der obengenannten Formulierungen hergestellt werden, wobei die physikalischen Eigenschaften und Stabilitäten der zu kombinierenden Wirkstoffe zu berücksichtigen sind.

Als Kombinationspartner für die erfindungsgemäßen Verbindungen der Formel (I) in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. in Weed Research 26 (1986) 441-445 oder "The Pesticide Manual", 16th edition, The British Crop Protection Council and the Royal Soc. of Chemistry, 2012 und der dort zitierten Literatur beschrieben sind.

Von besonderem Interesse ist die selektive Bekämpfung von Schadpflanzen in Kulturen von Nutz- und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z.B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugsweise Getreide.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der allgemeinen Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Äußere Bedingungen wie Temperatur, Feuchtigkeit etc. beeinflussen zu einem gewissen Teil die Aufwandmenge der Verbindungen der allgemeinen Formel (I) und/oder deren Salze. Die Aufwandmenge kann dabei innerhalb weiter Grenzen variieren. Für die Anwendung als Herbizid zur Bekämpfung von Schadpflanzen liegt die Gesamtmenge an Verbindungen der allgemeinen Formel (I) und deren Salze vorzugsweise im Bereich von 0,001 bis 10,0 kg/ha, bevorzugt im Bereich von 0,005 bis 5 kg/ha, weiter bevorzugt im Bereich von 0,01 bis 1,5 kg/ha, insbesondere bevorzugt im Bereich von 0,05 bis 1 kg/ha. Dies gilt sowohl für die Anwendung im Vorauflauf oder im Nachauflauf.

Bei der Anwendung von Verbindungen der allgemeinen Formel (I) und/oder deren Salzen als Pflanzenwachstumsregulator, beispielsweise als Halmverkürzer bei Kulturpflanzen, wie sie oben genannt worden sind, vorzugsweise bei Getreidepflanzen wie Weizen, Gerste, Roggen, Triticale, Hirse, Reis oder Mais, liegt die Gesamt-Aufwandmenge vorzugsweise im Bereich von 0,001 bis 2 kg/ha, vorzugsweise im Bereich von 0,005 bis 1 kg/ha, insbesondere im Bereich von 10 bis 500 g/ha, ganz besonders bevorzugt im Bereich von 20 bis 250 g/ha. Dies gilt sowohl für die Anwendung im Vorauflauf oder im Nachauflauf.

Die Applikation als Halmverkürzer kann in verschiedenen Stadien des Wachstums der Pflanzen erfolgen. Bevorzugt ist beispielsweise die Anwendung nach der Bestockung am Beginn des Längenwachstums.

Alternativ kommt bei der Anwendung als Pflanzenwachstumsregulator auch die Behandlung des Saatguts in Frage, welche die unterschiedlichen Saatgutbeiz- und Beschichtungstechniken einschließt. Die Aufwandmenge hängt dabei von den einzelnen Techniken ab und kann in Vorversuchen ermittelt werden.

Als Kombinationspartner für die erfindungsgemäßen Verbindungen der allgemeinen Formel (I) in erfindungsgemäßen Mitteln (z.B. Mischungsformulierungen oder im Tank-Mix) sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II oder Protoporphyrinogen-Oxidase beruhen, einsetzbar, wie sie z.B. aus Weed Research 26 (1986) 441-445 oder "The Pesticide Manual", 16th edition, The British Crop Protection Council und the Royal Soc. of Chemistry, 2012 und dort zitierter Literatur beschrieben sind. Nachfolgend werden beispielhaft bekannte Herbizide oder Pflanzenwachstumsregulatoren genannt, die mit den erfindungsgemäßen Verbindungen kombiniert werden können, wobei diese Wirkstoffe entweder mit ihrem "common name" in der englischsprachigen Variante gemäß International Organization for Standardization (ISO) oder mit dem chemischen Namen bzw. mit der Codenummer bezeichnet sind. Dabei sind stets sämtliche Anwendungsformen wie beispielsweise Säuren, Salze, Ester sowie auch alle isomeren Formen wie Stereoisomere und optische Isomere umfasst, auch wenn diese nicht explizit erwähnt sind.

Beispiele für solche herbiziden Mischungspartner:
Als bekannte Herbizide oder Pflanzenwachstumsregulatoren, die mit Verbindungen der allgemeinen Formel (I) kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt: Acetochlor, Acifluorfen, Acifluorfen-methyl, Acifluorfen-Natrium, Aclonifen, Alachlor, Allidochlor, Alloxydim, Alloxydim-Natrium, Ametryn, Amicarbazon, Amidochlor, Amidosulfuron, 4-Amino-3-chlor-6-(4-chlor-2-fluor-3-methylphenyl)-5-fluorpyridin-2-carbonsäure, Aminocyclopyrachlor, Aminocyclopyrachlor-Kalium, Aminocyclopyrachlor-methyl, Aminopyralid, Aminopyraliddimethylammonium, Aminopyralid-tripromine, Amitrol, Ammoniumsulfamate, Anilofos, Asulam, Asulam-Kalium, Asulam-Natrium, Atrazin, Azafenidin, Azimsulfuron, Beflubutamid, (S)-(-)-Beflubutamid, Beflubutamid-M, Benazolin, Benazolin-ethyl, Benazolin-dimethylammonium, Benazolin-Klaium, Benfluralin, Benfuresate, Bensulfuron, Bensulfuron-methyl, Bensulid, Bentazon, Bentazon-Natrium, Benzobicyclon, Benzofenap, Bicyclopyrone, Bifenox, Bilanafos, Bilanafos-Natium, Bipyrazone, Bispyribac, Bispyribac-Natium, Bixlozon, Bromacil, Bromacil-lithium, Bromacil-Natrium, Bromobutid, Bromofenoxim, Bromoxynil, Bromoxynilbutyrat, Bromoxynil-Kalium, Bromoxynilheptanoat und Bromoxynil-octanoat, Busoxinon, Butachlor, Butafenacil, Butamifos, Butenachlor, Butralin, Butroxydim, Butylat, Cafenstrol, Cambendichlor, Carbetamide, Carfentrazon, Carfentrazon-Ethyl, Chloramben, Chloramben-ammonium, Chloramben-diolamin, Chlroamben-methyl, Chlorambenmethylammonium, Chloramben-Natium, Chlorbromuron, Chlorfenac, Chlorfenac-ammonium, Chlorfenac-Natium, Chlorfenprop, Chlorfenprop-methyl, Chlorflurenol, Chlorflurenol-methyl, Chloridazon, Chlorimuron, Chlorimuron-ethyl, Chlorophthalim, Chlorotoluron, Chlorsulfuron, Chlorthal, Chlorthal-dimethyl, Chlorthal-monomethyl, Cinidon, Cinidon-ethyl, Cinmethylin, exo-(+)-Cinmethylin, d.h. (1R,2S,4S)-4-isopropyl-1-methyl-2-[(2-methylbenzyl)oxy]-7-oxabicyclo[2.2.1]heptan, exo-(-)-Cinmethylin, d.h. (1R,2S,4S)-4-isopropyl-1-methyl-2-[(2-methylbenzyl)oxy]-7-oxabicyclo[2.2.1]heptan, Cinosulfuron, Clacyfos, Clethodim, Clodinafop, Clodinafop-ethyl, Clodinafoppropargyl, Clomazon, Clomeprop, Clopyralid, Clopyralid-methyl, Clopyralid-olamin, Clopyralid-Kalium, Clopyralid-tripomin, Cloransulam, Cloransulam-methyl, Cumyluron, Cyanamide, Cyanazine, Cycloat, Cyclopyranil, Cyclopyrimorat, Cyclosulfamuron, Cycloxydim, Cyhalofop, Cyhalofop-butyl, Cyprazin, 2,4-D (sowie die Ammonium, Butotyl, Butyl, Cholin, Diethylammonium, Dimethylammonium, Diolamin, Doboxyl, Dodecylammonium, Etexyl, Ethyl, 2-Ethylhexyl, Heptylammonium, Isobutyl, Isooctyl, Isopropyl, Isopropylammonium, Lithium, Meptyl, Methyl, Kalium, Tetradecylammonium, Triethylammonium, Triisopropanolammonium, Tripromin and Trolamin Salze davon), 2,4-DB, 2,4-DB-butyl, 2,4-DB-Dimethylammonium, 2,4-DB-isooctyl, 2,4-DB-Kalium und 2,4-DB-Natrium, Daimuron (Dymron), Dalapon, Dalapon-Calcium, Dalapon-Magnesium, Dalapon-Natium, Dazomet, Dazomet-Natrium, n-Decanol, 7-Deoxy-D-sedoheptulose, Desmedipham, Detosylpyrazolat (DTP), Dicamba und seine Salze (z.B. Dicamba-biproamin, Dicamba-N,N-Bis(3-aminopropyl)methylamin, Dicamba-butotyl, Dicamba-cholin, Dicamba-Diglycolamin, Dicamba-Dimethylammonium, Dicamba-Diethanolaminemmonium, Dicamba-Diethylammonium, Dicambaisopropylammonium, Dicamba-methyl, Dicamba-monoethanolamin, Dicamba-olamin, Dicamba-Kalium, Dicamba-Natium, Dicamba-Triethanolamin), Dichlobenil, 2-(2,4-Dichlorbenzyl)-4,4-dimethyl-1,2-oxazolidin-3-on, 2-(2,5-Dichlorbenzyl)-4,4-dimethyl-1,2-oxazolidin-3-one, Dichlorprop, Dichlorprop-butotyl, Dichlorprop-Dimethylammonium, Dichhlorprop-etexyl, Dichlorpropethylammonium, Dichlorprop-isoctyl, Dichlorprop-methyl, Dichlorprop-Kalium, Dichlorprop-Natrium, Dichlorprop-P, Dichlorprop-P-Dimethylammonium, Dichlorprop-P-etexyl, Dichlorprop-P-Kalium, Dichlorprop-Natrium, Diclofop, Diclofop-methyl, Diclofop-P, Diclofop-P-methyl, Diclosulam, Difenzoquat, Difenzoquat-metilsulfate, Diflufenican, Diflufenzopyr, Diflufenzopyr-Natrium, Dimefuron, Dimepiperate, Dimesulfazet, Dimethachlor, Dimethametryn, Dimethenamid, Dimethenamid-P, Dimetrasulfuron, Dinitramine, Dinoterb, Dinoterb-Acetate, Diphenamid, Diquat, Diquat-Dibromid, Diquat-Dichloride, Dithiopyr, Diuron, DNOC, DNOC-Ammonium, DNOC-Kalium, DNOC-Natrium, Endothal, Endothal-Diammonium, Endothal-Dikalium, Endothal-Dinatrium, Epyrifenacil (S-3100), EPTC, Esprocarb, Ethalfluralin, Ethametsulfuron, Ethametsulfuron-Methyl, Ethiozin, Ethofumesate, Ethoxyfen, Ethoxyfen-Ethyl, Ethoxysulfuron, Etobenzanid, F-5231, d.h. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dion, Fenoxaprop, Fenoxaprop-P, Fenoxaprop-Ethyl, Fenoxaprop-P-Ethyl, Fenoxasulfone, Fenpyrazone, Fenquinotrione, Fentrazamid, Flamprop, Flamprop-Isoproyl, Flamprop-Methyl, Flamprop-M-Isopropyl, Flamprop-M-Methyl, Flazasulfuron, Florasulam, Florpyrauxifen, Florpyrauxifen-benzyl, Fluazifop, Fluazifop-Butyl, Fluazifop-Methyl, Fluazifop-P, Fluazifop-P-Butyl, Flucarbazone, Flucarbazone-Natrium, Flucetosulfuron, Fluchloralin, Flufenacet, Flufenpyr, Flufenpyr-Ethyl, Flumetsulam, Flumiclorac, Flumiclorac-Pentyl, Flumioxazin, Fluometuron, Flurenol, Flurenol-Butyl, -Dimethylammonium und -Methyl, Fluoroglycofen, Fluoroglycofen-Ethyl, Flupropanat, Flupropanat-Natrium, Flupyrsulfuron, Flupyrsulfuron-Methyl, Flupyrsulfuron-Methyl-Natrium, Fluridon, Flurochloridon, Fluroxypyr, Fluroxypyr-Butometyl, Fluroxypyr-Meptyl, Flurtamon, Fluthiacet, Fluthiacet-Methyl, Fomesafen, Fomesafen-Natrium, Foramsulfuron, Foramsulfuron-Natrium, Fosamine, Fosamine-Ammonium, Glufosinat, Glufosinat-Ammonium, Glufosinat-Natrium, L-Glufosinat-Ammonium, L-Glufosinat-Natrium, Glufosinat-P-Natrium, Glufosinat-P-Ammonium, Glyphosat, Glyphosat-Ammonium, Glyphosat-Isopropylammonium, Glyphosat-Diammonium, Glyphosat-Dimethylammonium, Glyphosat-Kalium, Glyphosat-Natrium, Glyphosat-Sesquinatrium und Glyphosat-Trimesium, H-9201, d.h. O-(2,4-Dimethyl-6-nitrophenyl)-O-ethyl-isopropylphosphoramidothioat, Halauxifen, Halauxifen-methyl, Halosafen, Halosulfuron, Halosulfuron-Methyl, Haloxyfop, Haloxyfop-P, Haloxyfop-Ethoxyethyl, Haloxyfop-P-Ethoxyethyl, Haloxyfop-Methyl, Haloxyfop-P-Methyl, Haloxifop-Natrium, Hexazinon, HNPC-A8169, i.e. Prop-2-yn-1-yl (2S)-2-{3-[(5-tert-butylpyridin-2-yl)oxy]phenoxy}propanoat, HW-02, d.h. 1-(Dimethoxyphosphoryl)-ethyl-(2,4-dichlorphenoxy)acetat, Hydantocidin, Imazamethabenz, Imazamethabenz-Methyl, Imazamox, Imazamox-Ammonium, Imazapic, Imazapic-Ammonium, Imazapyr, Imazapyr-Isopropylammonium, Imazaquin, Imazaquin-Ammonium, Imazaquin-Methyl, Imazethapyr, Imazethapyr-Ammonium, Imazosulfuron, Indanofan, Indaziflam, Iodosulfuron, Iodosulfuron-Methyl, Iodosulfuron-Methyl-Natrium, Ioxynil, Ioxynil-Lithium, -Octanoat, -Kalium und Natrium, Ipfencarbazon, Isoproturon, Isouron, Isoxaben, Isoxaflutole, Karbutilat, KUH-043, d.h. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, Ketospiradox, Ketospiradox-Kalium, Lactofen, Lenacil, Linuron, MCPA, MCPA-Butotyl, -Butyl, -Dimethylammonium, -Diolamin, -2-Ethylhexyl, -Ethyl, -Isobutyl, Isoctyl, -Isopropyl, -Isopropylammonium, - Methyl, Olamin, -Kalium, -Natrium und -Trolamin, MCPB, MCPB-Methyl, -Ethyl und -Natrium, Mecoprop, Mecoprop-Butotyl, Mecoprop- dimethylammonium, Mecoprop-Diolamin, Mecoprop-Etexyl, Mecoprop-Ethadyl, Mecoprop-Isoctyl, Mecoprop-Methyl, Mecoprop-Kalium, Mecoprop-Natrium, und Mecoprop-Trolamin, Mecoprop-P, Mecoprop-P-Butotyl, -Dimethylammonium, -2-Ethylhexyl und - Kalium, Mefenacet, Mefluidid, Mefluidid-Diolamin, Mefluidid-Kalium, Mesosulfuron, Mesosulfuron-Methyl, Mesosulfuron-Natrium, Mesotrion, Methabenzthiazuron, Metam, Metamifop, Metamitron, Metazachlor, Metazosulfuron, Methabenzthiazuron, Methiopyrsulfuron, Methiozolin, Methyl isothiocyanat, Metobromuron, Metolachlor, S-Metolachlor, Metosulam, Metoxuron, Metribuzin, Metsulfuron, Metsulfuron-Methyl, Molinat, Monolinuron, Monosulfuron, Monosulfuron-Methyl, MT-5950, d.h. N-[3-Chlor-4-(1-methylethyl)-phenyl]-2-methylpentanamid, NGGC-011, Napropamid, NC-310, i.e. 4-(2,4-Dichlorbenzoyl)-1-methyl-5-benzyloxypyrazol, NC-656, i.e. 3-[(Isopropylsulfonyl)methyl]-N-(5-methyl-1,3,4-oxadiazol-2-yl)-5-(trifluormethyl)[1,2,4]triazolo-[4,3-a]pyridin-8-carboxamid, Neburon, Nicosulfuron, Nonansäure (Pelargonsäure), Norflurazon, Ölsäure (Fettsäuren), Orbencarb, Orthosulfamuron, Oryzalin, Oxadiargyl, Oxadiazon, Oxasulfuron, Oxaziclomefone, Oxyfluorfen, Paraquat, Paraquat-dichlorid, Paraquat-Dimethylsulfat, Pebulat, Pendimethalin, Penoxsulam, Pentachlorphenol, Pentoxazon, Pethoxamid, Petroleumöl, Phenmedipham, Phenmedipham-Ethyl, Picloram, Picloram-dimethylammonium, Picloram-Etexyl, Picloram-Isoctyl, Picloram-Methyl, Picloram-Olamin, Picloram-Kalium, Picloram-Triethylammonium, Picloram-Tripromin, Picloram-Trolamin, Picolinafen, Pinoxaden, Piperophos, Pretilachlor, Primisulfuron, Primisulfuron-Methyl, Prodiamine, Profoxydim, Prometon, Prometryn, Propachlor, Propanil, Propaquizafop, Propazine, Propham, Propisochlor, Propoxycarbazone, Propoxycarbazone-Natrium, Propyrisulfuron, Propyzamid, Prosulfocarb, Prosulfuron, Pyraclonil, Pyraflufen, Pyraflufen-Ethyl, Pyrasulfotol, Pyrazolynat (Pyrazolat), Pyrazosulfuron, Pyrazosulfuron-Ethyl, Pyrazoxyfen, Pyribambenz, Pyribambenz-Isopropyl, Pyribambenz-Propyl, Pyribenzoxim, Pyributicarb, Pyridafol, Pyridat, Pyriftalid, Pyriminobac, Pyriminobac-Methyl, Pyrimisulfan, Pyrithiobac, Pyrithiobac-Natrium, Pyroxasulfon, Pyroxsulam, Quinclorac, Quinclorac-Dimethylammonium, Quinclorac-Methyl, Quinmerac, Quinoclamin, Quizalofop, Quizalofop-Ethyl, Quizalofop-P, Quizalofop-P-Ethyl, Quizalofop-P-Tefuryl, QYM201, i.e. 1-{2-Chlor-3-[(3-cyclopropyl-5-hydroxy-1-methyl-1H-pyrazol-4-yl)carbonyl]-6-(trifluormethyl)phe-nyl}piperidin-2-on, Rimsulfuron, Saflufenacil, Sethoxydim, Siduron, Simazine, Simetryn, SL-261, Sulcotrione, Sulfentrazone, Sulfometuron, Sulfometuron-Methyl, Sulfosulfuron, , SYP-249, d.h. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (Trichloressigsäure) und seine Salze, z.B. TCA-ammonium, TCA-Calcium, TCA-Ethyl, TCA-Magnesium, TCA-Natrium, Tebuthiuron, Tefuryltrione, Tembotrion, Tepraloxydim, Terbacil, Terbucarb, Terbumeton, Terbuthylazine, Terbutryn, Tetflupyrolimet, Thaxtomin, Thenylchlor, Thiazopyr, Thiencarbazone, Thiencarbazon-Methyl, Thifensulfuron, Thifensulfuron-Methyl, Thiobencarb, Tiafenacil, Tolpyralat, Topramezon, Tralkoxydim, Triafamon, Tri-allat, Triasulfuron, Triaziflam, Tribenuron, Tribenuron-Methyl, Triclopyr, Triclopyr-Butotyl, Triclopyr-Cholin, Triclopyr-Ethyl, Triclopyr-Triethylammonium, Trietazine, Trifloxysulfuron, Trifloxysulfuron-Natrium, Trifludimoxazin, Trifluralin, Triflusulfuron, Triflusulfuron-Methyl, Tritosulfuron, Harnstoffsulfat, Vernolat, XDE-848, ZJ-0862, d.h. 3,4-Dichlor-N-{2-[(4,6-dimethoxypyrimidin-2-yl)oxy]benzyl}anilin, 3-(2-Chlor-4-fluor-5-(3-methyl-2,6-dioxo-4-trifluormethyl-3,6-dihydropyrimidin-1(2H)-yl)phenyl)-5-methyl-4,5-dihydroisoxazole-5-carbonsäureethylester, Ethyl-[(3-{2-chlor-4-fluor-5-[3-methyl-2,6-dioxo-4-(trifluormethyl)-3,6-dihydropyrimidin-1(2H)-yl]phenoxy}pyridin-2-yl)oxy]acetat, 3-Chlor-2-[3-(difluormethyl)isoxazolyl-5-yl]phenyl-5-chlorpyrimidin-2-ylether, 2-(3,4-Dimethoxyphenyl)-4-[(2-hydroxy-6-oxocyclohex-1-en-1-yl)carbonyl]-6-methylpyridazine-3(*2H*)-on, 2-({2-[(2-Methoxyethoxy)methyl]-6-methylpyridin-3-yl}carbonyl)cyclohexane-1,3-dion, (5-Hydroxy-1-methyl-1H-pyrazol-4-yl)(3,3,4-trimethyl-1,1-dioxido-2,3-dihydro-1-benzothiophen-5-yl)methanon, 1-Methyl-4-[(3,3,4-trimethyl-1,1-dioxido-2,3-dihydro-1-benzothiophen-5-yl)carbonyl]-1H-pyrazol-5-yl propan-1-sulfonat, 4-{2-Chlor-3-[(3,5-dimethyl-1H-pyrazol-1-yl)methyl]-4-(methylsulfonyl)benzoyl}-1-methyl-1H-pyrazol-5-yl-1,3-dimethyl-1H-pyrazol-4-carboxylat; Cyanomethyl-4-amino-3-chlor-5-fluor-6-(7-fluor-1H-indol-6-yl)pyridin-2-carboxylat, Prop-2-yn-1-yl 4-amino-3-chlor-5-fluor-6-(7-fluor-1H-indol-6-yl)pyridin-2-carboxylat, Methyl-4-amino-3-chlor-5-fluor-6-(7-fluor-1H-indol-6-yl)pyridin-2-carboxylat, 4-Amino-3-chlor-5-fluor-6-(7-fluor-1H-indol-6-yl)pyridin-2-carbonsäure, Benzyl-4-amino-3-chlor-5-fluor-6-(7-fluor-1H-indol-6-yl)pyridin-2-carboxylat, Ethyl-4-amino-3-chlor-5-fluor-6-(7-fluor-1H-indol-6-yl)pyridin-2-carboxylat, Methyl-4-amino-3-chlor-5-fluor-6-(7-fluor-1-isobutyryl-1H-indol-6-yl)pyridin-2-carboxylat, Methyl 6-(1-acetyl-7-fluor-1H-indol-6-yl)-4-amino-3-chlor-5-fluorpyridin-2-carboxylat, Methyl-4-amino-3-chlor-6-[1-(2,2-dimethylpropanoyl)-7-fluor-1H-indol-6-yl]-5-fluorpyridin-2-carboxylat, Methyl-4-amino-3-chlor-5-fluor-6-[7-fluor-1-(methoxyacetyl)-1H-indol-6-yl]pyridin-2-carboxylat, Kalium 4-amino-3-chlor-5-fluor-6-(7-fluor-1H-indol-6-yl)pyridin-2-carboxylat, Natrium-4-amino-3-chlor-5-fluor-6-(7-fluor-1H-indol-6-yl)pyridin-2-carboxylat, Butyl-4-amino-3-chlor-5-fluoro-6-(7-fluoro-1H-indol-6-yl)pyridin-2-carboxylat, 4-Hydroxy-1-methyl-3-[4-(trifluoromethyl)pyridin-2-yl]imidazolidin-2-on, 3-(5-tert-butyl-1,2-oxazol-3-yl)-4-hydroxy-1-methylimidazolidin-2-on, 3-[5-Chlor-4-(trifluormethyl)pyridin-2-yl]-4-hydroxy-1-methylimidazolidin-2-on, 4-Hydroxy-1-methoxy-5-methyl-3-[4-(trifluormethyl)pyridin-2-yl]imidazolidin-2-on, 6-[(2-Hydroxy-6-oxocyclohex-1-en-1-yl)carbonyl]-1,5-dimethyl-3-(2-methylphenyl)chinazolin-2,4(1H,3H)-dion, 3-(2,6-Dimethylphenyl)-6-[(2-hydroxy-6-oxocyclohex-1-en-1-yl)carbonyl]-1-methylchinazolin-2,4(1H,3H)-dion, 2-[2-chlor-4-(methylsulfonyl)-3-(morpholin-4-ylmethyl)benzoyl]-3-hydroxycyclohex-2-en-1-on, 1-(2-carboxyethyl)-4-(pyrimidin-2-yl)pyridazin-1-iumsalz (mit passenden Anionen wie z.B Chlorid, Acetat oder Trifluoracetat), 1-(2-Carboxyethyl)-4-(pyridazin-3-yl)pyridazin-1-iumsalz (mit passenden Anionen wie z.B. Chlorid, Acetat oder Trifluoracetat), 4-(Pyrimidin-2-yl)-1-(2-sulfoethyl)pyridazin-1-ium salz iumsalz (mit passenden Anionen wie z.B Chlorid, Acetat oder Trifluoracetat), 4-(Pyridazin-3-yl)-1-(2-sulfoethyl)pyridazin-1-iumsalz (mit passenden Anionen wie z.B Chlorid, Acetat oder Trifluoracetat), 1-(2-Carboxyethyl)-4-(1,3-thiazol-2-yl)pyridazin-1-iumsalz (mit passenden Anionen wie z.B Chlorid, Acetat oder Trifluoracetat), 1-(2-Carboxyethyl)-4-(1,3,4-thiadiazol-2-yl)pyridazin-1-ium salz (mit passenden Anionen wie z.B Chlorid, Acetat oder Trifluoracetat).

Beispiele für Wuchsregulatoren und Pflanzenstimulantien als Mischungspartner sind:
Abscisinsäure und verwandte Analoga [z.B. (2Z,4E)-5-[6-Ethynyl-1-hydroxy-2,6-dimethyl-4-oxocyclohex-2-en-1-yl]-3-methylpenta-2,4-diensäure, methyl-(2Z,4E)-5-[6-ethynyl-1-hydroxy-2,6-dimethyl-4-oxocyclohex-2-en-1-yl]-3-methylpenta-2,4-dienoat, (2Z,4E)-3-ethyl-5-(1-hydroxy-2,6,6-trimethyl-4-oxocyclohex-2-en-1-yl)penta-2,4-diensäure, (2E,4E)-5-(1-hydroxy-2,6,6-trimethyl-4-oxocyclohex-2-en-1-yl)-3-(trifluoromethyl)penta-2,4-diensäure, methyl (2E,4E)-5-(1-hydroxy-2,6,6-trimethyl-4-oxocyclohex-2-en-1-yl)-3-(trifluoromethyl)penta-2,4-dienoat, (2Z,4E)-5-(2-hydroxy-1,3-dimethyl-5-oxobicyclo[4.1.0]hept-3-en-2-yl)-3-methylpenta-2,4-diensäure], Acibenzolar, Acibenzolar-S-methyl, S-Adenosylhomocystein, Allantoin, 2-Aminoethoxyvinylglycin (AVG), Aminooxyessigsäure and verwandte Ester [z.B. (Isopropyliden)-aminooxyessigsäure-2-(methoxy)-2-oxoethylester, (Isopropyliden)-aminooxyessigsäure-2-(hexyloxy)-2-oxoethylester, (Cyclohexyliden)-aminooxyessigsäure-2-(isopropyloxy)-2-oxoethylester], 1-Aminocycloprop-1-ylcarbonsäure N-Methyl-1-aminocyclopropyl-1-carbonsäure, 1-Aminocyclopropyl-1-carbonsäureamid, substituierte 1-Aminocyclopropyl-1-carbonsäurederivate wie sie in DE3335514, EP30287, DE2906507 oder US5123951 beschrieben werden, 1-Aminocyclopropyl-1-hydroxamsäure, 5-Aminolevulinsäure, Ancymidol, 6-Benzylaminopurin, Bikinin, Brassinolid, Brassinolide-ethyl, L-Canalin, Catechin und catechine (z.B. (2S,3R)-2-(3,4-Dihydroxyphenyl)-3,4-dihydro-2H-chromen-3,5,7-triol), Chitooligosaccharides (CO; COs unterscheiden sich von LCOs dadurch, daß ihnen die für LCOs charakteristische Fettsäureseitenkette fehlt. COs, in manchen Fällen als N-Acetylchitooligosaccharide bezeichnet, sind auch aus GlcNAc-Einheiten aufgebaut, aber haben Seitenketten, durch die sies ich von Chitinmolekülen unterscheiden [(C₈H₁₃NO₅)ₙ, CAS No. 1398-61-4] und chitosan Moleküle [(C₅H₁₁NO₄)ₙ, CAS No. 9012-76-4]), Chitin-artige Verbindungen, Chlormequat chloride, Cloprop, Cyclanilide, 3-(Cycloprop-1-enyl)propionsäure, 1-[2-(4-Cyano-3,5-dicyclopropylphenyl)acetamido]cyclohexancarbonsäure, 1-[2-(4-Cyano-3-cyclopropylphenyl)acetamido]cyclohexancarbonsäure, 1-Cyclopropenylmethanol, Daminozid, Dazomet, Dazomet-Natrium, n-Decanol, Dikegulac, Dikegulac-Natrium, Endothal, Endothal-di-Kalium, -diNatrium, und mono(N,N-dimethylalkylammonium), Ethephon, 1-Ethylcyclopropen,Flumetralin, Flurenol, Flurenol-butyl, Flurenol-methyl, Flurprimidol, Forchlorfenuron, Gibberellinsäure, Inabenfid, Indol-3-essigsäure (IAA), 4-Indol-3-ylbuttersäure, Isoprothiolan, Probenazole, Jasmonsäure, Jasmonsäureester oder andere Derivate (z.B. Jasmonsäuremethylester, Jasmonsäureethylester), Lipochitooligosaccharide (LCO, in manchen Fällen auch als Symbiotische Nodulationssignale (Nod oder Nod Faktoren) oder als Myc Faktoren bezeichnet, bestehen aus einem Oligosacchariderückgrat aus β-1,4-verknüpften *N*-Acetyl-D-Glucosaminresten ("GlcNAc") mit einer N-verknüpften Fettsäureseitenkette, die am nicht reduzierenden Ende ankondensiert ist. Wie aus der Literatur zu entnehmen ist, unterscheiden sich LCOs in der Zahl an GlcNAc-EInheiten in der Rückgratstruktur, in der Länge und dem Sättigungsgrad der Fettsäurekette sowie in der Substitution der reduzierenden und nicht-reduzierenden Zuckereinheiten), Linoleinsäure oder ihre Derivate, Linolensäure oder ihre Derivate, Maleinsäurehydrazid, Mepiquatchlorid, Mepiquatpentaborat, 1-Methylcyclopropen, 3-Methylcyclopropen, Methoxyvinylglycin (MVG), 3'-Methylabscisinsäure, 1-(4-Methylphenyl)-N-(2-oxo-1-propyl-1,2,3,4-tetrahydrochinolin-6-yl)methansulfonamid und verwandte substituierte (Tetrahydrochinolin-6-yl)methansulfonamide, (3E,3αR,8βS)-3-({[(2R)-4-Methyl-5-oxo-2,5-dihydrofuran-2-yl]oxy}methylen)-3,3a,4,8b-tetrahydro-2H-indeno[1,2-b]furan-2-on und verwandte Laktone wie sie in EP2248421 beschrieben sind, 2-(1-Naphthyl)acetamid, 1-Naphthylessigsäure, 2-Naphthyloxyessigsäure, Nitrophenolatmischung, 4-Oxo-4[(2-phenylethyl)amino]buttersäure, Paclobutrazol, 4-Phenylbuttersäure and ihre Salze (z.B. Natrium-4-phenylbutanoat, Kalium-4-phenylbutanoat), Phenylalanine, N-Phenylphthalamsäure, Prohexadione, Prohexadion-Calcium, , 1-n-Propylcyclopropen, Putrescin, Prohydrojasmon, Rhizobitoxin, Salicylsäure und Salicyclsäuremethylester, Sarcosin, Natriumcycloprop-1-en-1-ylacetat, Natriumcycloprop-2-en-1-ylacetat, Natrium-3-(cycloprop-2-en-1-yl)propanoat, Natrium-3-(cycloprop-1-en-1-yl)propanoat, Sidefungin, Spermidin, Spermine, Strigolactone, Tecnazene, Thidiazuron, Triacontanol, Trinexapac, Trinexapac-ethyl, Tryptophan, Tsitodef, Uniconazol, Uniconazol-P, 2-Fluoro-N-(3-methoxyphenyl)-9*H-*purin-6-amin.

Ebenfalls als Kombinationspartner für die erfindungsgemäßen Verbindungen der Formel (I) kommen beispielsweise die folgenden Safener in Frage:
- S1): Verbindungen aus der Gruppe heterocyclischer Carbonsäurederivate: 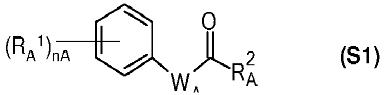
wobei die Symbole und Indizes folgende Bedeutungen haben:
- n_{A}: ist eine natürliche Zahl von 0 bis 5, vorzugsweise 0 bis 3;
- R_{A}¹: ist Halogen, (C₁-C₄)-Alkyl, (C₁-C₄)-Alkoxy, Nitro oder (C₁-C₄)-Haloalkyl;

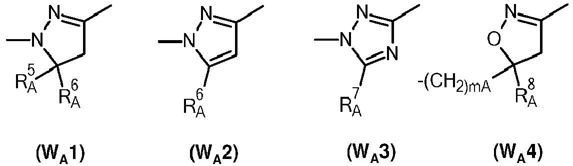

W_{A} ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N-Atom und höchstens ein O-Atom im Ring enthalten ist, vorzugsweise ein Rest aus der Gruppe (W_{A}¹) bis (W_{A}⁵),
m_{A} ist 0 oder 1;
R_{A}² ist OR_{A}³, SR_{A}³ oder NR_{A}³R_{A}⁴ oder ein gesättigter oder ungesättigter 3- bis 7-gliedriger Heterocyclus mit mindestens einem N-Atom und bis zu 3 Heteroatomen, vorzugsweise aus der Gruppe O und S, der über das N-Atom mit der Carbonylgruppe in (S1) verbunden ist und unsubstituiert oder durch Reste aus der Gruppe (C₁-C₄)-Alkyl, (C₁-C₄)-Alkoxy oder gegebenenfalls substituiertes Phenyl substituiert ist, vorzugsweise ein Rest der Formel OR_{A}³, NHR_{A}⁴ oder N(CH₃)₂, insbesondere der Formel OR_{A}³;
R_{A}³ ist Wasserstoff oder ein unsubstituierter oder substituierter aliphatischer Kohlenwasserstoffrest, vorzugsweise mit insgesamt 1 bis 18 C-Atomen;
R_{A}⁴ ist Wasserstoff, (C₁-C₆)-Alkyl, (C₁-C₆)-Alkoxy oder substituiertes oder unsubstituiertes Phenyl; R_{A}⁵ ist H, (C₁-C₈)-Alkyl, (C₁-C₈)-Haloalkyl, (C₁-C₄)-Alkoxy-(C₁-C₈)-Alkyl, Cyano oder COOR_{A}⁹, worin R_{A}⁹ Wasserstoff, (C₁-C₈)-Alkyl, (C₁-C₈)-Haloalkyl, (C₁-C₄)-Alkoxy-(C₁-C₄)-alkyl, (C₁-C₆)-Hydroxyalkyl, (C₃-C₁₂)-Cycloalkyl oder Tri-(C₁-C₄)-alkyl-silyl ist;
R_{A}⁶, R_{A}⁷, R_{A}⁸ sind gleich oder verschieden Wasserstoff, (C₁-C₈)-Alkyl, (C₁-C₈)-Haloalkyl, (C₃-C₁₂)-Cycloalkyl oder substituiertes oder unsubstituiertes Phenyl; vorzugsweise:

- S1^{a}): Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäureethylester (S1-1) ("Mefenpyr-diethyl"), und verwandte Verbindungen, wie sie in der WO-A-91/07874 beschrieben sind;
- S1^{b}): Derivate der Dichlorphenylpyrazolcarbonsäure (S1^{b}), vorzugsweise Verbindungen wie 1-(2,4-Dichlorphenyl)-5-methylpyrazol-3-carbonsäureethylester (S1-2), 1-(2,4-Dichlorphenyl)-5-isopropylpyrazol-3-carbonsäureethylester (S1-3), 1-(2,4-Dichlorphenyl)-5-(1,1-dimethyl-ethyl)pyrazol-3-carbonsäureethylester (S1-4) und verwandte Verbindungen, wie sie in EP-A-333131 und EP-A-269806 beschrieben sind;
- S1^{c}): Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethylester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
- S1^{d}): Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen, wie sie in EP-A-174562 und EP-A-346620 beschrieben sind;
- S1^{e}): Verbindungen vom Typ der 5-Benzyl- oder 5-Phenyl-2-isoxazolin-3- carbonsäure, oder der 5,5-Diphenyl-2-isoxazolin-3-carbonsäure(S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-3-carbonsäureethylester (S1-11) ("Isoxadifen-ethyl") oder -n-propylester (S1-12) oder 5-(4-Fluorphenyl)-5-phenyl-2-isoxazolin-3-carbon-säureethylester (S1-13), wie sie in der Patentanmeldung WO-A-95/07897 beschrieben sind.
- S2): Verbindungen aus der Gruppe der 8-Chinolinoxyderivate (S2):
- S2^{a}): Verbindungen vom Typ der 8-Chinolinoxyessigsäure (S2^{a}), vorzugsweise (5-Chlor-8-chinolinoxy)essigsäure-(1-methylhexyl)-ester ("Cloquintocet-mexyl") (S2-1), (5-Chlor-8-chinolinoxy)essigsäure-(1,3-dimethyl-but-1-yl)-ester (S2-2), (5-Chlor-8-chinolinoxy)essigsäure-4-allyl-oxy-butylester (S2-3), (5-Chlor-8-chinolinoxy)essigsäure-1-allyloxy-prop-2-ylester (S2-4), (5-Chlor-8-chinolinoxy)essigsäureethylester (S2-5), (5-Chlor-8-chinolinoxy)essigsäuremethylester (S2-6), (5-Chlor-8-chinolinoxy)essigsäureallylester (S2-7), (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und verwandte Verbindungen, wie sie in EP-A-86750, EP-A-94349 und EP-A-191736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
- S2^{b}): Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
- S3): Wirkstoffe vom Typ der Dichloracetamide (S3), die häufig als Vorauflaufsafener (bodenwirksame Safener) angewendet werden, wie z. B. "Dichlormid" (N,N-Diallyl-2,2-dichloracetamid) (S3-1), "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5), "DKA-24" (N-Allyl-N-[(allylaminocarbonyl)methyl]-dichloracetamid) der Firma Sagro-Chem (S3-6), "AD-67" oder "MON 4660" (3-Dichloracetyl-1-oxa-3-aza-spiro[4,5]decan) der Firma Nitrokemia bzw. Monsanto (S3-7), "TI-35" (1-Dichloracetyl-azepan) der Firma TRI-Chemical RT (S3-8), "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10), sowie dessen (R)-Isomer (S3-11).
- S4): Verbindungen aus der Klasse der Acylsulfonamide (S4):
- S4^{a}): N-Acylsulfonamide der Formel (S4^{a}) und deren Salze wie sie in der WO-A-97/45016 beschrieben sind, 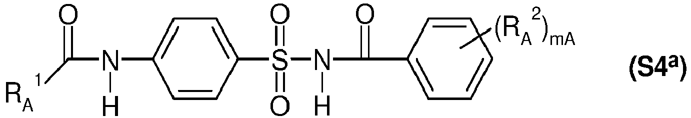 worin
R_{A}¹ (C₁-C₆)-Alkyl, (C₃-C₆)-Cycloalkyl, wobei die 2 letztgenannten Reste durch v_{A} Substituenten aus der Gruppe Halogen, (C₁-C₄)-Alkoxy, (C₁-C₆)-Haloalkoxy und (C₁-C₄)-Alkylthio und im Falle cyclischer Reste auch durch (C₁-C₄)-Alkyl und (C₁-C₄)-Haloalkyl substituiert sind;
R_{A}² Halogen, (C₁-C₄)-Alkyl, (C₁-C₄)-Alkoxy, CF_{3;}
m_{A} 1 oder 2;
vₐ ist 0, 1, 2 oder 3 bedeuten;
S4^{b}) Verbindungen vom Typ der 4-(Benzoylsulfamoyl)benzamide der Formel (S4^{b}) und deren Salze, wie sie in der WO-A-99/16744 beschrieben sind, 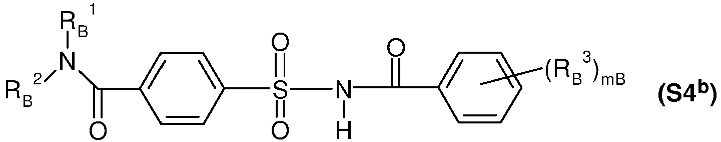 worin
R_{B}¹, R_{B}² unabhängig voneinander Wasserstoff, (C₁-C₆)-Alkyl, (C₃-C₆)-Cycloalkyl, (C₃-C₆)-Alkenyl, (C₃-C₆)-Alkinyl,
R_{B}³ Halogen, (C₁-C₄)-Alkyl, (C₁-C₄)-Haloalkyl oder (C₁-C₄)-Alkoxy und
m_{B} 1 oder 2 bedeuten, z.B. solche worin

R_{B}¹ = Cyclopropyl, R_{B}² = Wasserstoff und (R_{B}³) = 2-OMe ist ("Cyprosulfamide", S4-1),
R_{B}¹ = Cyclopropyl, R_{B}² = Wasserstoff und (R_{B}³) = 5-Cl-2-OMe ist (S4-2),
R_{B}¹ = Ethyl, R_{B}² = Wasserstoff und (R_{B}³) = 2-OMe ist (S4-3),
R_{B}¹ = Isopropyl, R_{B}² = Wasserstoff und (R_{B}³) = 5-Cl-2-OMe ist (S4-4) und
R_{B}¹ = Isopropyl, R_{B}² = Wasserstoff und (R_{B}³) = 2-OMe ist (S4-5);

- S4^{c}): Verbindungen aus der Klasse der Benzoylsulfamoylphenylharnstoffe der Formel (S4^{c}), wie sie in der EP-A-365484 beschrieben sind, 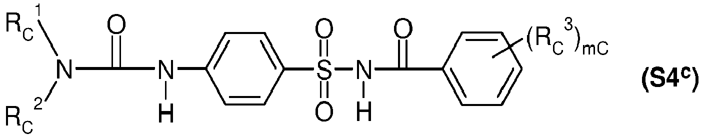 worin
Rc¹, R_{c}² unabhängig voneinander Wasserstoff, (C₁-C₈)-Alkyl, (C₃-C₈)-Cycloalkyl, (C₃-C₆)-Alkenyl, (C₃-C₆)-Alkinyl,
R_{c}³ Halogen, (C₁-C₄)-Alkyl, (C₁-C₄)-Alkoxy, CF₃ und
m_{c} 1 oder 2 bedeuten; beispielsweise 1-[4-(N-2-Methoxybenzoylsulfamoyl)phenyl]-3-methylharnstoff, 1-[4-(N-2-Methoxybenzoylsulfamoyl)phenyl]-3,3-dimethylharnstoff, 1-[4-(N-4,5-Dimethylbenzoylsulfamoyl)phenyl]-3-methylharnstoff;
S4^{d}) Verbindungen vom Typ der N-Phenylsulfonylterephthalamide der Formel (S4^{d}) und deren Salze, die z.B. bekannt sind aus CN 101838227, 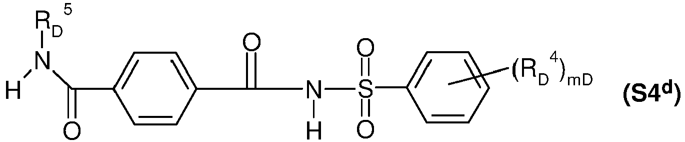 worin
R_{D}⁴ Halogen, (C₁-C₄)-Alkyl, (C₁-C₄)-Alkoxy, CF_{3;}
m_{D} 1 oder 2;
R_{D}⁵ Wasserstoff, (C₁-C₆)-Alkyl, (C₃-C₆)-Cycloalkyl, (C₂-C₆)-Alkenyl, (C₂-C₆)-Alkinyl, (C₅-C₆)-Cycloalkenyl bedeutet.
S5) Wirkstoffe aus der Klasse der Hydroxyaromaten und der aromatisch-aliphatischen Carbonsäurederivate (S5), z.B. 3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B. 1-Methyl-3-(2-thienyl)-1,2-dihydrochinoxalin-2-on, 1-Methyl-3-(2-thienyl)-1,2-dihydro-chinoxalin-2-thion, 1-(2-Aminoethyl)-3-(2-thienyl)-1,2-dihydro-chinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen aus der Klasse der Diphenylmethoxyessigsäurederivate (S7), z.B. Diphenylmethoxyessigsäuremethylester (CAS-Reg.Nr. 41858-19-9) (S7-1), Diphenylmethoxyessigsäureethylester oder Diphenylmethoxyessigsäure wie sie in der WO-A-98/38856 beschrieben sind.
S8) Verbindungen der Formel (S8), wie sie in der WO-A-98/27049 beschrieben sind, 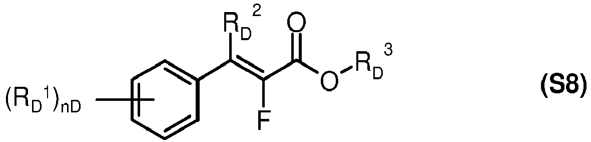 worin die Symbole und Indizes folgende Bedeutungen haben:
R_{D}¹ ist Halogen, (C₁-C₄)-Alkyl, (C₁-C₄)-Haloalkyl, (C₁-C₄)-Alkoxy, (C₁-C₄)-Haloalkoxy,
R_{D}² ist Wasserstoff oder (C₁-C₄)-Alkyl,
R_{D}³ ist Wasserstoff, (C₁-C₈)-Alkyl, (C₂-C₄)-Alkenyl, (C₂-C₄)-Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; oder deren Salze,
n_{D} ist eine ganze Zahl von 0 bis 2.
S9) Wirkstoffe aus der Klasse der 3-(5-Tetrazolylcarbonyl)-2-chinolone (S9), z.B. 1,2-Dihydro-4-hydroxy-1-ethyl-3-(5-tetrazolylcarbonyl)-2-chinolon (CAS-Reg.Nr.: 219479-18-2), 1,2-Dihydro-4-hydroxy-1-methyl-3-(5-tetrazolyl-carbonyl)-2-chinolon (CAS-Reg.Nr. 95855-00-8), wie sie in der WO-A-1999/000020 beschrieben sind.
S10) Verbindungen der Formeln (S10^{a}) oder (S10^{b}), wie sie in der WO-A-2007/023719 und WO-A-2007/023764 beschrieben sind, worin 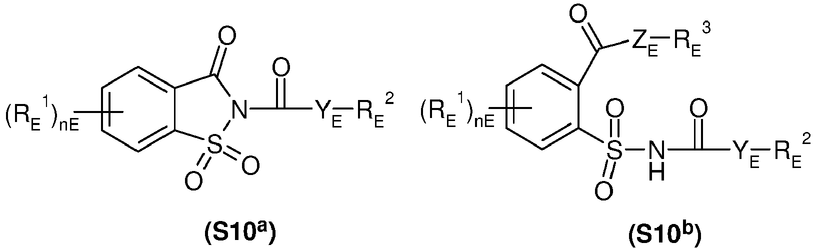 R_{E}¹ Halogen, (C₁-C₄)Alkyl, Methoxy, Nitro, Cyano, CF₃, OCF₃
Y_{E}, Z_{E} unabhängig voneinander O oder S,
n_{E} eine ganze Zahl von 0 bis 4,
R_{E}² (C₁-C₁₆)-Alkyl, (C₂-C₆)-Alkenyl, (C₃-C₆)-Cycloalkyl, Aryl; Benzyl, Halogenbenzyl,
R_{E}³ Wasserstoff oder (C₁-C₆)-Alkyl bedeuten.
S11) Wirkstoffe vom Typ der Oxyimino-Verbindungen (S11), die als Saatbeizmittel bekannt sind, wie z. B. "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist, "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und "Cyometrinil" oder "CGA-43089" ((Z)-Cyanomethoxyimino(phenyl)acetonitril) (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetat (CAS-Reg.Nr. 205121-04-6) (S12-1) und verwandte Verbindungen aus WO-A-1998/13361.
S13) Eine oder mehrere Verbindungen aus Gruppe (S13): "Naphthalic anhydrid" (1,8-Naphthalindicarbonsäureanhydrid) (S13-1), das als Saatbeiz-Safener für Mais gegen Schäden von Thiocarbamatherbiziden bekannt ist, "Fenclorim" (4,6-Dichlor-2-phenylpyrimidin) (S13-2), das als Safener für Pretilachlor in gesätem Reis bekannt ist, "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist, "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist, "MG 191" (CAS-Reg.Nr. 96420-72-3) (2-Dichlormethyl-2-methyl-1,3-dioxolan) (S13-5) der Firma Nitrokemia, das als Safener für Mais bekannt ist, "MG 838" (CAS-Reg.Nr. 133993-74-5) (2-propenyl 1-oxa-4-azaspiro[4.5]decan-4-carbodithioat) (S13-6) der Firma Nitrokemia "Disulfoton" (O,O-Diethyl S-2-ethylthioethyl phosphordithioat) (S13-7), "Dietholate" (O,O-Diethyl-O-phenylphosphorothioat) (S13-8), "Mephenate" (4-Chlorphenyl-methylcarbamat) (S13-9).
S14) Wirkstoffe, die neben einer herbiziden Wirkung gegen Schadpflanzen auch Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist, "Daimuron" oder "SK 23" (1-(1-Methyl-1-phenylethyl)-3-p-tolyl-harnstoff), das als Safener für Reis gegen Schäden des Herbizids Imazosulfuron bekannt ist, "Cumyluron" = "JC-940" (3-(2-Chlorphenylmethyl)-1-(1-methyl-1-phenyl-ethyl)harnstoff, siehe JP-A-60087254), das als Safener für Reis gegen Schäden einiger Herbizide bekannt ist, "Methoxyphenon" oder "NK 049" (3,3'-Dimethyl-4-methoxy-benzophenon), das als Safener für Reis gegen Schäden einiger Herbizide bekannt ist, "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Verbindungen der Formel (S15) oder deren Tautomere, 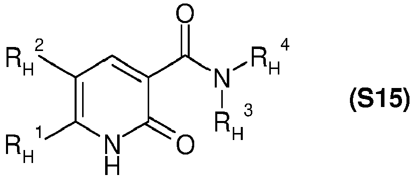 wie sie in der WO-A-2008/131861 und WO-A-2008/131860 beschrieben sind, worin
R_{H}¹ einen (C₁-C₆)-Haloalkylrest bedeutet und
R_{H}² Wasserstoff oder Halogen bedeutet und
R_{H}3, R_{H}⁴ unabhängig voneinander Wasserstoff, (C₁-C₁₆)-Alkyl, (C₂-C₁₆)-Alkenyl oder (C₂-C₁₆)-Alkinyl, wobei jeder der letztgenannten 3 Reste unsubstituiert oder durch einen oder mehrere Reste aus der Gruppe Halogen, Hydroxy, Cyano, (C₁-C₄)-Alkoxy, (C₁-C₄)-Haloalkoxy, (C₁-C₄)-Alkylthio, (C₁-C₄)-Alkylamino, Di[(C₁-C₄)alkyl]-amino, [(C₁-C₄)Alkoxy]-carbonyl, [(C₁-C₄)Haloalkoxy]-carbonyl, (C₃-C₆)-Cycloalkyl, das unsubstituiert oder substituiert ist, Phenyl, das unsubstituiert oder substituiert ist, und Heterocyclyl, das unsubstituiert oder substituiert ist, substituiert ist, oder (C₃-C₆)-Cycloalkyl, (C₄-C₆)-Cycloalkenyl, (C₃-C₆)-Cycloalkyl, das an einer Seite des Rings mit einem 4 bis 6-gliedrigen gesättigten oder ungesättigten carbocyclischen Ring kondensiert ist, oder (C₄-C₆)-Cycloalkenyl, das an einer Seite des Rings mit einem 4 bis 6-gliedrigen gesättigten oder ungesättigten carbocyclischen Ring kondensiert ist, wobei jeder der letztgenannten 4 Reste unsubstituiert oder durch einen oder mehrere Reste aus der Gruppe Halogen, Hydroxy, Cyano, (C₁-C₄)-Alkyl, (C₁-C₄)-Haloalkyl, (C₁-C₄)-Alkoxy, (C₁-C₄)-Haloalkoxy, (C₁-C₄)-Alkylthio, (C₁-C₄)-Alkylamino, Di[(C₁-C₄)alkyl]-amino, [(C₁-C₄)Alkoxy]-carbonyl, [(C₁-C₄)Haloalkoxy]-carbonyl, (C₃-C₆)Cycloalkyl, das unsubstituiert oder substituiert ist, Phenyl, das unsubstituiert oder substituiert ist, und Heterocyclyl, das unsubstituiert oder substituiert ist, substituiert ist, bedeutet oder
R_{H}³ (C₁-C₄)-Alkoxy, (C₂-C₄)-Alkenyloxy, (C₂-C₆)-Alkinyloxy oder (C₂-C₄)-Haloalkoxy bedeutet und
R_{H}⁴ Wasserstoff oder (C₁-C₄)-Alkyl bedeutet oder
R_{H}³ und R_{H}⁴ zusammen mit dem direkt gebundenen N-Atom einen vier- bis achtgliedrigen heterocyclischen Ring, der neben dem N-Atom auch weitere Heteroringatome, vorzugsweise bis zu zwei weitere Heteroringatome aus der Gruppe N, O und S enthalten kann und der unsubstituiert oder durch einen oder mehrere Reste aus der Gruppe Halogen, Cyano, Nitro, (C₁-C₄)-Alkyl, (C₁-C₄)-Haloalkyl, (C₁-C₄)-Alkoxy, (C₁-C₄)-Haloalkoxy und (C₁-C₄)-Alkylthio substituiert ist, bedeutet.
S16) Wirkstoffe, die vorrangig als Herbizide eingesetzt werden, jedoch auch Safenerwirkung auf Kulturpflanzen aufweisen, z. B.

(2,4-Dichlorphenoxy)essigsäure (2,4-D),
(4-Chlorphenoxy)essigsäure,
(R,S)-2-(4-Chlor-o-tolyloxy)propionsäure (Mecoprop),
4-(2,4-Dichlorphenoxy)buttersäure (2,4-DB),
(4-Chlor-o-tolyloxy)essigsäure (MCPA),
4-(4-Chlor-o-tolyloxy)buttersäure,
4-(4-Chlorphenoxy)buttersäure,
3,6-Dichlor-2-methoxybenzoesäure (Dicamba),
1-(Ethoxycarbonyl)ethyl-3,6-dichlor-2-methoxybenzoat (Lactidichlor-ethyl).

Bevorzugte Safener in Kombination mit den erfindungsgemäßen Verbindungend der Formel (I) und/oder deren Salze, insbesondere mit den Verbindungen der Formeln (I.XXX) bis (I.YYY) und/oder deren Salze sind: Cloquintocet-mexyl, Cyprosulfamid, Fenchlorazol-ethylester, Isoxadifen-ethyl, Mefenpyr-diethyl, Fenclorim, Cumyluron, S4-1 und S4-5, und besonders bevorzugte Safener sind: Cloquintocet-mexyl, Cyprosulfamid, Isoxadifen-ethyl und Mefenpyr-diethyl.

### Biologische Beispiele:

Die folgenden Abkürzungen werden für die in den folgenden Tabellen aufgeführten Kultur- und Schadpflanzen verwendet:
- ABUTH:: Abutilon theophrasti
- ALOMY:: Alopecurus myosuroides
- AMARE:: Amaranthus retroflexus
- BRSNS:: Brassica napus
- DIGSA:: Digitaria sanguinalis
- ECHCG:: Echinochloa crus-galli
- GLXMA:: Glycine max
- KCHSC:: Kochia scoparia
- LOLRI:: Lolium rigidum
- MATIN:: Matricaria inodora
- ORYZA:: Oryza sativa
- PHPBU:: Pharbitis purpurea
- POLCO:: Polygonum convolvulus
- SETVI:: Setaria viridis
- VERPE:: Veronica persica
- VIOTR:: Viola tricolor
- TRZAS:: Triticum aestivum
- ZEAMX:: Zea mays

### A. Herbizide Wirkung im Nachauflauf

Samen von mono- bzw. dikotylen Unkrautpflanzen wurden in Kunststoff- oder Holzfasertöpfen in sandigem Lehmboden ausgelegt, mit Erde abgedeckt und im Gewächshaus unter kontrollierten Wachstumsbedingungen angezogen. 2 bis 3 Wochen nach der Aussaat wurden die Versuchspflanzen im Einblattstadium behandelt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 1/ha auf die grünen Pflanzenteile gesprüht. Nach ca. 3 Wochen Standzeit der Versuchspflanzen im Gewächshaus, unter optimalen Wachstumsbedingungen, wurde die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

In den nachstehenden Tabellen A1 bis A11 sind die Wirkungen ausgewählter Verbindungen der allgemeinen Formel (I) gemäß der Tabelle 1 auf verschiedene Schadpflanzen und einer Aufwandmenge entsprechend 80 g/ha und niedriger, die gemäß zuvor genannter Versuchsvorschrift erhalten wurden, dargestellt.

**Tabelle A1a: Nachauflaufwirkung bei 5 g/ha gegen ABUTH in %**

| Beispielnummer | Dosierung [g/ha] | ABUTH |
|---|---|---|
| I.1-2 | 5 | 100 |
| I.1-176 | 5 | 100 |
| I.1-1 | 5 | 100 |
| I.1-41 | 5 | 100 |
| I.1-476 | 5 | 100 |
| I.1-286 | 5 | 100 |
| I.1-301 | 5 | 100 |

**Tabelle Alb: Nachauflaufwirkung bei 20 g/ha gegen ABUTH in %**

| Beispielnummer | Dosierung [g/ha] | ABUTH |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle A1c: Nachauflaufwirkung bei 80 g/ha gegen ABUTH in %**

| Beispielnummer | Dosierung [g/ha] | ABUTH |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A2a: Nachauflaufwirkung bei 20 g/ha gegen ALOMYin %**

| Beispielnummer | Dosierung [g/ha] | ALOMY |
|---|---|---|
| I.1-2 | 20 | 90 |
| I.1-176 | 20 | 80 |
| I.1-1 | 20 | 100 |
| I.1-41 | 20 | 90 |
| I.1-476 | 20 | 100 |
| I.1-286 | 20 | 100 |
| I.1-301 | 20 | 80 |

**Tabelle A2b: Nachauflaufwirkung bei 80 g/ha gegen ALOMY in %**

| Beispielnummer | Dosierung [g/ha] | ALOMY |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A3a: Nachauflaufwirkung bei 5 g/ha gegen AMARE in %**

| Beispielnummer | Dosierung [g/ha] | AMARE |
|---|---|---|
| I.1-2 | 5 | 100 |
| I.1-176 | 5 | 100 |
| I.1-1 | 5 | 100 |
| I.1-41 | 5 | 100 |
| I.1-476 | 5 | 100 |
| I.1-286 | 5 | 100 |
| I.1-301 | 5 | 100 |

**Tabelle A3b: Nachauflaufwirkung bei 20 g/ha gegen AMARE in %**

| Beispielnummer | Dosierung [g/ha] | AMARE |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle A3c: Nachauflaufwirkung bei 80 g/ha gegen AMARE in %**

| Beispielnummer | Dosierung [g/ha] | AMARE |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A4a: Nachauflaufwirkung bei 5 g/ha gegen DIGSA in %**

| Beispielnummer | Dosierung [g/ha] | DIGSA |
|---|---|---|
| I.1-2 | 5 | 100 |
| I.1-176 | 5 | 100 |
| I.1-1 | 5 | 100 |
| I.1-41 | 5 | 100 |
| I.1-476 | 5 | 100 |
| I.1-286 | 5 | 100 |
| I.1-301 | 5 | 80 |

**Tabelle A4b: Nachauflaufwirkung bei 20 g/ha gegen DIGSA in %**

| Beispielnummer | Dosierung [g/ha] | DIGSA |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle A4c: Nachauflaufwirkung bei 80 g/ha gegen DIGSA in %**

| Beispielnummer | Dosierung [g/ha] | DIGSA |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A5a: Nachauflaufwirkung bei 20 g/ha gegen LOLRI in %**

| Beispielnummer | Dosierung [g/ha] | LOLRI |
|---|---|---|
| I.1-1 | 20 | 100 |
| I.1-41 | 20 | 80 |
| I.1-286 | 20 | 90 |

**Tabelle A5b: Nachauflaufwirkung bei 80 g/ha gegen LOLRI in %**

| Beispielnummer | Dosierung [g/ha] | LOLRI |
|---|---|---|
| I.1-2 | 80 | 100 |
| I.1-176 | 80 | 90 |
| I.1-1 | 80 | 100 |
| I.1-41 | 80 | 80 |
| I.1-476 | 80 | 100 |
| I.1-286 | 80 | 100 |
| I.1-301 | 80 | 100 |

**Tabelle A6a: Nachauflaufwirkung bei 5 g/ha gegen MATIN in %**

| Beispielnummer | Dosierung [g/ha] | MATIN |
|---|---|---|
| I.1-2 | 5 | 100 |
| I.1-176 | 5 | 100 |
| I.1-1 | 5 | 100 |
| I.1-41 | 5 | 100 |
| I.1-476 | 5 | 100 |
| I.1-286 | 5 | 100 |
| I.1-301 | 5 | 100 |

**Tabelle A6b: Nachauflaufwirkung bei 20 g/ha gegen MATIN in %**

| Beispielnummer | Dosierung [g/ha] | MATIN |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle A6c: Nachauflaufwirkung bei 80 g/ha gegen MATIN in %**

| Beispielnummer | Dosierung [g/ha] | MATIN |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A7a: Nachauflaufwirkung bei 5 g/ha gegen PHBPU in %**

| Beispielnummer | Dosierung [g/ha] | PHBPU |
|---|---|---|
| I.1-2 | 5 | 100 |
| I.1-176 | 5 | 100 |
| I.1-1 | 5 | 80 |
| I.1-41 | 5 | 90 |
| I.1-476 | 5 | 100 |
| I.1-286 | 5 | 100 |
| I.1-301 | 5 | 100 |

**Tabelle A7b: Nachauflaufwirkung bei 20 g/ha gegen PHBPU in %**

| Beispielnummer | Dosierung [g/ha] | PHBPU |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle A7c: Nachauflaufwirkung bei 80 g/ha gegen PHBPU in %**

| Beispielnummer | Dosierung [g/ha] | PHBPU |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A8a: Nachauflaufwirkung bei 5 g/ha gegen POLCO in %**

| Beispielnummer | Dosierung [g/ha] | POLCO |
|---|---|---|
| I.1-2 | 5 | 100 |
| I.1-176 | 5 | 100 |
| I.1-1 | 5 | 100 |
| I.1-476 | 5 | 100 |
| I.1-286 | 5 | 100 |
| I.1-301 | 5 | 100 |

**Tabelle A8b: Nachauflaufwirkung bei 20 g/ha gegen POLCO in %**

| Beispielnummer | Dosierung [g/ha] | POLCO |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle A8c: Nachauflaufwirkung bei 80 g/ha gegen POLCO in %**

| Beispielnummer | Dosierung [g/ha] | POLCO |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A9a: Nachauflaufwirkung bei 5 g/ha gegen SETVI in %**

| Beispielnummer | Dosierung [g/ha] | SETVI |
|---|---|---|
| I.1-2 | 5 | 100 |
| I.1-176 | 5 | 100 |
| I.1-1 | 5 | 100 |
| I.1-41 | 5 | 100 |
| I.1-476 | 5 | 100 |
| I.1-286 | 5 | 100 |
| I.1-301 | 5 | 100 |

**Tabelle A9b: Nachauflaufwirkung bei 20 g/ha gegen SETVI in %**

| Beispielnummer | Dosierung [g/ha] | SETVI |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle A9c: Nachauflaufwirkung bei 20 g/ha gegen SETVI in %**

| Beispielnummer | Dosierung [g/ha] | SETVI |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A10a: Nachauflaufwirkung bei 5 g/ha gegen VERPE in %**

| Beispielnummer | Dosierung [g/ha] | VERPE |
|---|---|---|
| I.1-2 | 5 | 100 |
| I.1-176 | 5 | 100 |
| I.1-1 | 5 | 100 |
| I.1-41 | 5 | 100 |
| I.1-476 | 5 | 100 |
| I.1-286 | 5 | 100 |
| I.1-301 | 5 | 100 |

**Tabelle A10b: Nachauflaufwirkung bei 20 g/ha gegen VERPE in %**

| Beispielnummer | Dosierung [g/ha] | VERPE |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle AlOc: Nachauflaufwirkung bei 80 g/ha gegen VERPE in %**

| Beispielnummer | Dosierung [g/ha] | VERPE |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

**Tabelle A11a: Nachauflaufwirkung bei 5 g/ha gegen VIOTR in %**

| Beispielnummer | Dosierung [g/ha] | VIOTR |
|---|---|---|
| I.1-2 | 5 | 100 |
| I.1-176 | 5 | 100 |
| I.1-1 | 5 | 100 |
| I.1-41 | 5 | 100 |
| I.1-476 | 5 | 100 |
| I.1-286 | 5 | 100 |
| I.1-301 | 5 | 100 |

**Tabelle A11b: Nachauflaufwirkung bei 20 g/ha gegen VIOTR in %**

| Beispielnummer | Dosierung [g/ha] | VIOTR |
|---|---|---|
| I.1-2 | 20 | 100 |
| I.1-176 | 20 | 100 |
| I.1-1 | 20 | 100 |
| I.1-41 | 20 | 100 |
| I.1-476 | 20 | 100 |
| I.1-286 | 20 | 100 |
| I.1-301 | 20 | 100 |

**Tabelle A11c: Nachauflaufwirkung bei 80 g/ha gegen VIOTR in %**

| Beispielnummer | Dosierung [g/ha] | VIOTR |
|---|---|---|
| I.1-2 | 80 | 100 |
| I.1-176 | 80 | 100 |
| I.1-1 | 80 | 100 |
| I.1-41 | 80 | 100 |
| I.1-476 | 80 | 100 |
| I.1-286 | 80 | 100 |
| I.1-301 | 80 | 100 |

Wie die Ergebnisse zeigen, weisen erfindungsgemäße Verbindungen der allgemeinen Formel (I) bei Behandlung im Nachauflauf eine gute herbizide Wirksamkeit gegen Schadpflanzen auf wie z. B. *Abutilon theophrasti, Alopecurus myosuroides, Amaranthus retroflexus, Digitaria sanguinalis, Lolium rigidum, Matricaria inodora, Pharbitis purpurea, Polygonum convolvulus, Setaria viridis, Veronica persica* und *Viola tricolor* bei einer Aufwandmenge von 80 g Aktivsubstanz oder weniger pro Hektar, auf.

### B. Wirkung auf Kulturpflanzen im Nachauflauf

Samen von mono- bzw. dikotylen Kulturpflanzen wurden in Kunststoff- oder Holzfasertöpfen in sandigem Lehmboden ausgelegt, mit Erde abgedeckt und im Gewächshaus unter kontrollierten Wachstumsbedingungen angezogen. 2 bis 3 Wochen nach der Aussaat wurden die Versuchspflanzen im Einblattstadium behandelt. Die in Form von benetzbaren Pulvern (WP) oder als Emulsionskonzentrate (EC) formulierten erfindungsgemäßen Verbindungen wurden dann als wässrige Suspension bzw. Emulsion unter Zusatz von 0,5% Additiv mit einer Wasseraufwandmenge von umgerechnet 600 1/ha auf die grünen Pflanzenteile gesprüht. Nach ca. 3 Wochen Standzeit der Versuchspflanzen im Gewächshaus, unter optimalen Wachstumsbedingungen, wurde die Wirkung der Präparate visuell im Vergleich zu unbehandelten Kontrollen bonitiert. Beispielsweise bedeutet 100% Wirkung = Pflanzen sind abgestorben, 0% Wirkung = wie Kontrollpflanzen.

In den nachstehenden Tabellen B1 bis B3 sind die Wirkungen ausgewählter Verbindungen der allgemeinen Formel (I) gemäß der Tabelle 1 auf verschiedene Kulturpflanzen und einer Aufwandmenge entsprechend 20 g/ha und niedriger, die gemäß zuvor genannter Versuchsvorschrift erhalten wurden, dargestellt.

**Tabelle B1a: Nachauflaufwirkung bei 5 g/ha gegen ZEAMX in %**

| Beispielnummer | Dosierung [g/ha] | ZEAMX |
|---|---|---|
| I.1-2 | 5 | 20 |
| I.1-1 | 5 | 10 |
| I.1-476 | 5 | 20 |
| I.1-286 | 5 | 20 |
| I.1-301 | 5 | 20 |

**Tabelle B2a: Nachauflaufwirkung bei 5 g/ha gegen TRZAS in %**

| Beispielnummer | Dosierung [g/ha] | TRZAS |
|---|---|---|
| I.1-2 | 5 | 10 |
| I.1-176 | 5 | 10 |
| I.1-1 | 5 | 10 |
| I.1-41 | 5 | 10 |
| I.1-476 | 5 | 10 |
| I.1-286 | 5 | 10 |
| I.1-301 | 5 | 10 |

**Tabelle B2b: Nachauflaufwirkung bei 20 g/ha gegen TRZAS in %**

| Beispielnummer | Dosierung [g/ha] | TRZAS |
|---|---|---|
| I.1-2 | 20 | 20 |
| I.1-176 | 20 | 10 |
| I.1-41 | 20 | 10 |
| I.1-476 | 20 | 10 |
| I.1-286 | 20 | 20 |
| I.1-301 | 20 | 10 |

**Tabelle B3a: Nachauflaufwirkung bei 5 g/ha gegen ORYZA in %**

| Beispielnummer | Dosierung [g/ha] | TRZAS |
|---|---|---|
| I.1-2 | 5 | 0 |
| I.1-176 | 5 | 0 |
| I.1-1 | 5 | 20 |
| I.1-41 | 5 | 0 |
| I.1-286 | 5 | 0 |
| I.1-301 | 5 | 0 |

Wie die Ergebnisse zeigen, weisen erfindungsgemäße Verbindungen der allgemeinen Formel (I) bei Behandlung im Nachauflauf eine gute Kulturpflanzenverträglichkeit bei Organismen, wie *Zea mays und Triticum aestivum* bei einer Aufwandmenge von 20 g oder weniger pro Hektar.

## Patentansprüche

1. Substituierte N-Benzoesäureuracile der allgemeinen Formel (I) oder deren Salze 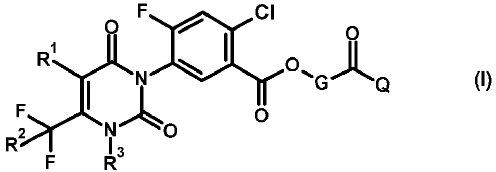 worin
R¹ für Wasserstoff, Methyl steht,
R² für Wasserstoff, Halogen, Trifluormethyl steht,
R³ für (C₁-C₆)-Alkyl steht,
G für einen Rest der nachfolgenden Formel 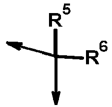 steht,
R⁵ und R⁶ unabhängig voneinander für Wasserstoff, (C₁-C₈)-Alkyl, (C₁-C₈)-Haloalkyl, (C₁-C₈)-Alkoxy-(C₁-C₈)-Alkyl stehen,
R⁵ und R⁶ mit dem Kohlenstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
Q für einen Rest der nachfolgenden Formeln 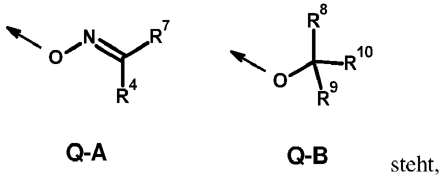
R⁴ und R⁷ unabhängig voneinander für (C₁-C₈)-Alkyl, (C₃-C₈)-Cycloalkyl, Aryl, Heteroaryl, Heterocyclyl stehen und
R⁴ und R⁷ mit dem Kohlenstoffatom, an das sie gebunden sind, einen vollständig gesättigten monocyclischen 3- bis 7-gliedrigen Carbocyclus bilden,
R⁸ für Wasserstoff, (C₁-C₈)-Alkyl, (C₁-C₈)-Haloalkyl, Aryl, Aryl-(C₁-C₈)-alkyl, Heteroaryl, (C₂-C₈)-Alkinyl, (C₂-C₈)-Alkenyl, C(O)R¹³, C(O)OR¹³, (C₁-C₈)-Alkoxy-(C₁-C₈)-alkyl steht,
R⁹ für Wasserstoff oder (C₁-C₈)-Alkyl steht,
R¹⁰ für Halogen, Cyano, NO₂, (C₁-C₈)-Haloalkyl, (C₃-C₈)-Cycloalkyl, (C₃-C₈)-Cycloalkyl-(C₁-C₈)-alkyl, (C₃-C₈)-Halocycloalkyl, (C₃-C₈)-Halocycloalkyl-(C₁-C₈)-alkyl, Aryl, Aryl-(C₁-C₈)-alkyl, Heteroaryl, Heteroaryl-(C₁-C₈)-alkyl, Heterocyclyl, Heterocyclyl-(C₁-C₈)-alkyl, R¹¹R¹²N-(C₁-C₈)-alkyl, R¹³O-(C₁-C₈)-alkyl, Cyano-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, OR¹³, NR¹¹R¹², SR¹⁴, S(O)R¹⁴, SO₂R¹⁴, R¹⁴S-(C₁-C₈)-alkyl, R¹⁴(O)S-(C₁-C₈)-alkyl, R¹⁴O₂S-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, Tris-[(C₁-C₈)-Alkyl]silyl, Bis-hydroxyboryl-(C₁-C₈)-alkyl, Bis-[(C₁-C₈)-alkoxy]boryl-(C₁-C₈)-alkyl, Tetramethyl-1,3,2-Dioxaborolan-2-yl, Tetramethyl-1,3,2-Dioxaborolan-2-yl-(C₁-C₈)-alkyl, Nitro-(C₁-C₈)-alkyl, C(O)OR ¹³, C(O)R¹³, C(O)NR¹¹R¹², R¹³O(O)C-(C₁-C₈)-alkyl, R¹¹R¹²N(O)C-(C₁-C₈)-alkyl, Bis-(C₁-C₈)-alkoxy-(C₁-C₈)-alkyl steht,
R¹¹ und R¹² gleich oder verschieden sind und unabhängig voneinander für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkylthio-(C₁-C₈)-alkyl, (C₁-C₈)-Haloalkylthio-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, C(O)R¹³, SO₂R¹⁴, Heterocyclyl, (C₁-C₈)-Alkoxycarbonyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Aryl-(C₁-C₈)-alkoxycarbonyl, Heteroaryl-(C₁-C₈)-alkoxycarbonyl, (C₂-C₈)-Alkenyloxycarbonyl, (C₂-C₈)-Alkinyloxycarbonyl, Heterocyclyl-(C₁-C₈)-alkyl stehen,oder
R¹¹ und R¹² mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
R¹³ für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Haloalkoxy-(C₁-C₈)-alkyl, (C₁-C₈)-Alkoxy-(C₁-C₈)-haloalkyl, (C₁-C₈)-Alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkyl, (C₁-C₈)-Alkoxy-(C₁-C₈)-alkoxy-(C₁-C₈)-alkoxy-(C₁-C₈)-alkoxy-(C₁-C₈)-alkyl, Aryl, Aryl-(C₁-C₈)-alkyl, Aryl-(C₁-C₈)-alkoxy-(C₁-C₈)-alkyl, Heteroaryl, Heteroaryl-(C₁-C₈)-alkyl, (C₃-C₈)-Cycloalkyl-(C₁-C₈)-alkyl, (C₄-C₁₀)-Cycloalkenyl-(C₁-C₈)-alkyl, Bis-[(C₁-C₈)-alkyl]aminocarbonyl-(C₁-C₈)-alkyl, (C₁-C₈)-Alkyl-aminocarbonyl-(C₁-C₈)-alkyl, Aryl-(C₁-C₈)-alkyl-aminocarbonyl-(C₁-C₈)-alkyl, Bis-[(C₁-C₈)-alkyl]amino-(C₂-C₆)-alkyl, (C₁-C₈)-Alkyl-amino-(C₂-C₆)-alkyl, Aryl-(C₁-C₈)-alkyl-amino-(C₂-C₆)-alkyl, R¹⁴S-(C₁-C₈)-alkyl, R¹⁴(O)S-(C₁-C₈)-alkyl, R¹⁴O₂S-(C₁-C₈)-alkyl, Hydroxycarbonyl-(C₁-C₈)-alkyl, Heterocyclyl, Heterocyclyl-(C₁-C₈)-alkyl, Tris-[(C₁-C₈)-Alkyl]silyl-(C₁-C₈)-alkyl, Bis-[(C₁-C₈)-Alkyl](aryl)silyl(C₁-C₈)-alkyl, [(C₁-C₈)-Alkyl]-bis-(aryl)silyl-(C₁-C₈)-alkyl, (C₁-C₈)-Alkylcarbonyloxy-(C₁-C₈)-alkyl, (C₃-C₈)-Cycloalkylcarbonyloxy-(C₁-C₈)-alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, Aryloxy-(C₁-C₈)-alkyl, Heteroaryloxy-(C₁-C₈)-alkyl, (C₁-C₈)-Alkoxycarbonyl steht
und
R¹⁴ für Wasserstoff, (C₁-C₈)-Alkyl, (C₂-C₈)-Alkenyl, (C₂-C₈)-Alkinyl, (C₁-C₈)-Cyanoalkyl, (C₁-C₁₀)-Haloalkyl, (C₂-C₈)-Haloalkenyl, (C₃-C₈)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-haloalkyl, Aryl, Aryl-(C₁-C₈)-alkyl, Heteroaryl, Heteroaryl-(C₁-C₈)-alkyl, Heterocyclyl-(C₁-C₈)-alkyl, (C₃-C₈)-Cycloalkyl-(C₁-C₈)-alkyl, (C₄-C₁₀)-Cycloalkenyl-(C₁-C₈)-alkyl, Bis-[(C₁-C₈)-alkyl]amino, (C₁-C₈)-Alkyl-amino, Aryl-(C₁-C₈)-amino, Aryl-(C₁-C₆)-alkyl-amino, Aryl-[(C₁-C₈)-alkyl]amino; (C₃-C₈)-Cycloalkyl-amino, (C₃-C₈)-Cycloalkyl-[(C₁-C₈)-alkyl]amino; N-Azetidinyl, N-Pyrrolidinyl, N-Piperidinyl. N-Morpholinyl steht.

2. Verbindungen der allgemeinen Formel (I) gemäß Anspruch 1 und/oder deren Salz, **dadurch gekennzeichnet, dass**
R¹ für Wasserstoff steht,
R² für Wasserstoff, Fluor, Chlor, Brom, Iod steht,
R³ für (C₁-C₅)-Alkyl steht,
G für einen Rest der nachfolgenden Formel 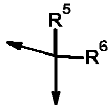 steht,
R⁵ und R⁶ unabhängig voneinander für Wasserstoff, (C₁-C₇)-Alkyl stehen, oder
R⁴ und R⁶ mit dem Kohlenstoffatom, an das sie gebunden sind, einen vollständig gesättigten monocyclischen 3- bis 6-gliedrigen Carbocyclus bilden
Q für einen Rest der nachfolgenden Formeln 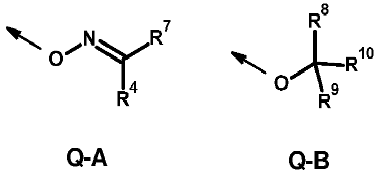 steht,
R⁴ und R⁷ unabhängig voneinander für (C₁-C₇)-Alkyl, (C₃-C₇)-Cycloalkyl, Aryl, Heteroaryl, Heterocyclyl stehen, oder
R⁴ und R⁷ mit dem Kohlenstoffatom, an das sie gebunden sind, einen vollständig gesättigten monocyclischen 3- bis 6-gliedrigen Carbocyclus bilden,
R⁸ für Wasserstoff, (C₁-C₇)-Alkyl, (C₁-C₇)-Haloalkyl, Aryl, Aryl-(C₁-C₇)-alkyl, Heteroaryl, (C₂-C₇)-Alkinyl, (C₂-C₇)-Alkenyl, C(O)R¹³, C(O)OR¹³, (C₁-C₇)-Alkoxy-(C₁-C₇)-alkyl steht,
R⁹ für Wasserstoff oder (C₁-C₇)-Alkyl steht,
R¹⁰ für Halogen, Cyano, NO₂, (C₁-C₇)-Haloalkyl, (C₃-C₇)-Cycloalkyl, (C₃-C₇)-Cycloalkyl-(C₁-C₇)-alkyl, (C₃-C₇)-Halocycloalkyl, (C₃-C₇)-Halocycloalkyl-(C₁-C₇)-alkyl, Aryl, Aryl-(C₁-C₇)-alkyl, Heteroaryl, Heteroaryl-(C₁-C₇)-alkyl, Heterocyclyl, Heterocyclyl-(C₁-C₇)-alkyl, R¹¹R¹²N-(C₁-C₇)-alkyl, R¹³O-(C₁-C₇)-alkyl, Cyano-(C₁-C₇)-alkyl, (C₁-C₇)-Alkylcarbonyloxy-(C₁-C₇)-alkyl, (C₃-C₇)-Cycloalkylcarbonyloxy-(C₁-C₇)-alkyl, Arylcarbonyloxy-(C₁-C₇)-alkyl, Heteroarylcarbonyloxy-(C₁-C₈)-alkyl, Heterocyclylcarbonyloxy-(C₁-C₇)-alkyl, OR¹³, NR¹¹R¹², SR¹⁴, S(O)R¹⁴, SO₂R¹⁴, R¹⁴S-(C₁-C₇)-alkyl, R¹⁴(O)S-(C₁-C₇)-alkyl, R¹⁴O₂S-(C₁-C₇)-alkyl, Tris-[(C₁-C₇)-Alkyl]silyl-(C₁-C₇)-alkyl, Bis-[(C₁-C₇)-Alkyl](aryl)silyl(C₁-C₇)-alkyl, [(C₁-C₇)-Alkyll-bis-(aryl)silyl-(C₁-C₇)-alkyl, Tris-[(C₁-C₇)-Alkyl]silyl, Bis-hydroxyboryl-(C₁-C₇)-alkyl, Bis-[(C₁-C₇)-alkoxy]boryl-(C₁-C₇)-alkyl, Tetramethyl-1,3,2-Dioxaborolan-2-yl, Tetramethyl-1,3,2-Dioxaborolan-2-yl-(C₁-C₈)-alkyl, Nitro-(C₁-C₈)-alkyl, C(O)OR¹³, C(O)R¹³, C(O)NR¹¹R¹², R¹³O(O)C-(C₁-C₈)-alkyl, R¹¹R¹²N(O)C-(C₁-C₈)-alkyl, Bis-(C₁-C₈)-alkoxy-(C₁-C₈)-alkyl steht,
R¹¹ und R¹² gleich oder verschieden sind und unabhängig voneinander für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₃-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Haloalkoxy-(C₁-C₇)-alkyl, (C₁-C₇)-Alkylthio-(C₁-C₇)-alkyl, (C₁-C₇)-Haloalkylthio-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, (C₃-C₇)-Cycloalkyl-(C₁-C₇)-alkyl, (C₄-C₁₀)-Cycloalkenyl-(C₁-C₇)-alkyl, C(O)R¹³, SO₂R¹⁴, Heterocyclyl, (C₁-C₇)-Alkoxycarbonyl, Bis-[(C₁-C₇)-alkyl]aminocarbonyl-(C₁-C₇)-alkyl, (C₁-C₇)-Alkyl-aminocarbonyl-(C₁-C₇)-alkyl, Aryl-(C₁-C₇)-alkyl-aminocarbonyl-(C₁-C₇)-alkyl, Aryl-(C₁-C₇)-alkoxycarbonyl, Heteroaryl-(C₁-C₇)-alkoxycarbonyl, (C₂-C₇)-Alkenyloxycarbonyl, (C₂-C₇)-Alkinyloxycarbonyl, Heterocyclyl-(C₁-C₇)-alkyl stehen, oder
R¹¹ und R¹² mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
R¹³ für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₃-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Haloalkoxy-(C₁-C₇)-alkyl, (C₁-C₇)-Alkoxy-(C₁-C₇)-haloalkyl, (C₁-C₇)-Alkoxy-(C₁-C₇)-alkoxy-(C₁-C₇)-alkyl, (C₁-C₇)-Alkoxy-(C₁-C₇)-alkoxy-(C₁-C₇)-alkoxy-(C₁-C₇)-alkyl, (C₁-C₇)-Alkoxy-(C₁-C₇)-alkoxy-(C₁-C₇)-alkoxy-(C₁-C₇)-alkoxy-(C₁-C₇)-alkyl, Aryl, Aryl-(C₁-C₇)-alkyl, Aryl-(C₁-C₇)-alkoxy-(C₁-C₇)-alkyl, Heteroaryl, Heteroaryl-(C₁-C₇)-alkyl, (C₃-C₇)-Cycloalkyl-(C₁-C₇)-alkyl, (C₄-C₁₀)-Cycloalkenyl-(C₁-C₇)-alkyl, Bis-[(C₁-C₇)-alkyl]aminocarbonyl-(C₁-C₇)-alkyl, (C₁-C₇)-Alkyl-aminocarbonyl-(C₁-C₇)-alkyl, Aryl-(C₁-C₇)-alkyl-aminocarbonyl-(C₁-C₇)-alkyl, Bis-[(C₁-C₇)-alkyl]amino-(C₂-C₆)-alkyl, (C₁-C₇)-Alkyl-amino-(C₂-C₆)-alkyl, Aryl-(C₁-C₇)-alkyl-amino-(C₂-C₆)-alkyl, R¹⁴S-(C₁-C₇)-alkyl, R¹⁴(O)S-(C₁-C₇)-alkyl, R¹⁴O₂S-(C₁-C₇)-alkyl, Hydroxycarbonyl-(C₁-C₇)-alkyl, Heterocyclyl, Heterocyclyl-(C₁-C₇)-alkyl, Tris-[(C₁-C₇)-Alkyl]silyl-(C₁-C₇)-alkyl, Bis-[(C₁-C₇)-Alkyl](aryl)silyl(C₁-C₇)-alkyl,[(C₁-C₇)-Alkyl]-bis-(aryl)silyl-(C₁-C₇)-alkyl,(C₁-C₇)-Alkylcarbonyloxy-(C₁-C₇)-alkyl, (C₃-C₇)-Cycloalkylcarbonyloxy-(C₁-C₇)-alkyl, Arylcarbonyloxy-(C₁-C₇)-alkyl, Heteroarylcarbonyloxy-(C₁-C₇)-alkyl, Heterocyclylcarbonyloxy-(C₁-C₇)-alkyl, Aryloxy-(C₁-C₇)-alkyl, Heteroaryloxy-(C₁-C₇)-alkyl, (C₁-C₇)-Alkoxycarbonyl steht
und
R¹⁴ für Wasserstoff, (C₁-C₇)-Alkyl, (C₂-C₇)-Alkenyl, (C₂-C₇)-Alkinyl, (C₁-C₇)-Cyanoalkyl, (C₁-C₁₀)-Haloalkyl, (C₂-C₇)-Haloalkenyl, (C₃-C₇)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₇)-Alkoxy-(C₁-C₇)-alkyl, (C₁-C₇)-Alkoxy-(C₁-C₇)-haloalkyl, Aryl, Aryl-(C₁-C₇)-alkyl, Heteroaryl, Heteroaryl-(C₁-C₇)-alkyl, Heterocyclyl-(C₁-C₇)-alkyl, (C₃-C₇)-Cycloalkyl-(C₁-C₇)-alkyl, (C₄-C₁₀)-Cycloalkenyl-(C₁-C₇)-alkyl, Bis-[(C₁-C₇)-alkyl]amino, (C₁-C₇)-Alkyl-amino, Aryl-(C₁-C₇)-amino, Aryl-(C₁-C₆)-alkyl-amino, Aryl-[(C₁-C₇)-alkyl]amino; (C₃-C₇)-Cycloalkyl-amino, (C₃-C₇)-Cycloalkyl-[(C₁-C₇)-alkyl]amino; N-Azetidinyl, N-Pyrrolidinyl, N-Piperidinyl. N-Morpholinyl, steht.

3. Verbindungen der allgemeinen Formel (I) gemäß Anspruch 1 und/oder deren Salz, **dadurch gekennzeichnet, dass**
R¹ für Wasserstoff steht,
R² für Wasserstoff, Fluor, Chlor, Brom steht,
R³ für (C₁-C₄)-Alkyl steht,
G für einen Rest der nachfolgenden Formel 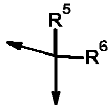 steht,
R⁵ und R⁶ unabhängig voneinander für Wasserstoff, (C₁-C₆)-Alkyl stehen, oder
R⁴ und R⁶ mit dem Kohlenstoffatom, an das sie gebunden sind, einen vollständig gesättigten monocyclischen 3- bis 6-gliedrigen Carbocyclus bilden
Q für einen Rest der nachfolgenden Formeln 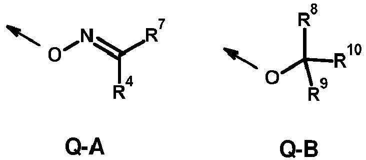 steht,
R⁴ und R⁷ unabhängig voneinander für (C₁-C₇)-Alkyl, (C₃-C₇)-Cycloalkyl, Aryl stehen, oder
R⁴ und R⁷ mit dem Kohlenstoffatom, an das sie gebunden sind, einen vollständig gesättigten monocyclischen 3- bis 6-gliedrigen Carbocyclus bilden,
R⁸ für Wasserstoff, (C₁-C₆)-Alkyl, (C₁-C₆)-Haloalkyl, Aryl, Aryl-(C₁-C₆)-alkyl, Heteroaryl, (C₂-C₆)-Alkinyl, (C₂-C₆)-Alkenyl, C(O)R¹³, C(O)OR¹³, (C₁-C₆)-Alkoxy-(C₁-C₆)-alkyl steht,
R⁹ für Wasserstoff oder (C₁-C₆)-Alkyl steht,
R¹⁰ für Halogen, Cyano, NO₂, (C₁-C₆)-Haloalkyl, (C₃-C₆)-Cycloalkyl, (C₃-C₆)-Cycloalkyl-(C₁-C₆)-alkyl, (C₃-C₆)-Halocycloalkyl, (C₃-C₆)-Halocycloalkyl-(C₁-C₆)-alkyl, Aryl, Aryl-(C₁-C₆)-alkyl, Heteroaryl, Heteroaryl-(C₁-C₆)-alkyl, Heterocyclyl, Heterocyclyl-(C₁-C₆)-alkyl, R¹¹R¹²N-(C₁-C₆)-alkyl, R¹³O-(C₁-C₆)-alkyl, Cyano-(C₁-C₆)-alkyl, (C₁-C₆)-Alkylcarbonyloxy-(C₁-C₇)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₈)-alkyl, Heterocyclylcarbonyloxy-(C₁-C₇)-alkyl, OR¹³, NR¹¹R¹², SR¹⁴, S(O)R¹⁴, SO₂R¹⁴, R¹⁴S-(C₁-C₆)-alkyl, R¹⁴(O)S-(C₁-C₆)-alkyl, R¹⁴O₂S-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, Tris-[(C₁-C₆)-Alkyl]silyl, Bis-hydroxyboryl-(C₁-C₆)-alkyl, Bis-[(C₁-C₆)-alkoxy]boryl-(C₁-C₆)-alkyl, Tetramethyl-1,3,2-Dioxaborolan-2-yl, Tetramethyl-1,3,2-Dioxaborolan-2-yl-(C₁-C₆)-alkyl, Nitro-(C₁-C₆)-alkyl, C(O)OR¹³, C(O)R¹³, C(O)NR¹¹R¹², R¹³O(O)C-(C₁-C₆)-alkyl, R¹¹R¹²N(O)C-(C₁-C₈)-alkyl, Bis-(C₁-C₆)-alkoxy-(C₁-C₆)-alkyl steht,
R¹¹ und R¹² gleich oder verschieden sind und unabhängig voneinander für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkylthio-(C₁-C₆)-alkyl, (C₁-C₆)-Haloalkylthio-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, C(O)R¹³, SO₂R¹⁴, Heterocyclyl, (C₁-C₆)-Alkoxycarbonyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Aryl-(C₁-C₆)-alkoxycarbonyl, Heteroaryl-(C₁-C₆)-alkoxycarbonyl, (C₂-C₆)-Alkenyloxycarbonyl, (C₂-C₆)-Alkinyloxycarbonyl, Heterocyclyl-(C₁-C₆)-alkyl stehen, oder
R¹¹ und R¹² mit dem Stickstoffatom, an das sie gebunden sind, einen vollständig gesättigten oder teilgesättigten, gegebenenfalls durch Heteroatome unterbrochenen und gegebenenfalls weiter substituierten 3 bis 10-gliedrigen monocyclischen oder bicyclischen Ring bilden,
R¹³ für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Haloalkoxy-(C₁-C₆)-alkyl, (C₁-C₆)-Alkoxy-(C₁-C₆)-haloalkyl, (C₁-C₆)-Alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkyl, (C₁-C₆)-Alkoxy-(C₁-C₆)-alkoxy-(C₁-C₆)-alkoxy-(C₁-C₆)-alkoxy-(C₁-C₆)-alkyl, Aryl, Aryl-(C₁-C₆)-alkyl, Aryl-(C₁-C₆)-alkoxy-(C₁-C₆)-alkyl, Heteroaryl, Heteroaryl-(C₁-C₆)-alkyl, (C₃-C₆)-Cycloalkyl-(C₁-C₆)-alkyl, (C₄-C₁₀)-Cycloalkenyl-(C₁-C₆)-alkyl, Bis-[(C₁-C₆)-alkyl]aminocarbonyl-(C₁-C₆)-alkyl, (C₁-C₆)-Alkyl-aminocarbonyl-(C₁-C₆)-alkyl, Aryl-(C₁-C₆)-alkyl-aminocarbonyl-(C₁-C₆)-alkyl, Bis-[(C₁-C₆)-alkyl]amino-(C₂-C₆)-alkyl, (C₁-C₆)-Alkyl-amino-(C₂-C₆)-alkyl, Aryl-(C₁-C₆)-alkyl-amino-(C₂-C₆)-alkyl, R¹⁴S-(C₁-C₆)-alkyl, R¹⁴(O)S-(C₁-C₆)-alkyl, R¹⁴O₂S-(C₁-C₆)-alkyl, Hydroxycarbonyl-(C₁-C₆)-alkyl, Heterocyclyl, Heterocyclyl-(C₁-C₆)-alkyl, Tris-[(C₁-C₆)-Alkyl]silyl-(C₁-C₆)-alkyl, Bis-[(C₁-C₆)-Alkyl](aryl)silyl(C₁-C₆)-alkyl, [(C₁-C₆)-Alkyl]-bis-(aryl)silyl-(C₁-C₆)-alkyl, (C₁-C₆)-Alkylcarbonyloxy-(C₁-C₆)-alkyl, (C₃-C₆)-Cycloalkylcarbonyloxy-(C₁-C₆)-alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, Aryloxy-(C₁-C₆)-alkyl, Heteroaryloxy-(C₁-C₆)-alkyl, (C₁-C₆)-Alkoxycarbonyl steht
und
R¹⁴ für Wasserstoff, (C₁-C₆)-Alkyl, (C₂-C₆)-Alkenyl, (C₂-C₆)-Alkinyl, (C₁-C₆)-Cyanoalkyl, (C₁-C₁₀)-Haloalkyl, (C₂-C₆)-Haloalkenyl, (C₃-C₆)-Haloalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₆)-Alkoxy-(C₁-C₆)-alkyl, (C₁-C₆)-Alkoxy-(C₁-C₆)-haloalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-Cycloalkyl-(C₁-C₆)-alkyl, (C₄-C₁₀)-Cycloalkenyl-(C₁-C₆)-alkyl, Bis-[(C₁-C₆)-alkyl]amino, (C₁-C₆)-Alkyl-amino, Aryl-(C₁-C₆)-amino, Aryl-(C₁-C₆)-alkyl-amino, Aryl-[(C₁-C₆)-alkyl]amino; (C₃-C₆)-Cycloalkyl-amino, (C₃-C₆)-Cycloalkyl-[(C₁-C₆)-alkyl]amino; N-Azetidinyl, N-Pyrrolidinyl, N-Piperidinyl. N-Morpholinyl steht.

4. Verbindungen der allgemeinen Formel (I) gemäß Anspruch 1 und/oder deren Salz, **dadurch gekennzeichnet, dass**
R¹ für Wasserstoff steht,
R² für Wasserstoff, Fluor, Chlor, steht,
R³ für Methyl, Ethyl, Prop-1-yl, 1-Methylethyl, But-1-yl, 1-Methylpropyl, 2-Methylpropyl steht,
G für einen Rest der nachfolgenden Formel 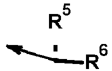 steht,
R⁵ und R⁶ unabhängig voneinander für Wasserstoff, Methyl, Ethyl, Prop-1-yl, 1-Methylethyl, But-1-yl, 1-Methylpropyl, 2-Methylpropyl, 1,1-Dimethylethyl, n-Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,1-Dimethylbutyl, 1,2-Dimethylbutyl, 1,3-Di-methylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl stehen, oder
R⁵ und R⁶ mit dem Kohlenstoffatom, an das sie gebunden sind, einen vollständig gesättigten monocyclischen 3- bis 6-gliedrigen Carbocyclus bilden,
und
Q für eine der nachfolgend spezifisch genannten Gruppierungen Q-1 bis Q-485 steht:
| | | | | |
|---|---|---|---|---|
| 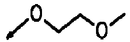 | 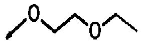 | 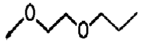 | 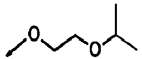 | 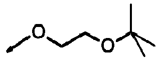 |
| Q-1 | Q-2 | Q-3 | Q-4 | Q-5 |
| 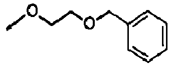 | 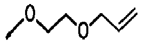 | 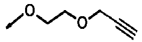 | 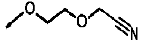 | 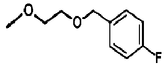 |
| Q-6 | Q-7 | Q-8 | Q-9 | Q-10 |
| 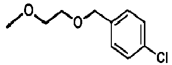 | 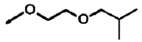 | 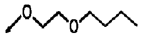 | 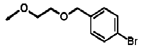 | 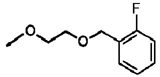 |
| Q-11 | Q-12 | Q-13 | Q-14 | Q-15 |
| 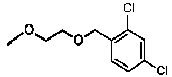 | 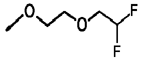 | 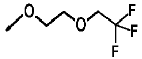 | 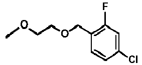 | 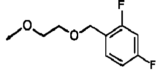 |
| Q-16 | Q-17 | Q-18 | Q-19 | Q-20 |
| 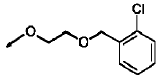 | 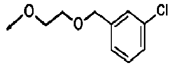 | 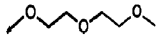 | 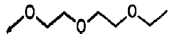 | 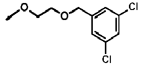 |
| Q-21 | Q-22 | Q-23 | Q-24 | Q-25 |
| 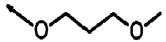 | 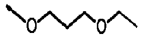 | 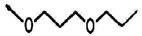 | 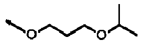 | 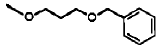 |
| Q-26 | Q-27 | Q-28 | Q-29 | Q-30 |
| 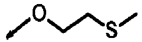 | 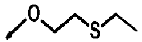 | 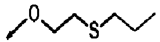 | 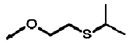 | 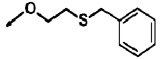 |
| Q-31 | Q-32 | Q-33 | Q-34 | Q-35 |
| 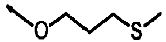 | 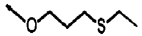 | 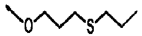 | 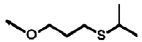 | 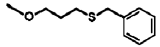 |
| Q-36 | Q-37 | Q-38 | Q-39 | Q-40 |
| 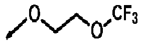 | 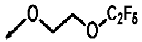 | 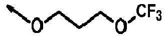 | 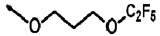 | 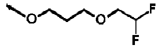 |
| Q-41 | Q-42 | Q-43 | Q-44 | Q-45 |
| 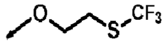 | 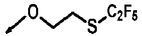 | 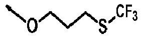 | 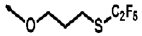 | 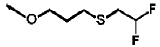 |
| Q-46 | Q-47 | Q-48 | Q-49 | Q-50 |
| 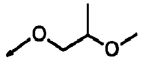 | 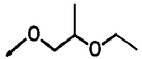 | 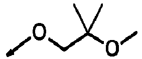 | 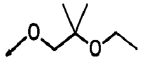 | 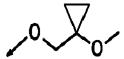 |
| Q-51 | Q-52 | Q-53 | Q-54 | Q-55 |
| 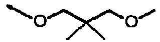 | 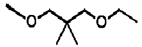 | 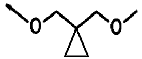 | 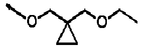 | 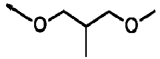 |
| Q-56 | Q-57 | Q-58 | Q-59 | Q-60 |
| 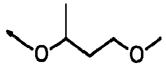 | 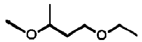 | 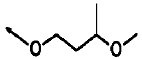 | 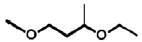 | 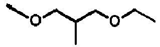 |
| Q-61 | Q-62 | Q-63 | Q-64 | Q-65 |
| 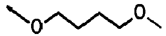 | 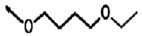 | 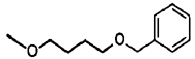 | 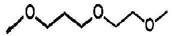 | 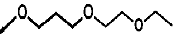 |
| Q-66 | Q-67 | Q-68 | Q-69 | Q-70 |
| 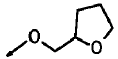 | 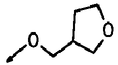 | 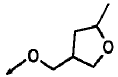 | 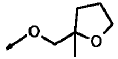 | 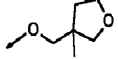 |
| Q-71 | Q-72 | Q-73 | Q-74 | Q-75 |
| 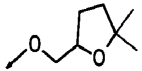 | 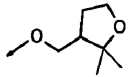 | 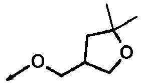 | 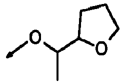 | 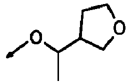 |
| Q-76 | Q-77 | Q-78 | Q-79 | Q-80 |
| 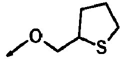 | 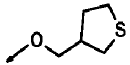 | 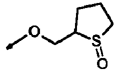 | 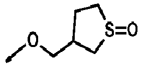 | 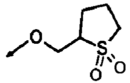 |
| Q-81 | Q-82 | Q-83 | Q-84 | Q-85 |
| 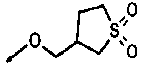 | 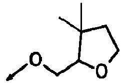 | 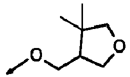 | 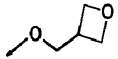 | 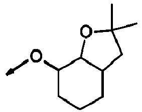 |
| Q-86 | Q-87 | Q-88 | Q-89 | Q-90 |
| 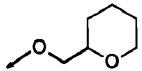 | 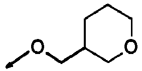 | 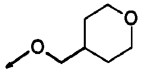 | 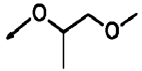 | 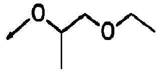 |
| Q-91 | Q-92 | Q-93 | Q-94 | Q-95 |
| 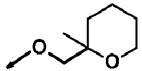 | 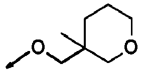 | 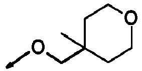 | 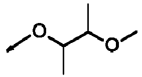 | 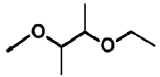 |
| Q-96 | Q-97 | Q-98 | Q-99 | Q-100 |
| 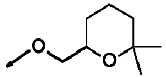 | 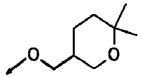 | 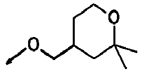 | 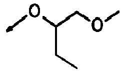 | 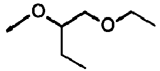 |
| Q-101 | Q-102 | Q-103 | Q-104 | Q-105 |
| 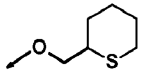 | 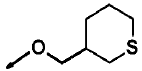 | 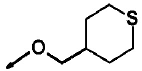 | 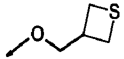 | 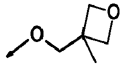 |
| Q-106 | Q-107 | Q-108 | Q-109 | Q-110 |
| 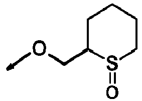 | 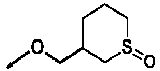 | 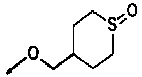 | 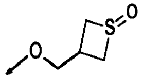 | 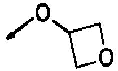 |
| Q-111 | Q-112 | Q-113 | Q-114 | Q-115 |
| 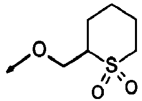 | 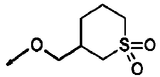 | 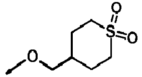 | 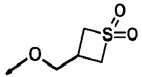 | 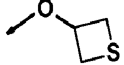 |
| Q-116 | Q-117 | Q-118 | Q-119 | Q-120 |
| 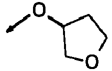 | 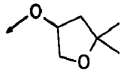 | 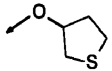 | 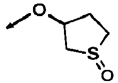 | 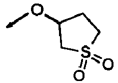 |
| Q-121 | Q-122 | Q-123 | Q-124 | Q-125 |
| 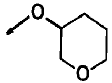 | 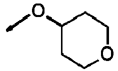 | 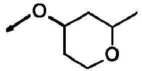 | 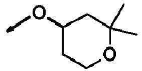 | 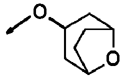 |
| Q-126 | Q-127 | Q-128 | Q-129 | Q-130 |
| 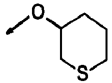 | 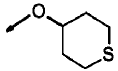 | 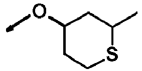 | 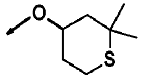 | 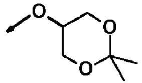 |
| Q-131 | Q-132 | Q-133 | Q-134 | Q-135 |
| 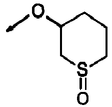 | 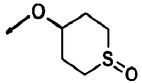 | 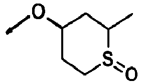 | 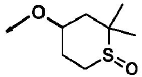 | 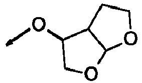 |
| Q-136 | Q-137 | Q-138 | Q-139 | Q-140 |
| 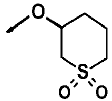 | 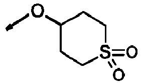 | 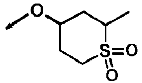 | 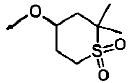 | 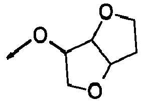 |
| Q-141 | Q-142 | Q-143 | Q-144 | Q-145 |
| 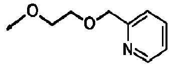 | 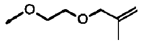 | 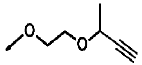 | 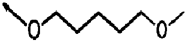 | 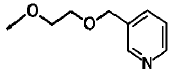 |
| Q-146 | Q-147 | Q-148 | Q-149 | Q-150 |
| 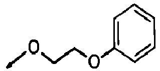 | 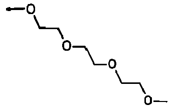 | 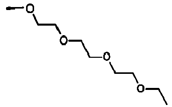 | 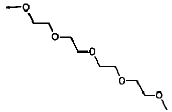 | 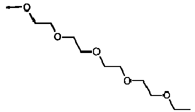 |
| Q-151 | Q-152 | Q-153 | Q-154 | Q-155 |
| 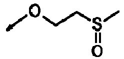 | 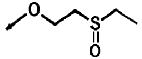 | 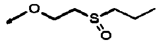 | 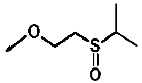 | 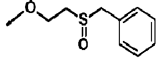 |
| Q-156 | Q-157 | Q-158 | Q-159 | Q-160 |
| 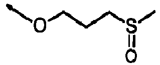 | 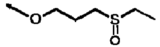 | 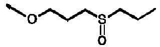 | 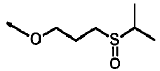 | 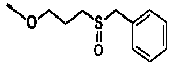 |
| Q-161 | Q-162 | Q-163 | Q-164 | Q-165 |
| 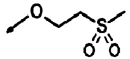 | 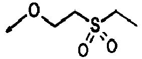 | 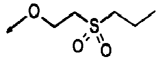 | 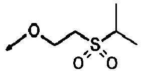 | 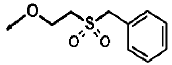 |
| Q-166 | Q-167 | Q-168 | Q-169 | Q-170 |
| 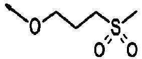 | 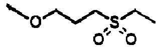 | 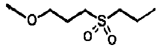 | 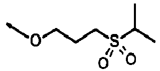 | 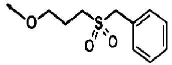 |
| Q-171 | Q-172 | Q-173 | Q-174 | Q-175 |
| 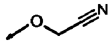 | 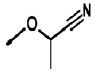 | 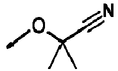 | 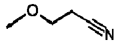 | 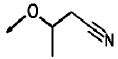 |
| Q-176 | Q-177 | Q-178 | Q-179 | Q-180 |
| 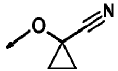 | 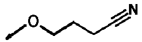 | 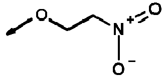 | 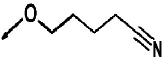 | 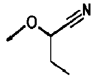 |
| Q-181 | Q-182 | Q-183 | Q-184 | Q-185 |
| 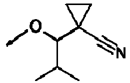 | 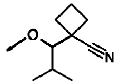 | 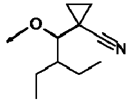 | 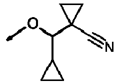 | 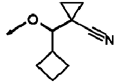 |
| Q-186 | Q-187 | Q-188 | Q-189 | Q-190 |
| 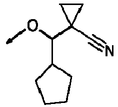 | 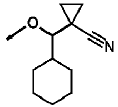 | 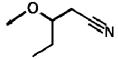 | 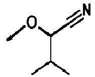 | 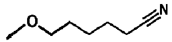 |
| Q-191 | Q-192 | Q-193 | Q-194 | Q-195 |
| 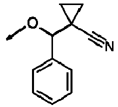 | 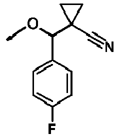 | 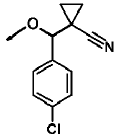 | 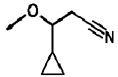 | 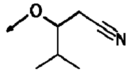 |
| Q-196 | Q-197 | Q-198 | Q-199 | Q-200 |
| 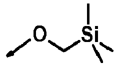 | 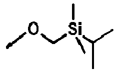 | 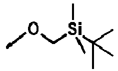 | 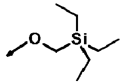 | 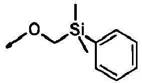 |
| Q-201 | Q-202 | Q-203 | Q-204 | Q-205 |
| 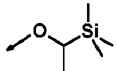 | 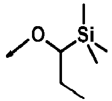 | 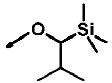 | 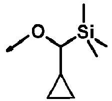 | 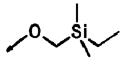 |
| Q-206 | Q-207 | Q-208 | Q-209 | Q-210 |
| 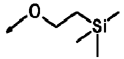 | 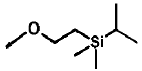 | 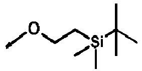 | 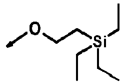 | 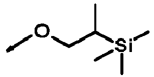 |
| Q-211 | Q-212 | Q-213 | Q-214 | Q-215 |
| 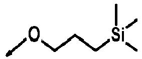 | 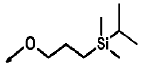 | 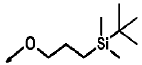 | 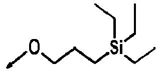 | 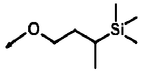 |
| Q-216 | Q-217 | Q-218 | Q-219 | Q-220 |
| 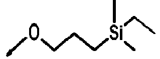 | 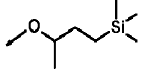 | 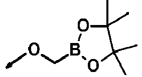 | 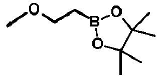 | 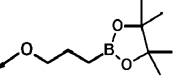 |
| Q-221 | Q-222 | Q-223 | Q-224 | Q-225 |
| 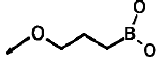 | 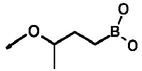 | 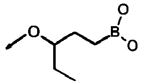 | 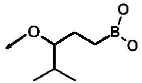 | 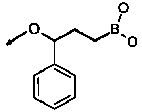 |
| Q-226 | Q-227 | Q-228 | Q-229 | Q-230 |
| 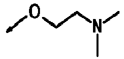 | 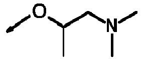 | 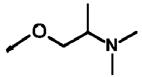 | 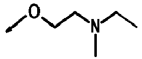 | 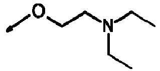 |
| Q-231 | Q-232 | Q-233 | Q-234 | Q-235 |
| 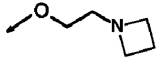 | 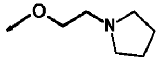 | 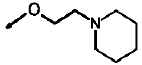 | 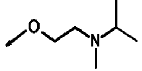 | 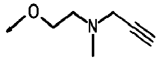 |
| Q-236 | Q-237 | Q-238 | Q-239 | Q-240 |
| 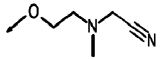 | 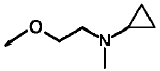 | 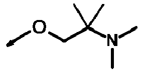 | 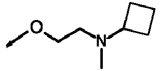 | 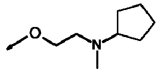 |
| Q-241 | Q-242 | Q-243 | Q-244 | Q-245 |
| 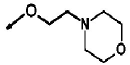 | 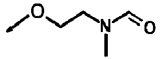 | 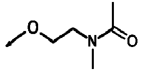 | 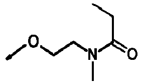 | 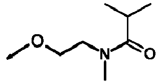 |
| Q-246 | Q-247 | Q-248 | Q-249 | Q-250 |
| 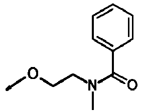 | 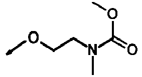 | 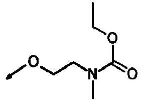 | 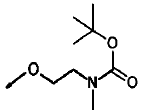 | 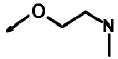 |
| Q-251 | Q-252 | Q-253 | Q-254 | Q-255 |
| 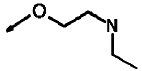 | 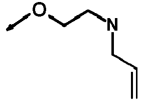 | 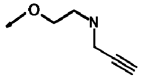 | 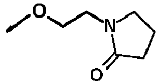 | 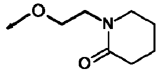 |
| Q-256 | Q-257 | Q-258 | Q-259 | Q-260 |
| 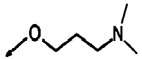 | 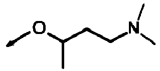 | 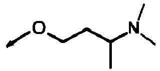 | 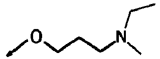 | 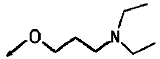 |
| Q-261 | Q-262 | Q-263 | Q-264 | Q-265 |
| 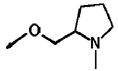 | 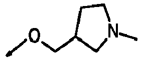 | 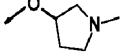 | 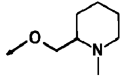 | 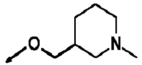 |
| Q-266 | Q-267 | Q-268 | Q-269 | Q-270 |
| 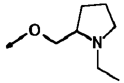 | 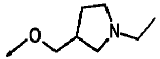 | 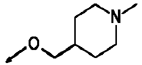 | 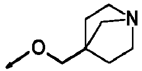 | 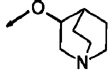 |
| Q-271 | Q-272 | Q-273 | Q-274 | Q-275 |
| 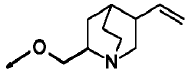 | 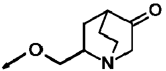 | 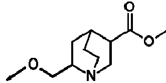 | 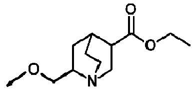 | 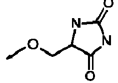 |
| Q-276 | Q-277 | Q-278 | Q-279 | Q-280 |
| 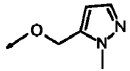 | 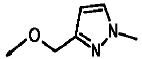 | 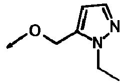 | 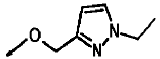 | 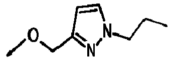 |
| Q-281 | Q-282 | Q-283 | Q-284 | Q-285 |
| 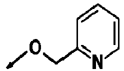 | 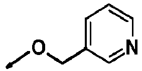 | 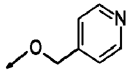 | 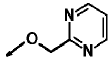 | 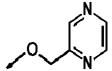 |
| Q-286 | Q-287 | Q-288 | Q-289 | Q-290 |
| 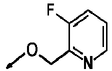 | 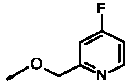 | 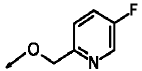 | 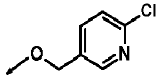 | 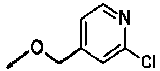 |
| Q-291 | Q-292 | Q-293 | Q-294 | Q-295 |
| 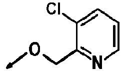 | 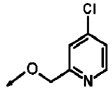 | 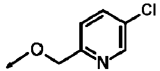 | 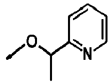 | 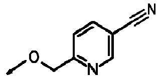 |
| Q-296 | Q-297 | Q-298 | Q-299 | Q-300 |
| 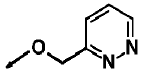 | 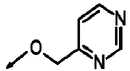 | 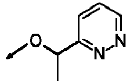 | 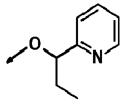 | 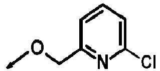 |
| Q-301 | Q-302 | Q-303 | Q-304 | Q-305 |
| 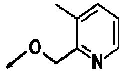 | 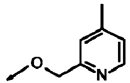 | 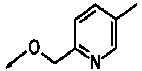 | 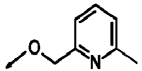 | 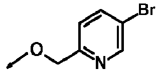 |
| Q-306 | Q-307 | Q-308 | Q-309 | Q-310 |
| 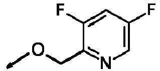 | 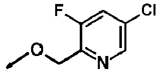 | 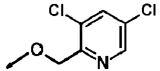 | 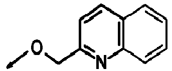 | 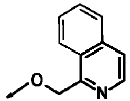 |
| Q-311 | Q-312 | Q-313 | Q-314 | Q-315 |
| 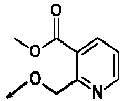 | 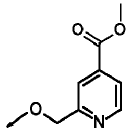 | 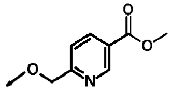 | 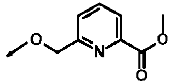 | 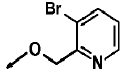 |
| Q-316 | Q-317 | Q-318 | Q-319 | Q-320 |
| 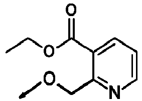 | 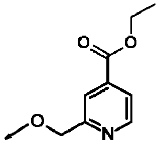 | 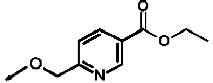 | 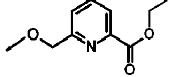 | 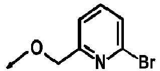 |
| Q-321 | Q-322 | Q-323 | Q-324 | Q-325 |
| 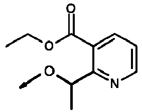 | 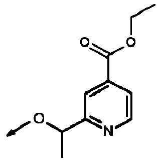 | 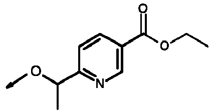 | 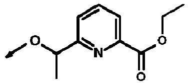 | 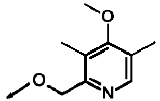 |
| Q-326 | Q-327 | Q-328 | Q-329 | Q-330 |
| 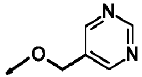 | 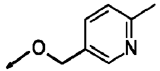 | 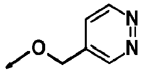 | 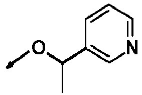 | 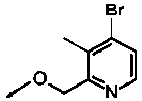 |
| Q-331 | Q-332 | Q-333 | Q-334 | Q-335 |
| 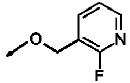 | 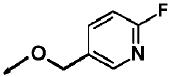 | 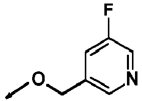 | 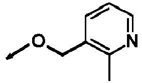 | 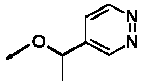 |
| Q-336 | Q-337 | Q-338 | Q-339 | Q-340 |
| 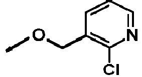 | 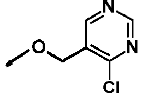 | 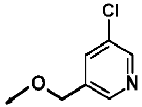 | 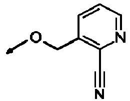 | 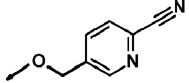 |
| Q-341 | Q-342 | Q-343 | Q-344 | Q-345 |
| 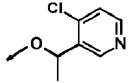 | 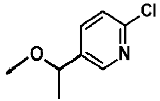 | 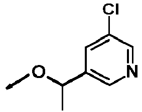 | 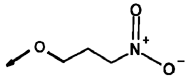 | 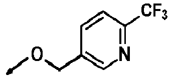 |
| Q-346 | Q-347 | Q-348 | Q-349 | Q-350 |
| 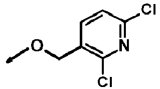 | 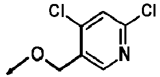 | 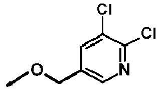 | 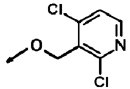 | 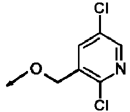 |
| Q-351 | Q-352 | Q-353 | Q-354 | Q-355 |
| 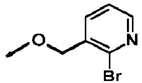 | 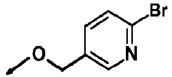 | 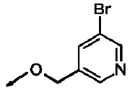 | 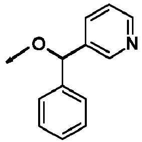 | 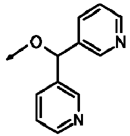 |
| Q-356 | Q-357 | Q-358 | Q-359 | Q-360 |
| 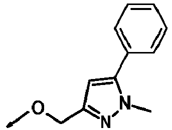 | 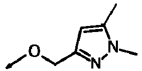 | 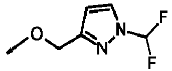 | 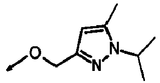 | 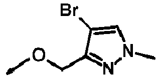 |
| Q-361 | Q-362 | Q-363 | Q-364 | Q-365 |
| 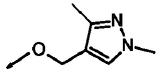 | 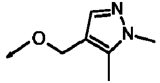 | 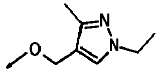 | 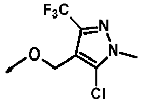 | 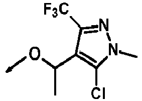 |
| Q-366 | Q-367 | Q-368 | Q-369 | Q-370 |
| 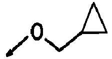 | 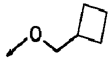 | 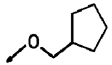 | 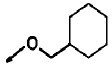 | 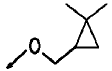 |
| Q-371 | Q-372 | Q-373 | Q-374 | Q-375 |
| 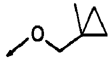 | 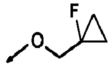 | 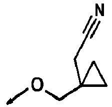 | 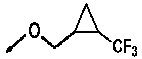 | 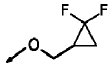 |
| Q-376 | Q-377 | Q-378 | Q-379 | Q-380 |
| 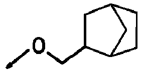 | 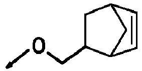 | 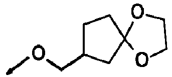 | 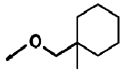 | 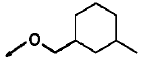 |
| Q-381 | Q-382 | Q-383 | Q-384 | Q-385 |
| 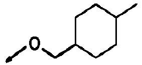 | 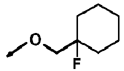 | 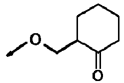 | 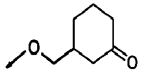 | 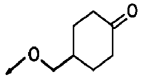 |
| Q-386 | Q-387 | Q-388 | Q-389 | Q-390 |
| 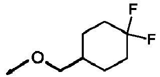 | 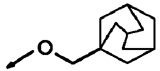 | 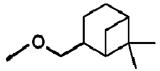 | 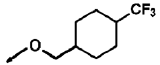 | 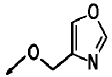 |
| Q-391 | Q-392 | Q-393 | Q-394 | Q-395 |
| 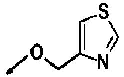 | 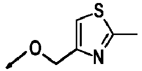 | 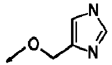 | 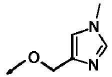 | 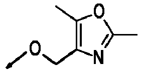 |
| Q-396 | Q-397 | Q-398 | Q-399 | Q-400 |
| 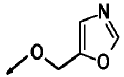 | 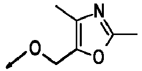 | 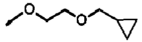 | 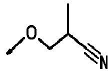 | 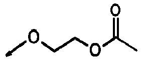 |
| Q-401 | Q-402 | Q-403 | Q-404 | Q-405 |
| 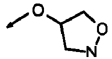 | 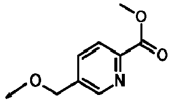 | 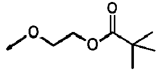 | 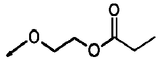 | 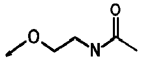 |
| Q-406 | Q-407 | Q-408 | Q-409 | Q-410 |
| 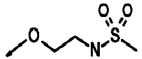 | 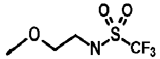 | 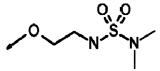 | 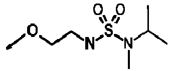 | 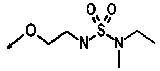 |
| Q-411 | Q-412 | Q-413 | Q-414 | Q-415 |
| 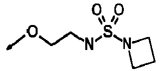 | 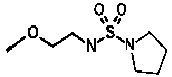 | 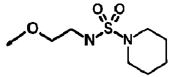 | 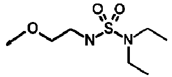 | 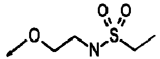 |
| Q-416 | Q-417 | Q-418 | Q-419 | Q-420 |
| 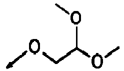 | 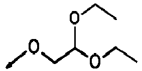 | 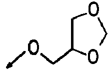 | 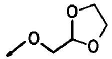 | 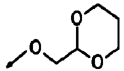 |
| Q-421 | Q-422 | Q-423 | Q-424 | Q-425 |
| 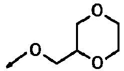 | 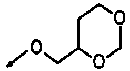 | 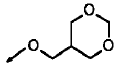 | 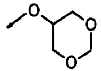 | 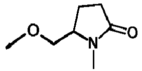 |
| Q-426 | Q-427 | Q-428 | Q-429 | Q-430 |
| 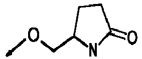 | 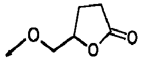 | 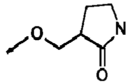 | 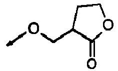 | 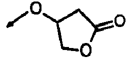 |
| Q-431 | Q-432 | Q-433 | Q-434 | Q-435 |
| 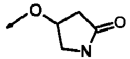 | 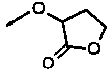 | 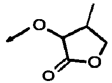 | 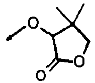 | 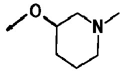 |
| Q-436 | Q-437 | Q-438 | Q-439 | Q-440 |
| 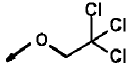 | 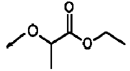 | 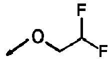 | 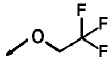 | 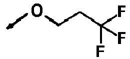 |
| Q-441 | Q-442 | Q-443 | Q-444 | Q-445 |
| 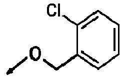 | 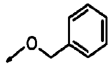 | 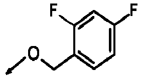 | 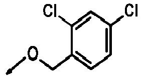 | 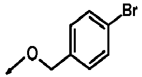 |
| Q-446 | Q-447 | Q-448 | Q-449 | Q-450 |
| 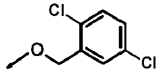 | 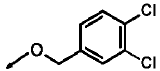 | 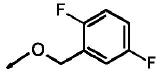 | 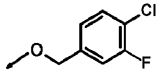 | 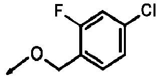 |
| Q-451 | Q-452 | Q-453 | Q-454 | Q-455 |
| 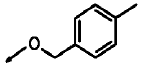 | 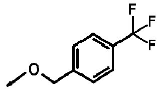 | 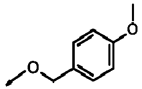 | 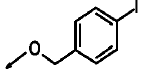 | 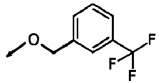 |
| Q-456 | Q-457 | Q-458 | Q-459 | Q-460 |
| 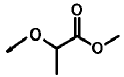 | 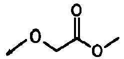 | 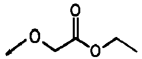 | 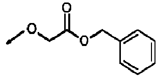 | 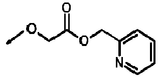 |
| Q-461 | Q-462 | Q-463 | Q-464 | Q-465 |
| 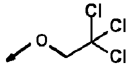 | 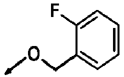 | 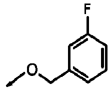 | 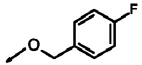 | 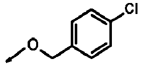 |
| Q-466 | Q-467 | Q-468 | Q-469 | Q-470 |
| 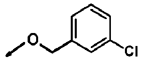 | 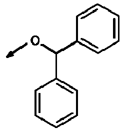 | 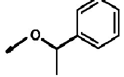 | 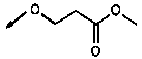 | 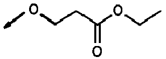 |
| Q-471 | Q-472 | Q-473 | Q-473 | Q-475 |
| 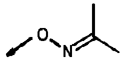 | 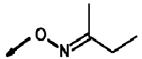 | 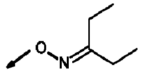 | 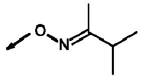 | 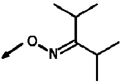 |
| Q-476 | Q-477 | Q-478 | Q-479 | Q-480 |
| 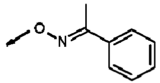 | 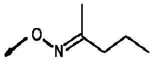 | 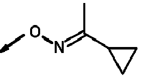 | 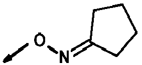 | 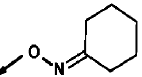 |
| Q-481 | Q-482 | Q-483 | Q-484 | Q-485 |

5. Verbindungen der allgemeinen Formel (I) gemäß Anspruch 1 und/oder deren Salz, **dadurch gekennzeichnet, dass**
R¹ für Wasserstoff steht,
R² für Wasserstoff, Fluor, Chlor, steht,
R³ für Methyl steht,
G für einen Rest der nachfolgenden Formel 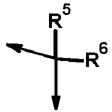 steht,
R⁵ und R⁶ unabhängig voneinander für Wasserstoff, Methyl, Ethyl, bevorzugt Methyl stehen, oder
R⁵ und R⁶ mit dem Kohlenstoffatom, an das sie gebunden sind, einen vollständig gesättigten monocyclischen 3- bis 6-gliedrigen Carbocyclus bilden,
und
Q für eine der in Anspruch 4 spezifisch genannten Gruppierungen Q-1 bis Q-485 steht:

6. Verbindungen der allgemeinen Formel (I) gemäß Anspruch 1 und/oder deren Salz, **dadurch gekennzeichnet, dass**
R¹ für Wasserstoff steht,
R² für Wasserstoff, Fluor, Chlor, steht,
R³ für Methyl steht,
G für einen Rest der nachfolgenden Formel 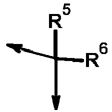 steht,
R⁵ und R⁶ für Methyl stehen
und
Q für eine der in Anspruch 4 genannten Gruppierungen Q-1, Q-2, Q-41, Q-176, Q-286, Q-301, Q-444, Q-476 steht.

7. Verwendung einer oder mehrere Verbindungen der allgemeinen Formel (I) wie in einem der Ansprüche 1 bis 6 definiert und/oder deren Salze, als Herbizid und/oder Pflanzenwachstumsregulator, vorzugsweise in Kulturen von Nutz- und/oder Zierpflanzen.

8. Herbizides und/oder pflanzenwachstumsregulierendes Mittel, **dadurch gekennzeichnet, dass** das Mittel eine oder mehrere Verbindungen der allgemeinen Formel (I) wie in einem der Ansprüche 1 bis 6 definiert und/oder deren Salze enthält, und ein oder mehrere weitere Stoffe ausgewählt aus den Gruppen (i) und/oder (ii), mit
(i) ein oder mehrere weitere agrochemisch wirksame Stoffe, vorzugsweise ausgewählt aus der Gruppe bestehend aus Insektiziden, Akariziden, Nematiziden, weiteren Herbiziden, Fungiziden, Safenern, Düngemitteln und/oder weiteren Wachstumsregulatoren,
(ii) ein oder mehrere im Pflanzenschutz übliche Formulierungshilfsmittel.

9. Verfahren zur Bekämpfung von Schadpflanzen oder zur Wachstumsregulierung von Pflanzen, **dadurch gekennzeichnet, dass** eine wirksame Menge
- einer oder mehrerer Verbindungen der allgemeinen Formel (I), wie in einem der Ansprüche 1 bis 6 definiert und/oder deren Salze, oder
- eines Mittels nach Anspruch 8, auf die Pflanzen, Pflanzensamen, den Boden, in dem oder auf dem die Pflanzen wachsen, oder die Anbaufläche appliziert wird.
